# EUROPEAN PATENT SPECIFICATION

(11) **EP 4 240 288 B1**
(45) Date of publication and mention of the grant of the patent: **05.11.2025**
(21) Application number: 20823921.0
(22) Date of filing: 05.11.2020
(51) Int. Cl.: A61F 2/30, A61F 2/44

(54) **EXPANDABLE INTER-BODY DEVICE AND SYSTEM**
EXPANDIERBARE ZWISCHENKÖRPERVORRICHTUNG UND SYSTEM
SYSTÈME ET DISPOSITIF INTERVERTÉBRAUX EXTENSIBLES

(43) Date of publication of application: 13.09.2023
(73) Proprietor: Warsaw Orthopedic, Inc., Warsaw, Indiana 46581 (US); Peultier, Bertrand, 25370 Les Hopitaux Neufs (FR)
(72) Inventor: JOSSE, Loic, Collierville, 38017 (US); PEULTIER, Bertrand, 25370 Les Hôpitaux Neufs (FR)
(74) Representative: Viering, Jentschura & Partner mbB Patent- und Rechtsanwälte
(86) International application number: PCT/IB2020/000953
(87) International publication number: WO 2022/096912

(56) References cited:
- US-A1- 2019 336 301
- US-B2- 10 709 572

## Description

### FIELD

The present disclosure generally relates to medical devices for the treatment of musculoskeletal disorders, and more particularly to a surgical device that includes an expandable spinal implant, systems for implanting and manipulating the expandable spinal implant, and a method (not claimed) for treating a spine.

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, they may fail to relieve the symptoms associated with these disorders. Surgical treatment of these spinal disorders includes fusion, fixation, correction, discectomy, laminectomy and implantable prosthetics. As part of these surgical treatments, spinal constructs, such as, for example, bone fasteners, spinal rods and interbody devices can be used to provide stability to a treated region. For example, during surgical treatment, interbody devices may be introduced to a space between adjacent vertebral bodies (the interbody space) to properly space the vertebral bodies and provide a receptacle for bone growth promoting materials, *e.g*., grafting.

More recently, interbody devices have been introduced that provide additional capability beyond static spacing of the vertebral bodies. For example, some devices have expansion capability such that the implant may be introduced to the interbody space in a collapsed state and then expanded to produce additional spacing and, in some cases, introduce or restore curvature to the spine by expanding selectively. However, many existing expandable interbody designs have limited ranges of expansion.

An additional problem exists related to subsidence of spinal surfaces due to existing interbody devices having inadequately-sized load-bearing surfaces. In the case of expandable devices, the loads on the load-bearing surfaces, including loads generated during expansion of the implant, are often significant. An expandable implant with relatively large surface areas is needed to bear the loads, including the loads generated during implant expansion, in an attempt to avoid a need for follow-on surgery due to subsidence of spinal surfaces.

A further problem is instability of existing expandable interbody devices as they are expanded. Often, the load-bearing surfaces move relative to one another, as well as relative to an inserter, as the interbody device is expanded such that there is a risk of undesired shifts in the positioning of the interbody device within the interverterbral space. Additionally, and depending at least partly on the particular insertion technique employed, anatomical features such as the iliac crest and rib cage pose challenges to the adjustment of inter-body designs in situ.

The present disclosure seeks to address these and other shortcomings in the existing relevant arts.

US 2019/336301 A1 discloses an intervertebral implant that iterates between collapsed and expanded configurations includes first and second plates spaced from one another along a first direction and defining bone-contacting surfaces facing away from each other along the first direction. An expansion assembly is positioned between the plates with respect to the first direction and includes a first support wedge that supports the first plate and defines a first ramp and a second support wedge that supports the second plate and defines second and third ramps. The expansion assembly includes an expansion wedge defining a fourth ramp. The first, second, third, and fourth ramps are each inclined with respect to a second direction that is substantially perpendicular to the first direction. At least one of the first and second support wedges is slidable along the respective supported first or second plate. The implant includes an actuator configured to apply a drive force to the expansion wedge so as to cause 1) the fourth ramp to ride along the third ramp so as to increase a distance between the bone-contacting surfaces along the first direction, and 2) the second ramp to ride along the first ramp, thereby further increasing the distance, thereby iterating the implant from the collapsed to the expanded configuration.

According to US 10 709 572 B2, an expandable interbody fusion implant device has a frame, two ramp assemblies and two overlying base plates driven by two independent drive shafts. The two ramp assemblies include a distal ramp assembly and a proximal ramp assembly. Each ramp assembly has a translating ramp, a first pivoting hinged ramp and a second pivoting hinged ramp. The two overlying base plates include a first base plate overlying a second base plate. Each base plate is hinged to the distal ramp assembly and the proximal ramp assembly at an end of one of said pivoting hinged ramps of each ramp assembly. The two independently driven drive shafts include a first drive shaft for translating the distal ramp assembly and a second drive shaft for translating the proximal ramp assembly to independently expand the implant proximally or distally or both.

### SUMMARY

The techniques of this disclosure generally relate to highly adjustable interbody devices that are expandable to selectively increase/decrease a spacing distance between endplates of the interbody device and adjustable to selectively increase/decrease an angle of inclination between endplates of the interbody device.

The invention provides an expandable spinal implant according to claim 1. Further embodiments are described in the dependent claims.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1A is a perspective view of one embodiment of an expandable spinal implant in a fully contracted position in accordance with the principles of the present disclosure;
FIG. 1B is an exploded parts view of the embodiment of FIG. 1A in accordance with the principles of the present disclosure;
FIG. 1C is a perspective view of one embodiment of an expandable spinal implant in a contracted or closed configuration in accordance with the principles of the present disclosure;
FIG. 1D is a perspective view of one embodiment of an expandable spinal implant in an expanded or opened configuration in accordance with the principles of the present disclosure;
FIGS. 2A and 2B are a top down views of the embodiment of FIGS. 1A and 1B in accordance with the principles of the present disclosure;
FIGS. 2C and 2D are side views of the embodiment of FIGS. 1A and 1B in a contracted position in accordance with the principles of the present disclosure;
FIGS. 2E and 2F are side views of the embodiment of FIGS. 1A and 1B in an expanded position in accordance with the principles of the present disclosure;
FIG. 3A is a perspective view of one embodiment of an expandable spinal implant in a closed configuration in accordance with the principles of the present disclosure;
FIG. 3B is a perspective view of one embodiment of an expandable spinal implant in an expanded configuration in accordance with the principles of the present disclosure;
FIG. 4A is a top down view of the embodiment of FIGS. 2A-2C in accordance with the principles of the present disclosure;
FIG. 4B is a side view of the embodiment of FIGS. 2A-2C in a contracted position in accordance with the principles of the present disclosure;
FIG. 4C is a side view of the embodiment of FIGS. 2A-2C in a partially expanded and inclined position in accordance with the principles of the present disclosure;
FIG. 4D is a side view of the embodiment of FIGS. 2A-2C in a fully expanded position in accordance with the principles of the present disclosure;
FIG. 5A is a top down view of one embodiment in accordance with the principles of the present disclosure;
FIG. 5B is a front side view of the embodiment of FIG. 5A in accordance with the principles of the present disclosure;
FIG. 5C is an alternate side view of the embodiment of FIG. 5A in accordance with the principles of the present disclosure;
FIGS. 6A-6C are top down views of three exemplary footprint sizes of a top endplate in accordance with the principles of the present disclosure;
FIGS. 7A-7C are top down views of three exemplary footprint sizes of a bottom endplate in accordance with the principles of the present disclosure;
FIG. 8 is perspective view of one embodiment of an expandable spinal implant system in accordance with the principles of the present disclosure;
FIG. 9A is a cutout perspective showing a surgical tool in a first adjustment position where an exemplary spinal implant is in a contracted position;
FIG. 9B is a cutout perspective showing the surgical tool in the first adjustment position after adjusting the exemplary spinal implant from the contracted position to a first expanded position;
FIG. 10A is a cutout perspective showing the surgical tool in a second adjustment position where the exemplary spinal implant is in the first expanded position of FIG. 9B;
FIG. 10B is a cutout perspective showing the surgical tool in the second position after adjusting the exemplary spinal implant from the first expanded position to an expanded and angled position;
FIGS. 11A and 11B are perspective views of a moving mechanism in a contracted position and an expanded position, respectively, in accordance with the principles of the present disclosure;
FIGS. 12A and 12B are perspective views of the moving mechanism of FIGS. 11A and 11B in the contracted position and the expanded position, respectively, with a bottom endplate in accordance with the principles of the present disclosure;
FIGS. 13A and 13B are perspective views of the moving mechanism of FIGS. 12A and 12B in the contracted position and the expanded position, respectively, with a top endplate and the bottom endplate in accordance with the principles of the present disclosure;
FIGS. 14A and 14B are cut-out views of a moving mechanism in accordance with the principles of the present disclosure;
FIG. 15 is a cross section of the moving mechanism of FIGS. 14A and 14B along a longitudinal axis thereof in accordance with the principles of the present disclosure;
FIG. 16 is a perspective view of a top endplate and bottom endplate of one embodiment of an expandable spinal implant in accordance with the principles of the present disclosure;
FIG. 17 is an exploded view of the top endplate and bottom endplate of FIG. 16 in accordance with the principles of the present disclosure;
FIGS. 18A-18B are perspective views of a first surgical tool of an expandable spinal implant system in accordance with the principles of the present disclosure;
FIGS. 19A-19C are side views of first surgical tool and adjustment rod of an expandable spinal implant system, respectively, in accordance with the principles of the present disclosure;
FIG. 20 illustrates a perspective view of one embodiment of an expandable spinal implant system having anchoring screws in accordance with the principles of the present disclosure;
FIGS. 21A-21B illustrate a lateral side view and front side view, respectively, of one embodiment of an expandable spinal implant system having anchoring screws in accordance with the principles of the present disclosure;
FIG. 22A is a side view of a second surgical device suitable for use with the embodiment of FIGS. 20 in accordance with the principles of the present disclosure;
FIG. 22B is a side view of an enlarged region of FIG. 22A in accordance with the principles of the present disclosure;
FIGS. 23A-23C are various perspective views of exemplary anchoring screws suitable for use with the embodiment of FIG. 20 in conjunction with the second surgical tool of FIGS 22A-22B in accordance with the principles of the present disclosure;
FIGS. 24A-24D are various side views and top down views of exemplary bone grafts in accordance with the principles of the present disclosure;
FIG. 25A and FIG. 25B illustrate a first bent position and a second bent position, respectively, of one embodiment of an expandable spinal implant in accordance with the principles of the present disclosure;
FIGS. 26-28 illustrate a left side view, right side view, and front side view, respectively, of an installed expandable spinal implant positioned between adjacent vertebral bodies in accordance with the principles of the present disclosure;
FIG. 29A is a perspective view of one embodiment of an expandable spinal implant in accordance with the principles of the present disclosure;
FIG. 29B is an exploded view of the embodiment of FIG. 29A in accordance with the principles of the present disclosure;
FIG. 30A is a top down view of one embodiment of an expandable spinal implant in accordance with the principles of the present disclosure;
FIG. 30B is perspective view of one embodiment of an expandable spinal implant in accordance with the principles of the present disclosure;
FIG. 30C is a perspective view of one embodiment of an expandable spinal implant with a top endplate removed in accordance with the principles of the present disclosure;
FIG. 30D is an alternate perspective view of one embodiment of an expandable spinal implant with a top endplate removed in accordance with the principles of the present disclosure;
FIG. 30E is a top down view of one embodiment of a top endplate in accordance with the principles of the present disclosure;
FIG. 30F is a top down view of one embodiment of a bottom endplate in accordance with the principles of the present disclosure;
FIG. 31 is a perspective view of one embodiment of an expandable spinal implant system illustrating three alternate angular positions of an insertion tool in accordance with the principles of the present disclosure;
FIG. 32A is a top down view of one embodiment of an expandable spinal implant in accordance with the principles of the present disclosure;
FIG. 32B is a perspective view of the embodiment of FIG. 32A in accordance with the principles of the present disclosure;
FIG. 33A is a perspective view of one embodiment of an expandable spinal implant in accordance with the principles of the present disclosure;
FIG. 33B is a perspective view of the embodiment of FIG. 33A in an expanded position in accordance with the principles of the present disclosure;
FIG. 33C is a perspective view of the embodiment of FIG. 33A in a first tilted position in accordance with the principles of the present disclosure;
FIG. 33D is a perspective view of the embodiment of FIG. 33A in a second tilted position in accordance with the principles of the present disclosure;
FIG. 34 is a perspective view of one embodiment of an expandable spinal implant system in accordance with the principles of the present disclosure;
FIG. 35 is a perspective view of one embodiment of an expandable spinal implant system illustrating three alternate angular positions of an insertion tool in accordance with the principles of the present disclosure;
FIG. 36 is a perspective view of one embodiment of an expandable spinal implant including a screw guide endplate having at least one aperture configured to receive a anchoring screw therein;
FIG. 37 is a front view of the embodiment of FIG. 36;
FIGS. 38A and 38B are various perspective views of a screw guide endplate having at least one aperture configured to receive a anchoring screw therein;
FIGS. 39A and 39B are top down view of a top endplate and a bottom endplate including at least one slotted aperture configured to receive a anchoring screw therein;
FIG. 40 is a perspective view of one embodiment of an expandable spinal implant including a screw guide endplate having at least one aperture configured to receive a anchoring screw therein;
FIG. 41 is a front view of the embodiment of FIG. 40;
FIG. 42A is a front views of a screw guide endplate having at least one aperture configured to receive a anchoring screw therein;
FIG. 42B is a front view of the screw guide endplate of FIG. 42A including anchoring screws installed in each of the corresponding apertures;
FIG. 43A and FIG. 43B are various perspective views of a screw guide endplate having at least one aperture configured to receive a anchoring screw therein;
FIGS. 44A and 44B are top down views of a top endplate and a bottom endplate including at least one recessed portion configured to accommodate a anchoring screw; and
FIG. 45 is a reference diagram illustrating various cardinal directions and planes with respect to a patient that the exemplary embodiments of FIGS. 1-44B may operate, adjust, and/or move along in accordance with the principles of the present disclosure.

### DETAILED DESCRIPTION

The exemplary embodiments of, for example, an anterior expandable inter-body device, lateral expandable inter-body device, inter-body device systems, and inter-body device methods of use are discussed in terms of medical devices for the treatment of musculoskeletal disorders and more particularly, in terms of various inter-body devices suitable as spinal implants for anterior surgical techniques, oblique surgical techniques, and lateral surgical techniques. Exemplary embodiments are also discussed with related emphasis on specialized adjustment instruments such as, for example, an instrument capable of adjusting a spacing of the aforementioned various interbody devices between adjacent vertebrates of a spine by expansion and contraction as well as adjusting an angle of inclination with respect to the coronal plane and/or sagittal plane of a patient. Disclosed devices and systems may be capable of adjusting the curvature of a patient's spine for lordosis correction and a kyphosis correction. Likewise, an instrument capable of installing various anchoring screws is described in conjunction with disclosed inter-body devices.

As used herein, standard anatomical terms of location have their ordinary meaning as they would be understood by a person of ordinary skill in the art unless clearly defined or explained otherwise. It should be understood that various aspects disclosed herein may be combined in different combinations than the combinations specifically presented in the description and accompanying drawings. For example, characteristics of one embodiment may be combined or substituted with characteristics of another different embodiment unless those characteristics are clearly explained as being mutually exclusive. It should also be understood that, depending on the example, certain acts or events of any of the processes or methods described herein may be performed in a different sequence, may be added, merged, or left out altogether (*e.g.,* all described acts or events may not be necessary to carry out the disclosed techniques and methods). In addition, while certain aspects of this disclosure are described as being performed by a single module or unit for purposes of clarity, it should be understood that the techniques of this disclosure may be performed by a combination of units or modules associated with, for example, a medical device.

In some embodiments, the present system includes an expandable spinal implant suitable for insertion for oblique techniques, postero-lateral procedures and/or transforaminal lumbar interbody fusions (sometimes referred to as TLIF procedures), direct posterior (sometimes referred to as PLIF procedures), direct lateral (sometimes referred to as DLIF procedures), anterior lumbar interbody fusions (sometimes refered to as ALIF procedures), or variations of these procedures, in which the present implant is inserted into an interverterbral space and then expanded in order to impart and/or augment a lordotic and/or kyphotic curve of the spine.

In some embodiments, the spinal implant system may also be employed to restore and/or impart sagittal balance to a patient by increasing and/or restoring an appropriate lordotic and/or kyphotic angle between vertebral bodies at a selected level where the spinal implant is implanted and expanded. Additionally, some embodiments may also be employed to restore and/or impart coronal balance for correction of, for example, scoliosis. In the various embodiments described, the spinal implant system may be useful in a variety of complex spinal procedures for treating spinal conditions beyond one-level fusions. Furthermore, the spinal implant system described in the enclosed embodiments may also be used as a fusion device with an expandable height for tailoring the implant to a particular interbody disc space to restore the spacing between adjacent vertebral bodies and facilitate spinal fusion between the adjacent vertebral bodies.

In some embodiments, and as mentioned above, the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. In some embodiments, the present disclosure may be employed with other osteal and bone related applications, including those associated with diagnostics and therapeutics. In some embodiments, the disclosed spinal implant system may be alternatively employed in a surgical treatment with a patient in a prone or supine position, and/or employ various surgical approaches to the spine, including anterior, posterior, posterior mid-line, direct lateral, postero-lateral oblique, and/or antero lateral oblique approaches, and in other body regions. The present disclosure may also be alternatively employed with procedures for treating the lumbar, cervical, thoracic, sacral and pelvic regions of a spinal column. The spinal implant system of the present disclosure may also be used on animals, bone models and other non-living substrates, such as, for example, in training, testing and demonstration.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. In some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior". Generally, similar spatial references of different aspects or components, *e.g*., a "proximal end" of an end plate and a "proximal end" of a wedge, indicate similar spatial orientation and/or positioning, *i.e.,* that each "proximal end" is situated on or directed towards the same end of the device. Further, the use of various spatial terminology herein should not be interpreted to limit the various insertion techniques or orientations of the implant relative to the positions in the spine.

As used in the specification and including the appended claims, "treating" or "treatment" of a disease or condition refers to performing a procedure that may include administering one or more drugs, biologics, bone grafts (including allograft, autograft, xenograft, for example) or bone-growth promoting materials to a patient (human, normal or otherwise or other mammal), employing implantable devices, and/or employing instruments that treat the disease, such as, for example, micro-discectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, *e.g*., arresting its development, or relieving the disease, *e.g*., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise. The term "bone growth promoting material" as used herein may include, but is not limited to: bone graft (autograft, allograft, xenograft) in a variety of forms and compositions (including but not limited to morselized bone graft); osteoinductive material such as bone morphogenetic proteins (BMP) (including but not limited to INFUSE^{®} available from Medtronic) and alternative small molecule osteoinductive substances; osteoconductive materials such as demineralized bone matrix (DBM) in a variety of forms and compositions (putty, chips, bagged (including but not limited to the GRAFTON^{®} family of products available from Medtronic)); collagen sponge; bone putty; ceramic-based void fillers; ceramic powders; and/or other substances suitable for inducing, conducting or facilitating bone growth and/or bony fusion of existing bony structures. Such bone growth promoting materials may be provided in a variety of solids, putties, liquids, colloids, solutions, or other preparations suitable for being packed or placed into or around the various implants 100, 200, 300 and embodiments described herein.

The components of the expandable spinal implant systems described herein can be fabricated from biologically acceptable materials suitable for medical applications, including metals, synthetic polymers, ceramics and bone material and/or their composites. For example, the components of expandable spinal implant system, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (*e.g.,* Nitinol, super elasto-plastic metals, such as GUM METAL^{®}), ceramics and composites thereof such as calcium phosphate (*e.g*., SKELITE^{™}), thermoplastics such as polyaryletherketone (PAEK) including polyetheretherketone (PEEK), polyetherketoneketone (PEKK) and polyetherketone (PEK), carbon-PEEK composites, PEEK-BaSO4 polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaprolactone and their combinations.

Various components of spinal implant system may be formed or constructed of material composites, including but not limited to the above-described materials, to achieve various desired characteristics such as strength, rigidity, elasticity, compliance, biomechanical performance, durability and radiolucency or imaging preference. The components of expandable spinal implant system, individually or collectively, may also be fabricated from a heterogeneous material such as a combination of two or more of the above-described materials. The components of the expandable spinal implant systems may be monolithically formed, integrally connected or include fastening elements and/or instruments, as described herein. For example, in some embodiments the expandable spinal implant systems may comprise expandable spinal implants 100, 200, 300 comprising PEEK and/or titanium structures with radiolucent markers (such as tantalum pins and/or spikes) selectively placed in the implant to provide a medical practitioner with placement and/or sizing information when the expandable spinal implant 100, 200, 300 is placed in the spine. The components of the expandable spinal implant system may be formed using a variety of subtractive and additive manufacturing techniques, including, but not limited to machining, milling, extruding, molding, 3D-printing, sintering, coating, vapor deposition, and laser/beam melting. Furthermore, various components of the expandable spinal implant system may be coated or treated with a variety of additives or coatings to improve biocompatibility, bone growth promotion or other features. For example, the endplates 110, 120, may be selectively coated with bone growth promoting or bone ongrowth promoting surface treatments that may include, but are not limited to: titanium coatings (solid, porous or textured), hydroxyapatite coatings, or titanium plates (solid, porous or textured).

The expandable spinal implant system may be employed, for example, with a minimally invasive procedure, including percutaneous techniques, mini-open and open surgical techniques to deliver and introduce instrumentation and/or one or more spinal implants at a surgical site within a body of a patient, for example, a section of a spine. In some embodiments, the expandable spinal implant system may be employed with surgical procedures, as described herein, and/or, for example, corpectomy, discectomy, fusion and/or fixation treatments that employ spinal implants to restore the mechanical support function of vertebrae. In some embodiments, the expandable spinal implant system may be employed with surgical approaches, including but not limited to: anterior lumbar interbody fusions (ALIF), posterior lumbar interbody fusion (PLIF), oblique lumbar interbody fusion, transforaminal lumbar interbody fusion (TLIF), various types of anterior fusion procedures, and any fusion procedure in any portion of the spinal column (sacral, lumbar, thoracic, and cervical, for example).

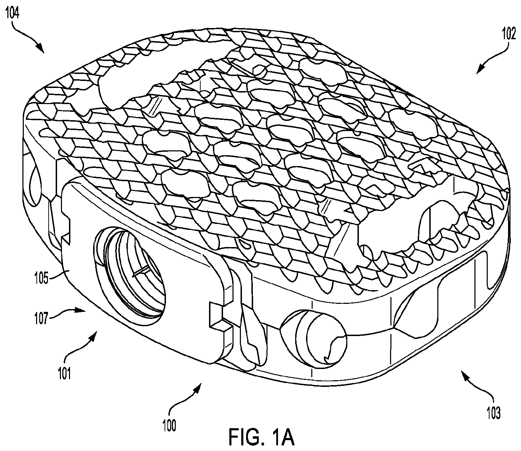

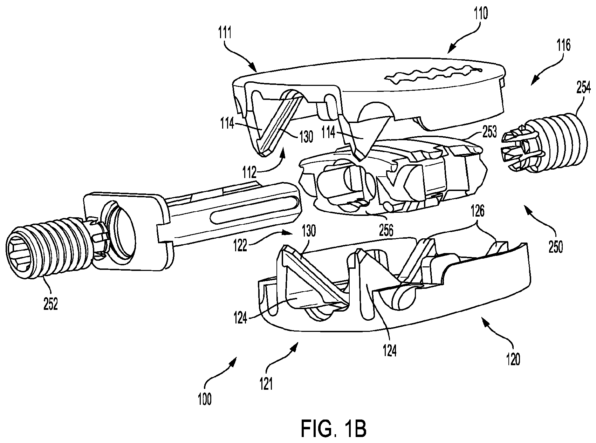

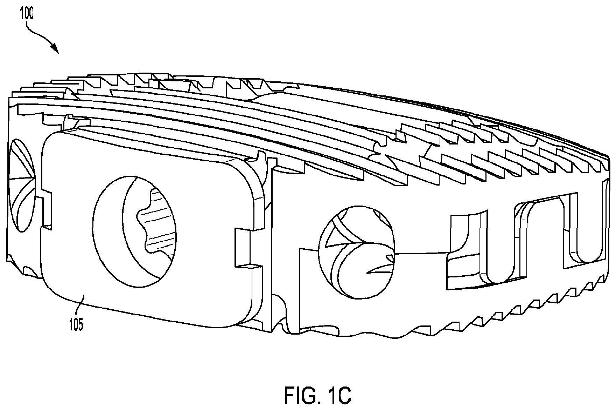

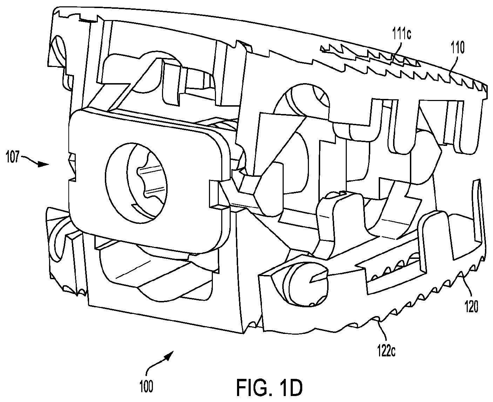

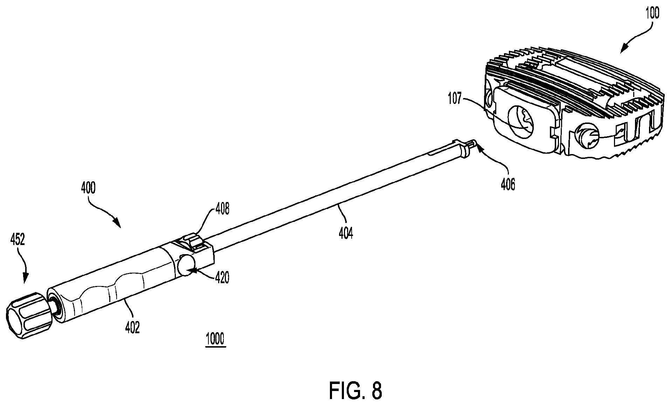

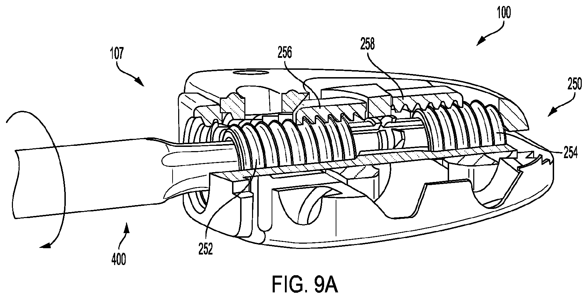

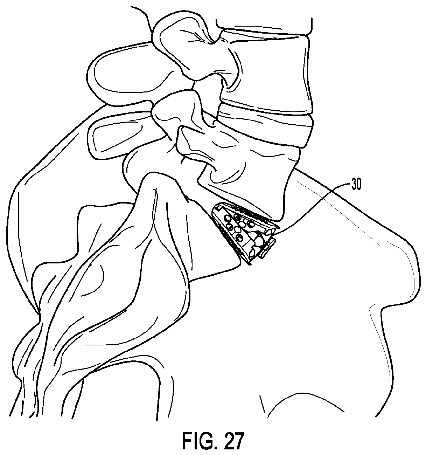

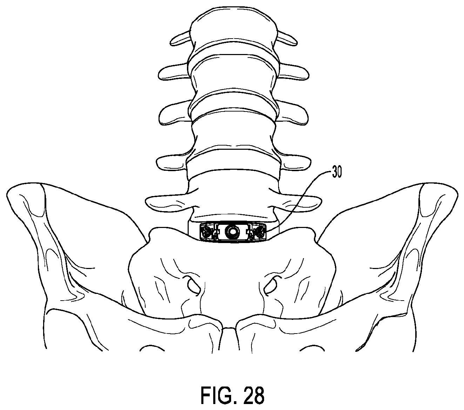

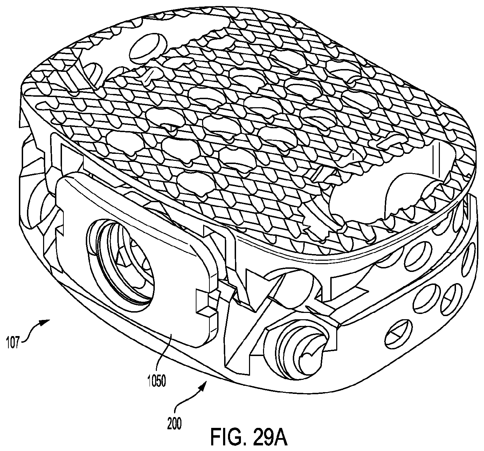

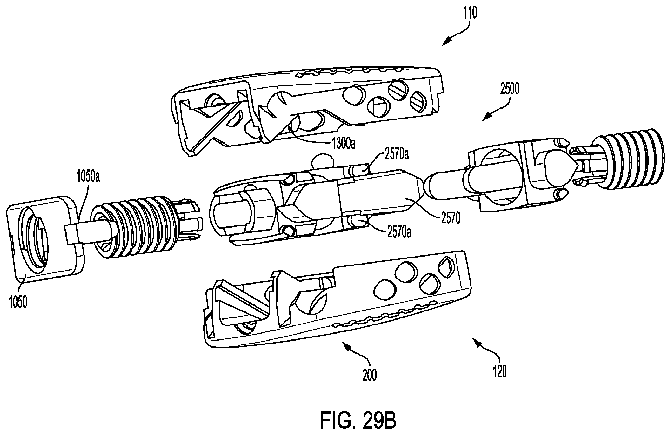

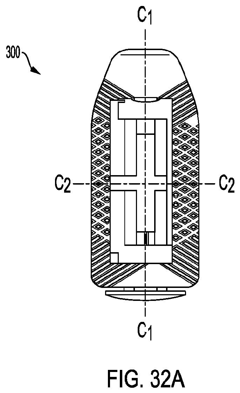

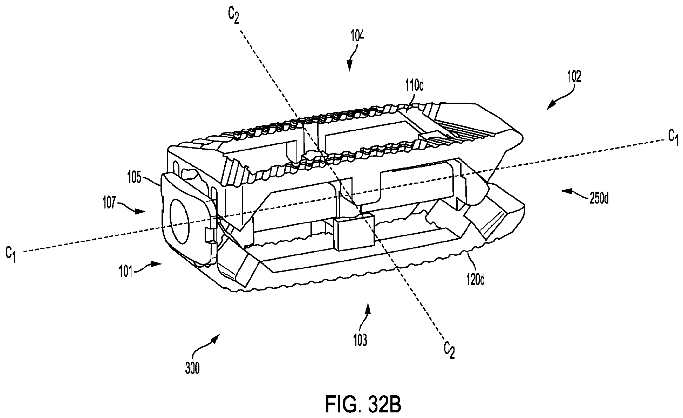

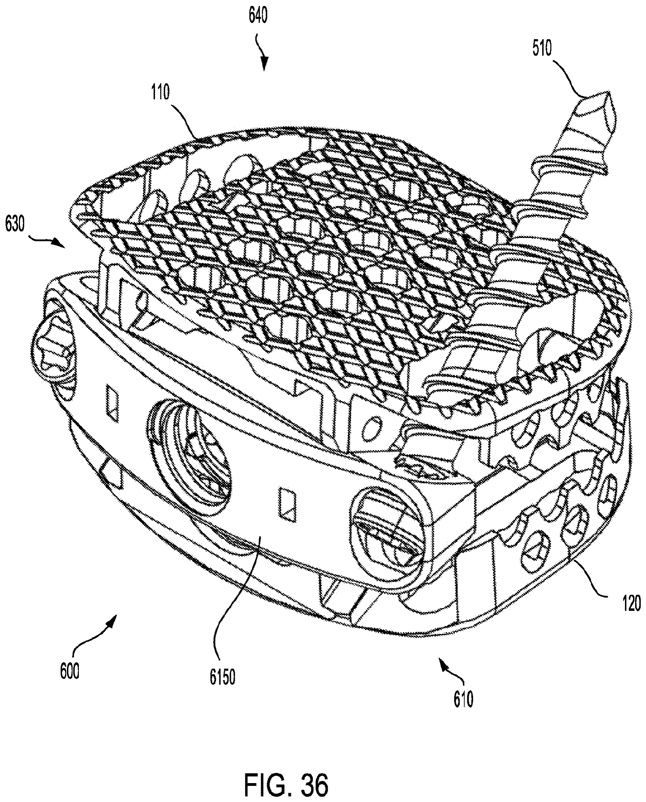

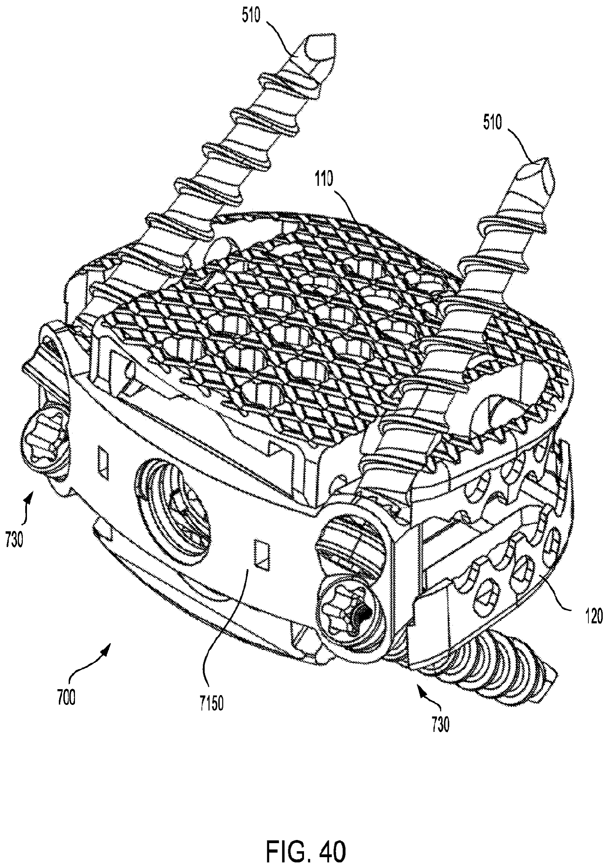

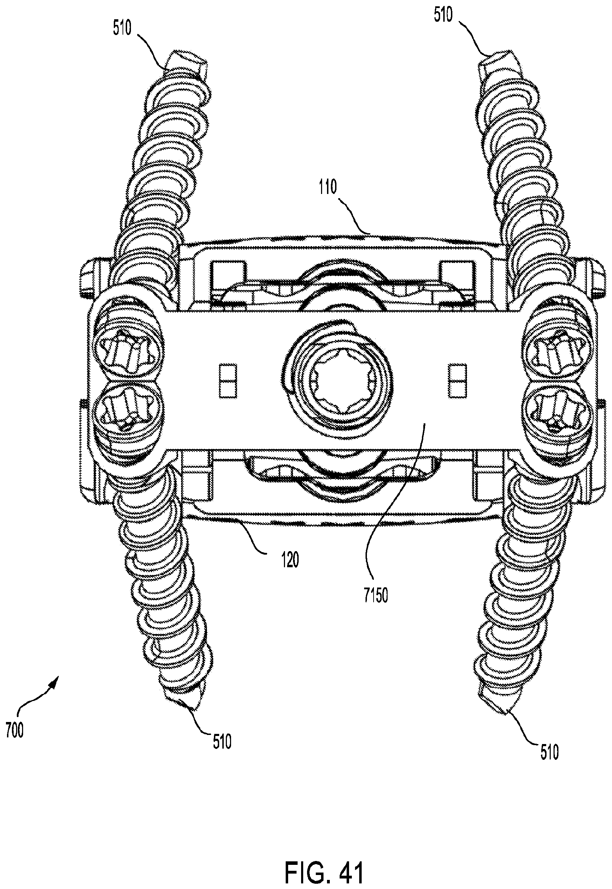

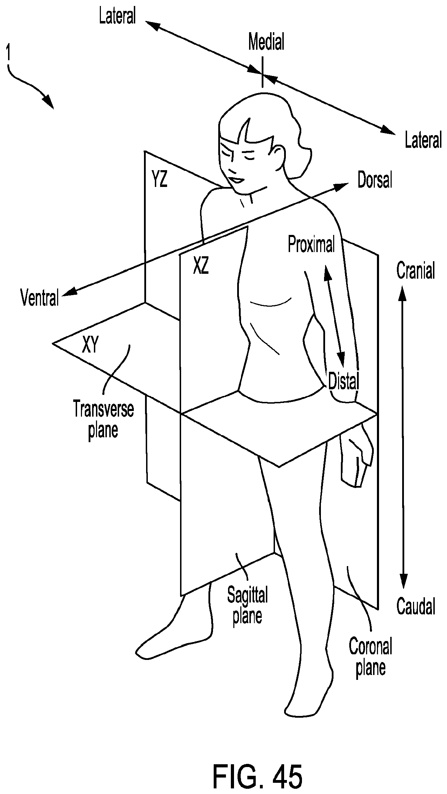

Generally in FIGS. 1-44B, five exemplary embodiments of an expandable spinal implants 100, 200, 300, 600, and 700 are shown (spinal implant 100 is highlighted in exemplary FIGS. 1-28, implant 200 is highlighted in exemplary FIGS. 29-31, implant 300 is highlighted in exemplary FIGS. 32-35, implant 600 is highlighted in exemplary FIGS. 36-39B, implant 700 is highlighted in FIGS. 40-44B). Exemplary embodiments of surgical tools 400, 450, and 500 are highlighted in exemplary FIGS. 8, 18-23C and disclosed in conjunction with an inter-body spinal implant system. For example, surgical tools 400, 450, and 500 are discussed concurrently with exemplary spinal implant 100. It shall be understood that the same or similar surgical tools highlighted in exemplary FIGS. 8, 18-23C may be employed with expandable spinal implants 200, 300, 600, and 700. Similar and/or identical numbering of corresponding elements may be used interchangeably between the various exemplary embodiments of an expandable spinal implants 100, 200, 300, 600, and 700 for ease of understanding and convenience in explanation. For example, moving mechanism 250 is predominately discussed concurrently with exemplary spinal implant 100 and is highlighted in exemplary FIGS. 9A-15 although the same or similar moving mechanism 250 may be employed with expandable spinal implants 200, 300, 600, and 700. FIG. 45 is provided solely as a reference illustration showing a patient 1 and various standard medical terms and orientations with respect to cardinal directions and planes of the body of patient 1 in which expandable spinal implants 100, 200, 300, 600, and 700 may act.

Referring generally to FIGS. 1-28 a first exemplary expandable spinal implant 100, moving mechanism 250, first surgical tool 400, and second surgical tool 500 are illustrated. Spinal implant 100 may be configured to be inserted in an intervertebral disc space between adjacent vertebral bodies accordingly to a variety of surgical techniques, *e.g.,* anterior techniques, oblique techniques, and lateral techniques.

FIG. 1A shows the spinal implant 100 in a perspective view and FIG. 1B shows the spinal implant 100 in an exploded parts view. Exemplary spinal implant 100 includes a top endplate 110 (first endplate) and a bottom endplate 120 (second endplate) and a moving mechanism 250, which will be described in greater detail below. Spinal implant 100 includes a proximal end 101 and a distal end 102 opposite the proximal end 101, and a first lateral end 103 and a second lateral end 104 opposite the first lateral end 103. The first and second lateral ends 103, 104 extend between the proximal end 101 and the distal end 102. The proximal end 101 includes an exposed screw guide endplate 105 defining a corresponding screw guide aperture 107, which are disposed between endplates 110 and 120. The screw guide endplate 105 and guide aperture 107 will be described in greater detail below.

Top endplate 110 may include a first outside surface 111 and a first inside surface 112 opposite the first outside surface 111. Similarly, bottom endplate 120 may include a second outside surface 121 and a second inside surface 122. The outside surfaces 111, 121 may be configured to be positioned between and/or contact vertebral bodies in a patients spine and have various surface characteristics. For example, in some embodiments, outside surfaces 111 and 122 may have a substantially linear surface profiles extending across faces of textured surfaces thereof. In other embodiments, outside surfaces 111 and 122 may have curved surface profiles extending across faces of textured surfaces thereof. Further details of endplates 110, 120 will be described in greater detail below. Inside surfaces 111, 122, may surround moving mechanism 250 and have various contours, guides, cavities, and other operable characteristics that facilitate movement and/or provide mechanical advantage to other operable and movable corresponding parts to facilitate contraction, angular adjustment, lateral bending, absorption of compression forces, shear forces, etc. as will be explained in greater detail below.

In the exemplary embodiment, top endplate 110 includes a pair of first proximal ramps 114 and a pair of first distal ramps 116 opposite the first proximal ramps 114. Each ramp of the first proximal ramps 114 includes an inclined surface extending away from inside surface 112 and moving mechanism 250. Similarly, each ramp of first distal ramps 116 includes an inclined surface extending away from inside surface 112 and moving mechanism 250. Bottom endplate 120 includes a pair of second proximal ramps 124 and a pair of second distal ramps 126 opposite the second proximal ramps 124. Each ramp of the second proximal ramps 124 includes an inclined surface extending away from inside surface 122 and moving mechanism 250. Similarly, each ramp of second distal ramps 126 includes an inclined surface extending away from inside surface 112 and moving mechanism 250. Furthermore, each ramp 114, 116, 124, 126 includes a corresponding guide wall 130 extending along an inside surface thereof and extending in a direction substantially parallel to the inclined surface of the corresponding ramp.

Exemplary spinal implant 100 includes a moving mechanism 250 that is operably coupled to top endplate 110 and bottom endplate 120 as will be explained in greater detail below. Moving mechanism 250 includes a first set screw 252 and a corresponding first trolley 256 operably coupled thereto, and a second set screw 254 and a corresponding second trolley 258 operably coupled thereto. A first functional feature of moving mechanism 250 is that it is further configured to increase and decrease a spacing between the top and bottom endplates 110, 120 upon simultaneous rotation of the first and second set screws 252, 254 in a clockwise and counterclockwise direction, respectively. A second functional feature of moving mechanism 250 is that it is further configured to increase and decrease an angle of inclination between the top and bottom endplates 110, 120 upon rotation of the first set screw 252 in a clockwise and counterclockwise direction, respectively. Additional functions and attributes of moving mechanism 250 will be described in greater detail below.

FIG. 1C is a perspective view of spinal implant 100 in a contracted position and FIG. 1D is a perspective view of spinal implant 100 in an expanded position. In the contracted position of FIG. 1C, top endplate 110 and bottom endplate 120 are contracted to a fully closed position. In the expanded position of FIG. 1B, top endplate 110 and bottom endplate 120 are expanded to a mid-way position, *i.e.,* endplates 110 and 120 can additionally expand if desired. In some embodiments, top endplate 110 may be referred to as an anterior wedge or anterior endplate and bottom endplate 120 may be referred to as a posterior wedge or posterior endplate.

As explained above, spinal implant 100 includes a proximal end 101 and a distal end 102 opposite the proximal end 101, and a first lateral end 103 and a second lateral end 104 opposite the first lateral end 103. It shall be understood that reference to other parts of spinal implant 100 may be in terms of the above orientation with reference to spinal implant 100 generally, *e.g.,* endplate 110 may also include a proximal end 101 and a distal end 102 opposite the proximal end 101, and a first lateral end 103 and a second lateral end 104 opposite the first lateral end 103.

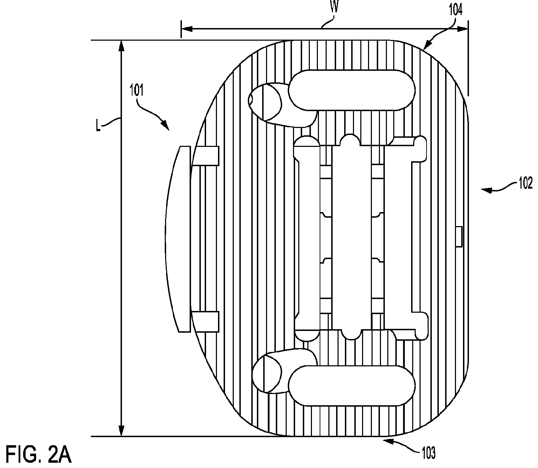

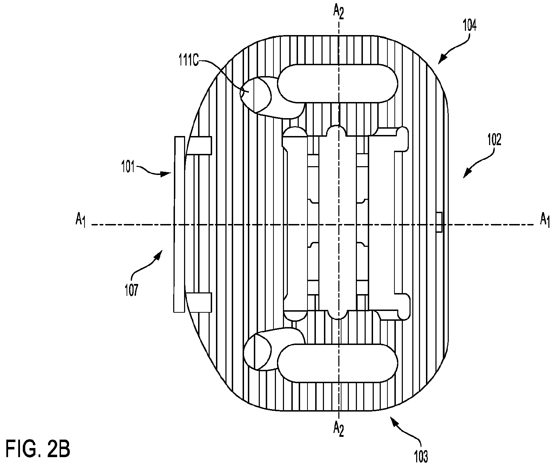

FIGS. 2A and 2B illustrate a top down view of spinal implant 100. Spinal implant 100 has a length L and a width W predominately defined by a footprint of endplates 110, 120. Spinal implant 100 has a first reference axis A₁ and a second reference axis A₂. First reference axis A₁ may be understood as a projection passing through a central portion of guide aperture 107 in a direction parallel to an end surface of first and second lateral ends 103, 104, *e.g.,* first reference axis A₁ may pass through the center of spinal implant 100 in a width wise direction. Second reference axis A₂ may be understood as a projection intersecting first reference axis A₁ and passing through the center of spinal implant 100 in a length wise direction. Top endplate 110 may have a plurality of channels 111c spaced apart from one another and extending in a length wise direction thereof, *e.g.,* in a direction parallel with reference axis A₂. Similarly, bottom endplate 120 may have a plurality of channels 122c spaced apart from one another and extending in a length wise direction thereof. *e.g.,* in a direction parallel with reference axis A₂. In the exemplary embodiment, channels 111c, 122c may each have an inclined edge portion that assists with positioning the spinal implant 100 between vertebral bodies and provides a surface for promoting bone growth thereon.

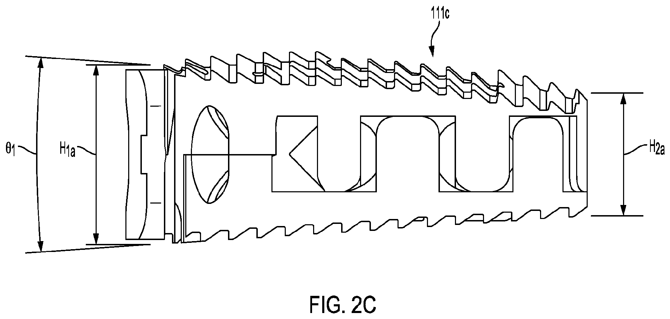

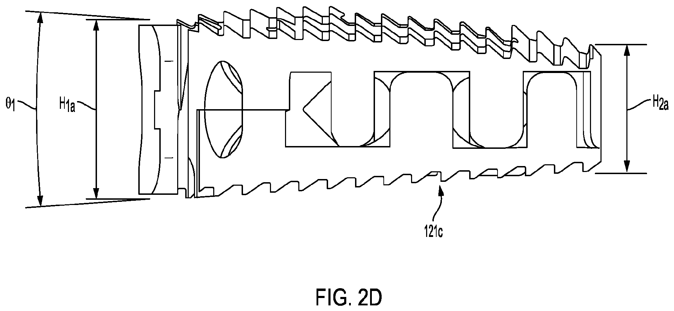

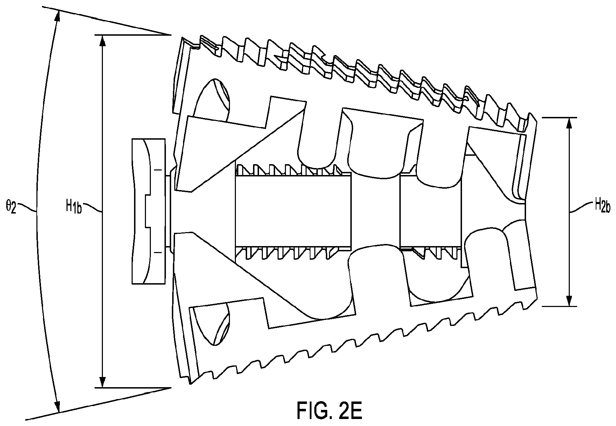

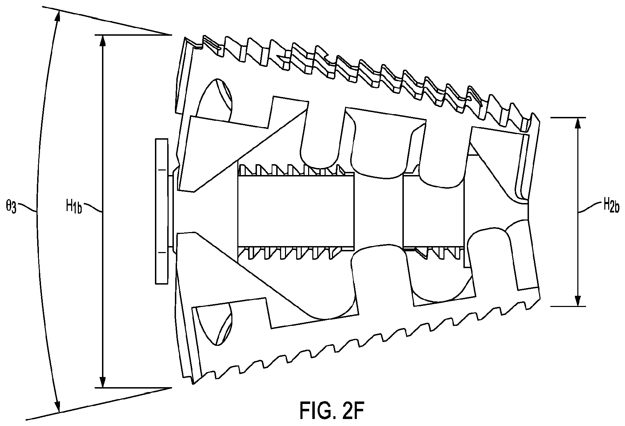

FIGS. 2C and 2D illustrate spinal implant 100 in a side view in a contracted position and FIGS. 2E and 2F illustrate spinal implant 100 in a side view in an expanded position. It shall be understood that FIGS. 2C-2F schematically illustrate spinal implant 100 with some internal parts being illustrated or simplified and others being omit for ease of explanation. For example, FIGS. 2C-2F are illustrated schematically solely to assist in explaining various positions of first and second endplates 110, 120 with respect to one another. In the contracted position, a first height H_{1A} of proximal end 101 may be about 10 mm and in the expanded position a second height H_{1B} of proximal end 101 may be about 22 mm. In the contracted position, a first height H_{2A} of distal end 102 may be about 7 mm and in the expanded position a second height H_{2B} of distal end 102 may be about 12 mm. Additionally, in the contracted position, a first angle of inclination θ₁ between endplates 110, 120 may be about 7° and in the expanded position a second angle of inclination θ₂ between endplates 110, 120 may be about 25°. Although specific ranges are provided herein with reference to exemplary spinal implant 100, other embodiments may have alternate corresponding dimensions, *i.e.,* height, from those provided above. Likewise, other embodiments may have alternate corresponding angles of inclination between endplates 110, 120.

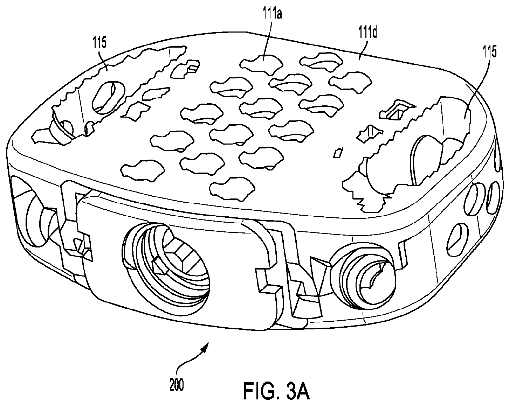

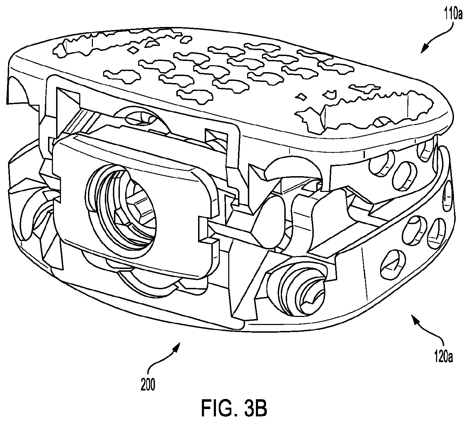

FIGS. 3A and 3B are perspective view of an alternate embodiment of a second spinal implant 200. Spinal implant 200 may have the same characteristics or similar characteristics as spinal implant 100. As illustrated, spinal implant 200 includes a top patterned endplate 110a and a bottom patterned endplate 120a. Top patterned endplate 110a includes an outside surface 111 and an inside surface 112 opposite the outside surface 111. Similarly, bottom patterned endplate 120a includes a first outside surface 121 and a first inside surface 122 opposite the outside surface 111. As illustrated, the outside surface 111 includes a plurality of raised diamond shaped surfaces 111d (a diamond tread pattern) and a plurality of first openings 111a that may each have a diamond like shape, a circular shape, and/or a diamond like shape including chamfered or rounded corners. Although not visible in FIGS. 3A and 3B, it shall be understood that bottom patterned endplate 120a may also have a plurality of raised diamond shaped surfaces and a plurality of openings the same as or similar to the plurality of raised diamond shaped surfaces 111d and the plurality of first openings 111a of top patterned endplate 110a.

As illustrated, the plurality of first openings 111a are circular and disposed in a central region of top patterned endplate 110a, although they may have alternate shapes and/or be disposed in alternate locations in other embodiments. For example, first and second outside surfaces 111 and 122 may comprise various anti-migration, anti-expulsion, and/or osseointegration features including, but not limited to: ridges, teeth, pores, and coatings (including but not limited to porous titanium coatings such as those provided on Capstone PTCTM implants available from Medtronic). The endplates 110a, 120a may further comprise at least one second opening 115 defined therein, and configured to allow bone growth materials to be packed, placed, or loaded into spinal implant 200. In the exemplary embodiment a pair of second openings 115 are shown with each having a D like shape.

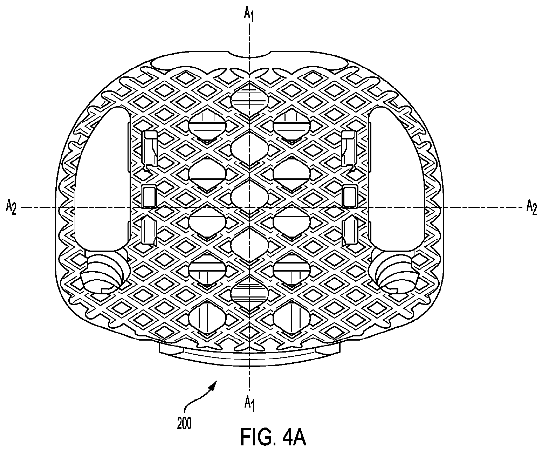

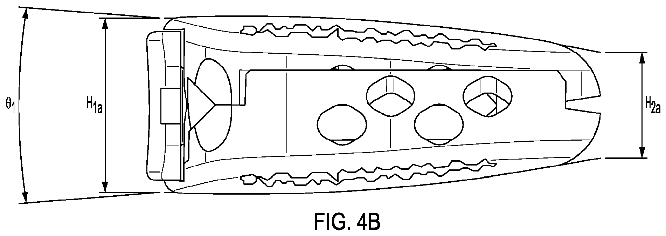

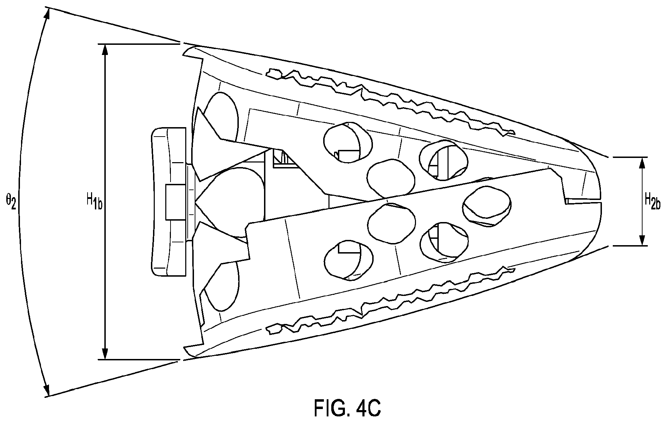

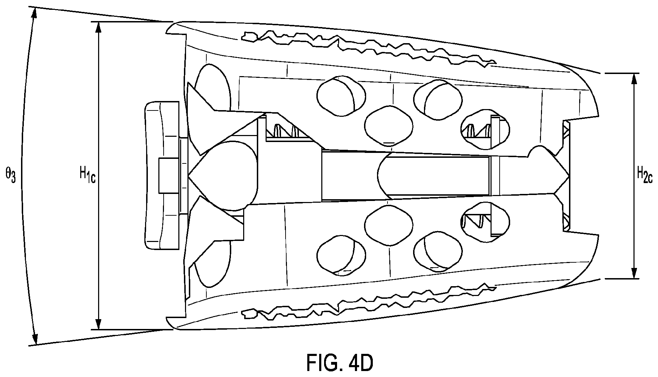

FIG. 4A illustrates spinal implant 200 in a top down view and each of FIGS. 4B-4D illustrate spinal implant 200 in a side view in a different respective position. FIG. 4B illustrates spinal implant 200 in a first position, FIG. 4C illustrates spinal implant 200 in a second position and FIG. 4D illustrates spinal implant 200 in a third position. In the first position, a first height H_{1A} of proximal end 101 may be about 10 mm, in the second position a second height H_{1B} of proximal end 101 may be about 18 mm, and in the third position a third height H_{1C} of proximal end 101 may be about 18 mm. In the first position, a first height H_{2A} of distal end 102 may be about 6 mm, in the second position a second height H_{2B} of distal end 102 may be about 5 mm, and in the third position a third height H_{1C} of distal end 102 may be about 11.8 mm (approximately 12 mm). Additionally, in the first position, a first angle of inclination θ₁ between endplates 110a, 120a may be about 9°, in the second position a second angle of inclination θ₂ between endplates 110a, 120a may be about 30°, and in the third position a third angle of inclination θ₃ between endplates

110a, 120a may be about 13°. In some embodiments, the first position may correspond to a fully contracted position, the second position may correspond to a maximum inclination angle, and the third position may correspond to a fully expanded position. Although specific ranges are provided herein with reference to exemplary spinal implant 100, other embodiments may have alternate corresponding dimensions, *i.e.,* height, from those provided above. Likewise, other embodiments may have alternate corresponding angles of inclination between endplates 110a, 120a.

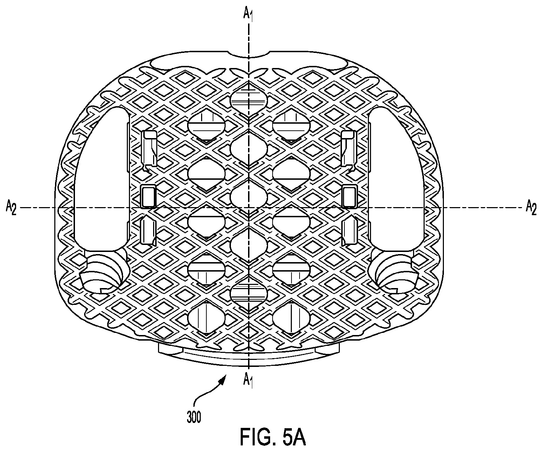

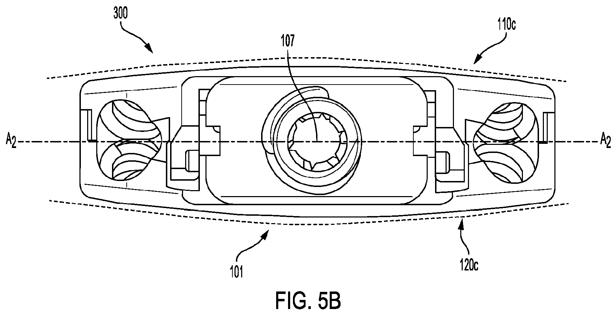

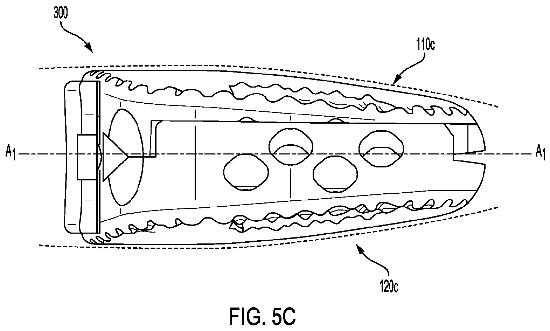

FIG. 5A is a top down view of a spinal implant 300. Spinal implant 300 may have the same characteristics or similar characteristics as spinal implant 200 and spinal implant 100. FIGS. 5B and 5C are alternate side views of the embodiment of FIG. 5A. As illustrated spinal implant 300 includes a first reference axis A₁ and a second reference axis A₂. First reference axis A₁ passes through the center of spinal implant 300 in a width wise direction and second reference axis A₂ passes through the center of spinal implant 300 in a length wise direction. First and second reference axes A₁ and A₂ may be understood as linear projections that are perpendicular with respect to one another. Additionally, first reference axis A₁ may pass through the center of guide aperture 107 and other components operably disposed therein, *e.g*., moving mechanism 250 as will be discussed in greater detail below.

As illustrated, spinal implant 300 includes a top curved endplate 110c and a bottom curved endplate 120c. The top curved endplate 110c features a concave surface profile with respect to the first and second reference axes A₁ and A₂ projecting thereunder. The concave surface profile is emphasized by the curved line thereabove. The bottom curved endplate 120 features a convex surface profile with respect to the first and second reference axes A₁ and A₂ projecting thereabove. The convex surface profile is emphasized by the curved line therebelow.

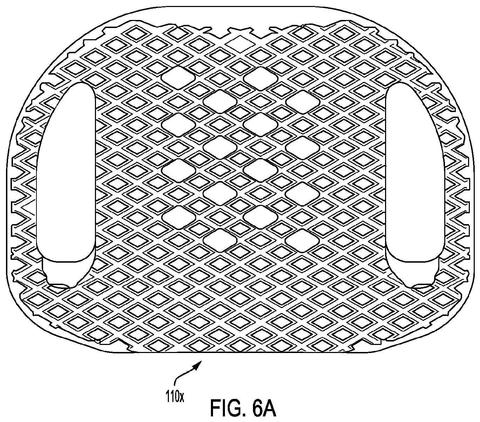

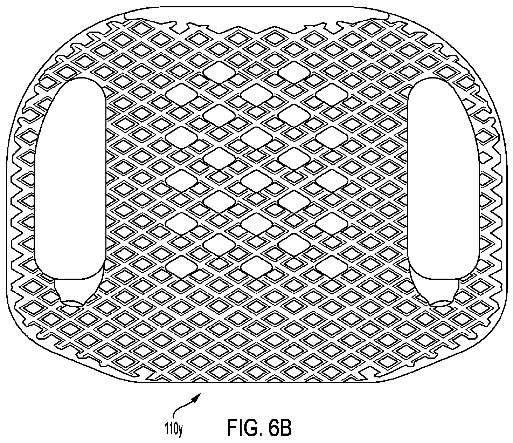

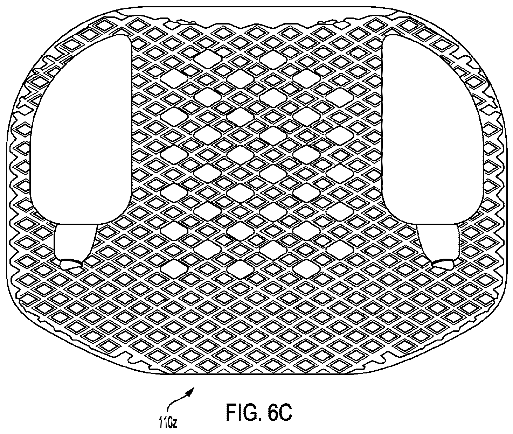

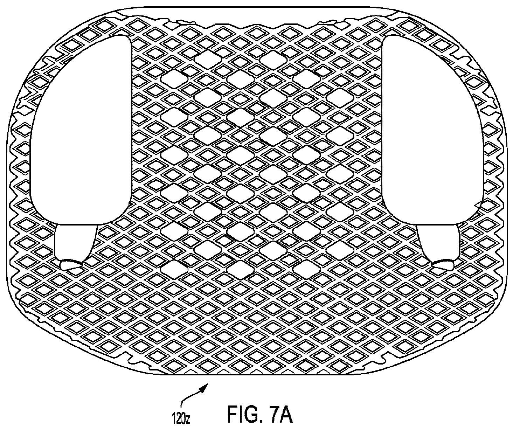

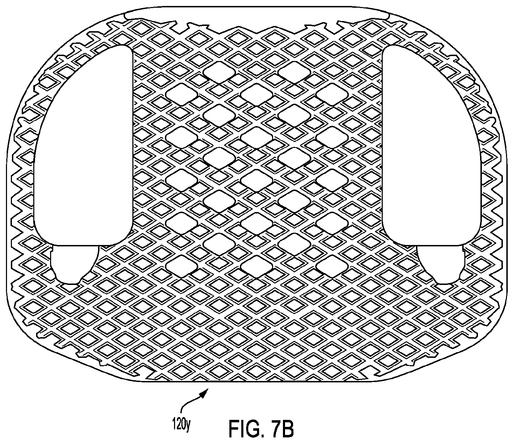

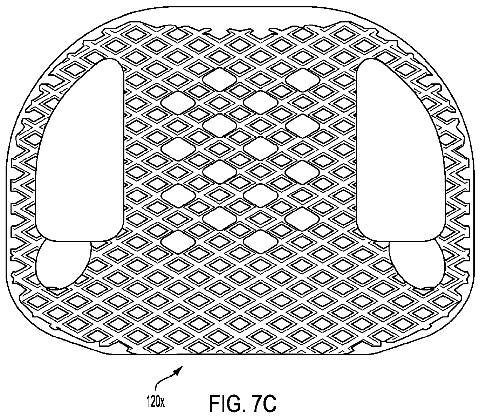

FIGS. 6A-6C are top down views of three exemplary footprint sizes of a first top endplate 110x, second top endplate 110y, and third top endplate 110z. It shall be understood that first, second, and third top endplates 110x, 110y, and 110z may be substituted for endplates 110, 110a, and 110c in accordance with the principles of the present disclosure. FIGS. 7A-7C are top down views of three exemplary footprint sizes of a first bottom endplate 120x, second bottom endplate 120y, and third bottom endplate 120z. It shall be understood that first, second, and third bottom endplates 120x, 120y, and 120z may be substituted for endplates 120, 120a, and 120c in accordance with the principles of the present disclosure. First top endplate 110x and first bottom endplate 120x may have a length of about 32 mm and a width of about 25 mm. Second top endplate 110y and second bottom endplate 120y may have a length of about 37 mm and a width of about 29 mm. Third top endplate 110z and third bottom endplate 120z may have a length of about 42mm and a width of about 32 mm. It shall be understood that first top endplate 110x and first bottom endplate 110y are suitable for patients with relatively small vertebrae, second top endplate 110y and second bottom endplate 110y are suitable for patients with relatively larger vertebrae than the previous example, and third top endplate 110z and third bottom endplate 110z are suitable for patients with relatively larger vertebrae than the previous two examples. In this way, spinal implants 100, 200, and 300 may be configured to have any of the exemplary footprint sizes explained above depending on a particular patient's vertebral anatomy. For example, as part of an initial assessment a surgeon may assess which of the available footprint sizes is best suited for a particular patient's vertebral anatomy. It shall be understood that the above exemplary footprint sizes are non-limiting exemplary embodiments and that other footprint sizes may be used with any of spinal implants 100, 200, 300 provided the chosen footprint size is suitable for a particular patient's anatomy. However, the three exemplary footprint sizes explained above are generally suitable for the majority of patients.

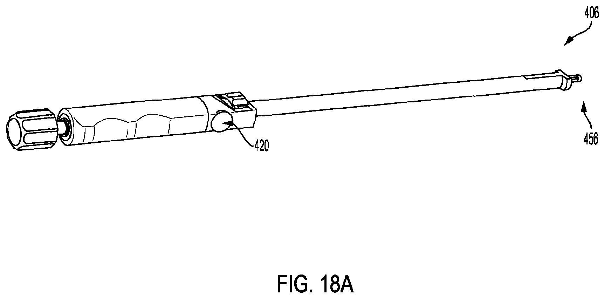

FIG. 8 is a perspective view of one embodiment of an expandable spinal implant system 1000 in accordance with the principles of the present disclosure. First surgical tool 400 includes a handle 402, shaft 404, tip 406, locking mechanism 408, and adjustment knob 452. Tip 406 is configured to be inserted inside of guide aperture 107 and operably connected to spinal implant 100. First surgical tool 400 is configured to perform a variety of functions for operably manipulating spinal implant 100. For example, first surgical tool 400 is configured to operably engage with spinal implant 100 via a secured connection such that a spinal implant 100 may be inserted between vertebral bodies of a patient according to anterior surgical techniques, oblique surgical techniques, and lateral surgical techniques. Additionally, first surgical tool 400 is configured to operably engage with spinal implant 100 to adjust spinal implant 100 from a contracted position to an expanded position and vice-versa. Furthermore, first surgical tool 400 is configured to operably engage with spinal implant 100 to adjust an angle of inclination between endplates 110, 120. Further still, spinal implant 100 may be adjusted in situ between vertebral bodies after spinal implant 100 is inserted into a patient. Additional attributes of the surgical tool will be disclosed below with reference to FIGS. 18A-19B

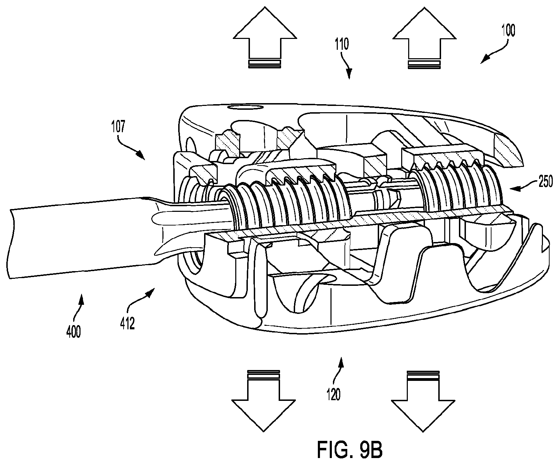

FIG. 9A is a cutout perspective showing first surgical tool 400 in a first adjustment position where the spinal implant 100 is in a contracted position and FIG. 9B is a cutout perspective showing first surgical tool 400 in the first adjustment position after adjusting the spinal implant 100 from the contracted position to a first expanded position. As illustrated, tip 406 is inserted through guide aperture 107 and into moving mechanism 250. Moving mechanism 250 includes a first set screw 252 and a second set screw 254 having respective internal cavities configured to operably receive tip 406. In some embodiments, first set screw 252 may be referred to as an anterior screw and second set screw 254 may be referred to as a posterior screw. The first and second set screws 252, 254 have a helical thread pitch that corresponds to keyed projections of first and second trolleys 256, 258, respectively. In the exemplary embodiment, the second set screw 254 has a reverse thread pitch and a shorter length than first set screw 252. In some embodiments, the thread pitch may be an M6 thread pitch, however other embodiments may have other thread pitches.

Each internal cavity of set screws 252, 254 comprises an internal circumferential surface that is keyed to the outside circumferential surface 456 of tip 406 of first surgical tool 400. For example, the outside circumferential surface 456 may resemble the geometry of the tip of a torx driver, hex driver, or the like and the internal circumferential surfaces of the first and second set screws 252, 254 may resemble the geometry of the cavity of the head of a torx screw, hex screw, or the like. In some embodiments, the internal circumferential surfaces of the first and second set screws 252, 254 may be configured for a Torx T20 driver or the like, however other embodiments may be differently sized. In other embodiments, the connection between the outer circumferential surface 456 and the inner circumferential surfaces of first and second set screws 252, 254 may comprise a variety of drive interfaces including but not limited to: multi-lobular drives; hexalobular drives; cross or Phillips head drives; straight or "flat head" drives; square or other polygonal drives; and/or combinations thereof. It shall be understood that any suitable geometrical shape or surface profile may be used by the exemplary embodiments disclosed herein provided the outside circumferential surface 456 is operably keyed to engage with the internal circumferential surfaces of the first and second set screws 252, 254.

In the exemplary embodiment, outside circumferential surface 456 is engaged with both the first and second set screws 252, 254 and when first surgical tool 400 is rotated in a first direction (clockwise direction) the outside circumferential surface 456 translates both set screws 252, 254 thereby causing the first and second trolleys 256, 258 to move away from one another in opposite directions. In turn, the first and second trolleys 256, 258 cause the top and bottom endplates 110, 120 to move apart from one another an equal amount in the expansion direction indicated by the arrows. The expansion direction may be a generally vertical direction projecting away from and perpendicular to the generally horizontal direction of a rotation axis of the moving mechanism. Likewise, when first surgical tool 400 is rotated in a second direction (counter-clockwise direction) the outside circumferential surface 456 translates both set screws 252, 254 thereby causing the first and second trolleys 256, 258 to move towards one another (not illustrated). In turn, the first and second trolleys 256, 258 urge the top and bottom endplates 110, 120 to move towards one another an equal amount in a contraction direction (not illustrated). The contraction direction may be a generally vertical direction projecting towards and perpendicular to the generally horizontal direction of the rotation axis of the moving mechanism. In summary, when positioning the first surgical tool 400 in the first position and rotating the first surgical tool 400 in either the first or second direction the moving mechanism 250 operably adjusts a spacing between the top and bottom endplates by simultaneous rotation of the first and second set screws 252, 254 along the rotation axis.

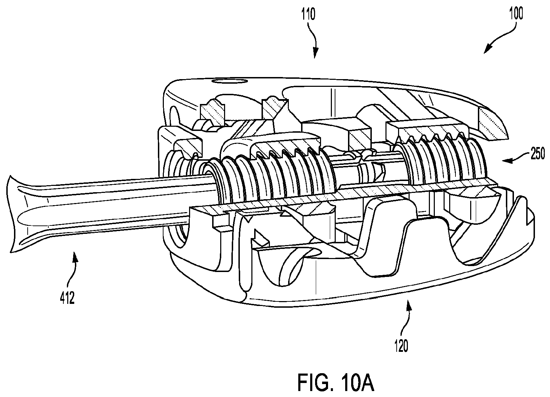

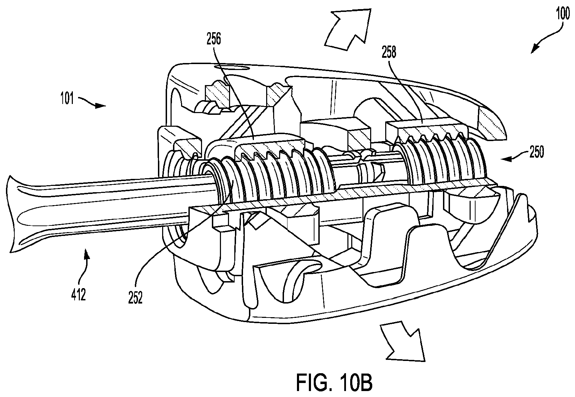

FIG. 10A is a cutout perspective showing first surgical tool 400 in a second adjustment position where the spinal implant 100 is in the first expanded position of FIG. 9B. As illustrated, first surgical tool 400 is retracted from moving mechanism 250 such that the outside circumferential surface 456 is only engaged with the first set screw 252, *i.e.,* first surgical tool 400 is in the second position. When first surgical tool 400 is in the second position and rotated in a first direction (clockwise direction) the outside circumferential surface 456 translates only the first set screw 252 thereby causing only the first trolley 256 to move towards the proximal end 101 of spinal implant 100 and allowing the second trolley 258 to remain stationary in place. In turn, the first trolley 256 urges the proximal end 101 of top and bottom endplates 110, 120 thereby causing top and bottom endplates 110, 120 to move apart from one another at the proximal end 101 in the direction shown by the arrows thereby increasing an angle of inclination between the top and bottom endplates 110, 120. Likewise, when first surgical tool 400 is in the second position and is rotated in the second direction (counter-clockwise direction) the outside circumferential surface 456 translates only the first set screw 252 thereby causing the first trolley 256 to move towards the stationary second trolley 258. In effect, the top and bottom endplates 110, 120 move towards one another at the proximal end 101 (not illustrated) thereby decreasing an angle of inclination between the top and bottom endplates 110, 120. In summary, when positioning the first surgical tool 400 in the second position and rotating the first surgical tool 400 in either the first or second direction the moving mechanism 250 operably adjusts an angle of inclination between the top and bottom endplates 110, 120 upon rotating the first set screw along the rotation axis.

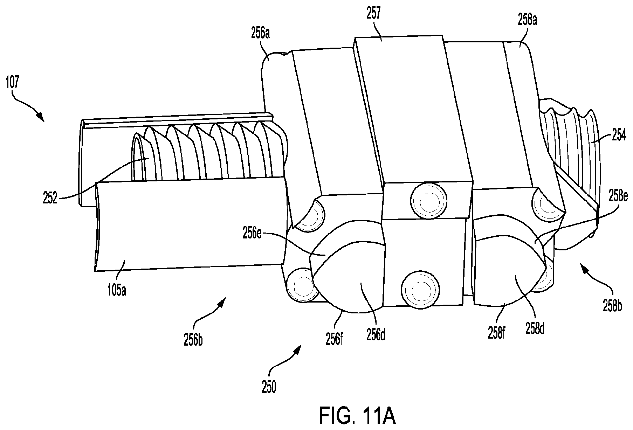

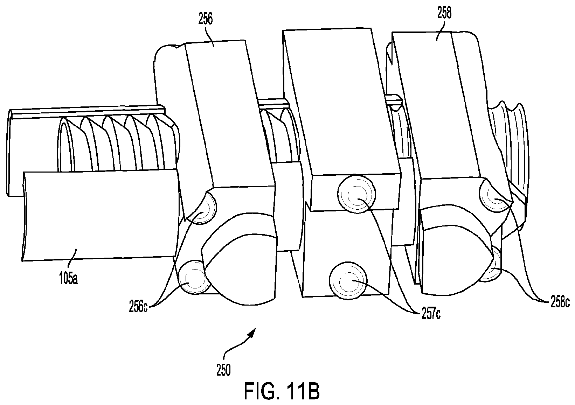

FIGS. 11A and 11B are perspective views of a moving mechanism 250 in a contracted position and an expanded position, respectively. Moving mechanism 250 is suitable for use in any exemplary embodiments disclosed herein. As illustrated moving mechanism 250 includes a screw guide housing 105a coupled to screw guide endplate 105 (not illustrated) and a central buttress block 257. Screw guide housing 105a may operably retain first and second screws 252, 254 therein and thereby define a rotation axis of moving mechanism 250 projecting in a longitudinal direction thereof. First and second trolleys 256, 258 are operably coupled to first and second set screws 252, 254 and are further configured to move along outside surfaces of screw guide housing 105a upon rotation of first and second set screws 252, 254.

First trolley 256 includes a first beveled edge 256a and a second beveled edge 256b opposite the first beveled edge 256a, the first and second beveled edges 256a, 256b are disposed on opposite sides of the rotation axis of the moving mechanism 250. Second trolley 258 includes a third beveled edge 258a and a fourth beveled edge 258b (not illustrated) opposite the third beveled edge 258a, the third and fourth beveled edges 258a, 258b are disposed on opposite sides of the rotation axis of the moving mechanism 250. Additionally, first trolley 256 has a first side surface and a second side surface opposite the first side surface, the first and second side surfaces being on opposite sides of the rotation axis of the moving mechanism 250. Likewise, second trolley 256 has a third side surface and a fourth side surface opposite the third side surface, the third and fourth side surfaces being on opposite sides of the rotation axis of the moving mechanism 250. Furthermore, buttress block 257 has a seventh and eighth side surface opposite the seventh side surface, the seventh and eighth side surfaces being on opposite sides of the rotation axis of the moving mechanism 250.

First trolley 256 includes a first plurality of projections 256c, the second trolley 258 includes a second plurality of projections 258c, and the buttress block 257 includes a third plurality of projections 257c. In the exemplary embodiment, first trolley 256 has two projections 256c projecting perpendicularly out from first side surface and two projections 256c projecting perpendicularly out from second side surface. Likewise, second trolley 258 has two projections 258c projecting perpendicularly out from third side surface and two projections 258c projecting perpendicularly out from fourth side. Furthermore, buttress block 257 has two projections 257c projecting perpendicularly out from seventh side surface and two projections 258c projecting perpendicularly out from eighth side surface. The first and second plurality of projections 256c, 258c may be conically shaped projections having a dome like shape or a hemispherical shape, for example. In the non-limiting exemplary embodiment, each projection of the first and second plurality of projections 256c, 258c comprises a hemispherical projection having a flat surface that coincides with a corresponding surface of one of the first through fourth beveled edges 256a, 256b, 258a, 258b. However, other embodiments may have other shapes and/or surface profiles as may be consistent with the disclosure herein.

First trolley 256 includes a first plurality of wedges 256d and second trolley 258 includes a second plurality of wedges 258d. For example, first trolley 256 includes a first wedge 256d projecting away from the first side surface in a transverse direction of the moving mechanism 250 and a second wedge 256d projecting away from the second side surface in the transverse direction of the moving mechanism. Likewise, second trolley 258 includes a third wedge 258d projecting away from the third side surface in a transverse direction of the moving mechanism 250 and a fourth wedge 258d projecting away from the fourth side surface in the transverse direction of the moving mechanism. In the exemplary embodiment, each wedge of the first plurality of wedges 256d includes a corresponding upper contact surface 256e and a corresponding lower contact surface 256f and each respective upper contact surface 256e meets a corresponding lower contact surface 256f at an apex point (not labeled). Likewise each wedge of the second plurality of wedges 258d includes a corresponding upper contact surface 258e and a corresponding lower contact surface 258f and each respective upper contact surface 258e meets a corresponding lower contact surface 258f at an apex point (not labeled). In the exemplary embodiment, each upper contact surface 256e, 258e and each lower contact surface 256f, 258f has a curved surface profile. For example, each upper contact surface 256e, 258e is concave with respect to a corresponding apex point and each lower contact surface 256f, 258f is convex with respect to a corresponding apex point.

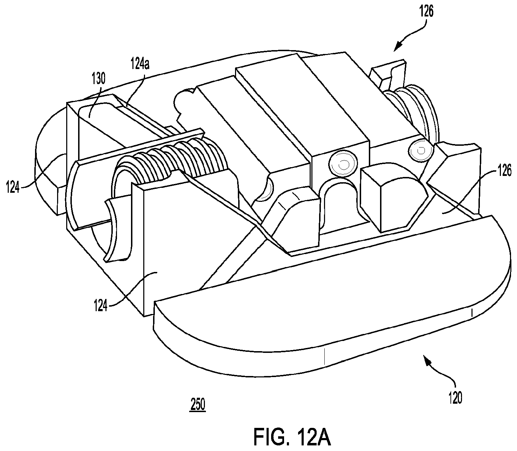

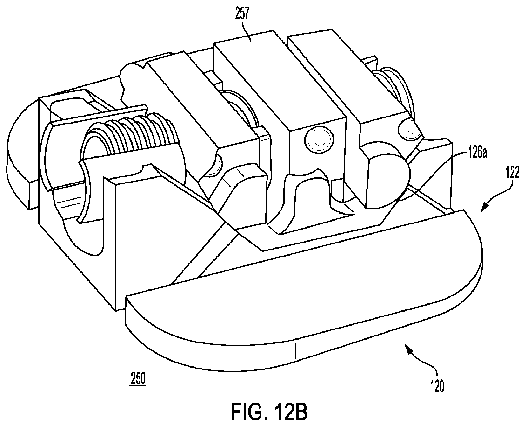

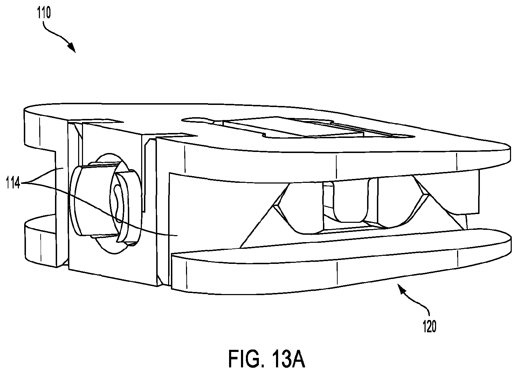

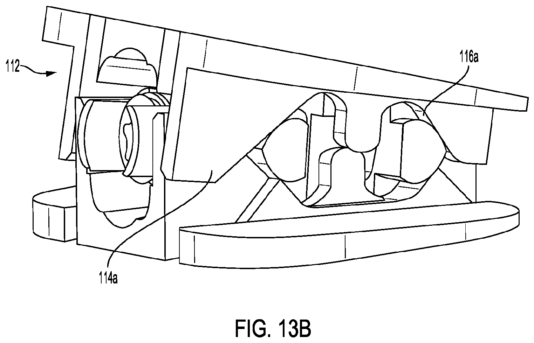

FIGS. 12A and 12B are perspective views of moving mechanism 250 of FIGS. 11A and 11B in the contracted position and the expanded position, respectively, shown with an exemplary bottom endplate 120. FIGS. 13A and 13B are perspective views of the moving mechanism 250 of FIGS. 12A and 12B in the contracted position and the expanded position, respectively, with a top endplate 110 and a bottom endplate 120. It shall be understood that FIGS. 12A-13B schematically moving mechanism 250 with some internal parts being illustrated or simplified and others being omit for ease of explanation. For example, FIGS. 12A-13B are illustrated schematically solely to assist in explaining operable characteristics of moving mechanism 250. FIGS 12A and 12B show bottom endplate 120 having a pair of second proximal ramps 124 and a pair of second distal ramps 126 disposed opposite the pair of second proximal ramps 124. Each ramp of second proximal ramps 124 may include a first inclined contact surface 124a extending away from buttress block 257 and inclined with respect to an inside surface 122 of endplate 120. Similarly, each ramp of second distal ramps 126 may include a second inclined contact surface 126a extending away from buttress block 257 and inclined with respect to an inside surface 122 of endplate 120. In the exemplary embodiment, the first inclined contact surfaces extend a first length (first distance) and the second inclined contact surfaces extend a second length (second distance) and the first length is greater than the second length.

FIGS 13A and 13B show top endplate 110 having a pair of first proximal ramps 114 and a pair of first distal ramps 116 disposed opposite the pair of first proximal ramps 114. Each ramp of first proximal ramps 114 may include a third inclined contact surface 114a extending away from buttress block 257 and inclined with respect to an inside surface 112 of endplate 110. Similarly, each ramp of first distal ramps 116 may include a fourth inclined contact surface 116a extending away from buttress block 257 and inclined with respect to an inside surface 112 of endplate 110. In the exemplary embodiment, the third inclined contact surfaces extend a third length (third distance) and the fourth inclined contact surfaces extend a fourth length (fourth distance) and the third length is greater than the fourth length.

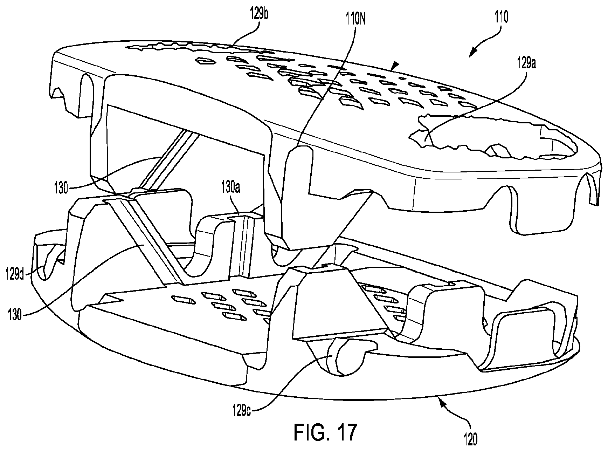

Each ramp of ramps 114, 116, 124, 126 may have an inside surface disposed adjacent to and facing the rotation axis of moving mechanism 250 and an outside surface opposite the inside surface and facing away from the rotation axis of moving mechanism 250. Additionally, each ramp of ramps 114. 116, 124, 126 may include a corresponding guide wall 130, which is best illustrated in FIGS. 12A and 17. Each guide wall 130 may extend along the inside surface of a corresponding ramp in a parallel direction to the corresponding contact surface. For example, with reference to FIGS. 12A-13B, guide wall 130 extends along the inside surface of proximal ramp 124 in a direction that is substantially parallel to first inclined contact surface 124a. As best understood with reference to FIGS. 12A-12B, each bottom most projection 256c of the first trolley 256 is disposed inside of a corresponding guide wall 130 of the second proximal ramps 124. Likewise, each bottom most projection 258c of the second trolley 258 is disposed inside of a corresponding guide wall 130 of the second distal ramps 126. Similarly, although not directly visible, in FIGS. 13A-13B each top most projection 256c of the first trolley 256 is disposed inside of a corresponding guide wall 130 of first proximal ramps 114. Likewise, each top most projection 258c of second trolley 258 is disposed inside of a corresponding guide wall 130 of first distal ramps 116.

With reference to FIGS 13A and 13B, when first surgical tool 400 is in the first position and translates first and second screws 252, 254 in the first direction the first and second trolleys 256, 258 move away from one another in opposite directions and the top endplate 110 and bottom endplate 120 move away from one another as the spinal implant 100 expands. For example, in some embodiments, beveled edges 256a, 256b of the first trolley 256 act against endplates 110, 120 at a proximal end 101 thereof and the first plurality of wedges 256d contact and slide along a corresponding ramp of the first and second first proximal ramps 114, 124. However, in other embodiments, 256e and 256f may act against inclined contact surface 124a in lieu of providing beveled edges 256a, 256b. In some embodiments, beveled edges 258a, 258b of the second trolley 258 act against endplates 110, 120 at a distal end 102 thereof and the second plurality of wedges 258d contact and slide along a corresponding ramp of the first and second first distal ramps 116, 126. However, in other embodiments, 258e and 258f may push against inclined contact surface 126a in lieu of providing beveled edges 256a, 256b. Additionally, each projection 256c of the first trolley 256 slides along a corresponding guide wall 130 of the first and second first proximal ramps 114, 124 and each projection 258c of the second trolley 258 slides along a corresponding guide wall 130 of the first and second distal ramps 116, 126. Furthermore, during the expansion of spinal implant 100 each projection 257c of buttress block 257 may slide vertically in a corresponding vertical guide wall 130a *(see* FIG. 17) of the top and bottom endplates 110, 120. In this way, the spinal implant 100 moves from a contracted position to an expanded position. It shall be understood that movement of spinal implant from the expanded position to the contracted position occurs in substantially the same way.

When first surgical tool 400 is in the second position and translates only the first screw 252 in the first direction the first trolley 256 moves away from buttress block 257 and stationary second trolley 258 and an angle of inclination between the top endplate 110 and bottom endplate 120 increases. For example, beveled edges 256c of first trolley 256 may push against endplates 110, 120 at a proximal end 101 thereof and/or the first plurality of wedges 256d may contact and slide along a corresponding ramp of the first and second first proximal ramps 114, 124 as explained above. Additionally, each projection 256c of the first trolley 256 slides along a corresponding guide wall 130 of the first and second first proximal ramps 114, 124 as explained above. The second trolley 258 remains stationary with beveled edges 258a. 258b remaining in contact with endplates 110, 120 at a distal end 102 thereof and the second plurality of wedges 258d remaining in contact with a corresponding ramp of the first and second distal ramps 116, 126. Due to first trolley 256 acting against endplates 110, 120 by moving away from buttress block 127 and second trolley 258 remaining stationary the second plurality of wedges 258d pivot along a corresponding ramp of the first and second distal ramps 116, 126 and each projection 258c of the second trolley 258 pivots and/or incrementally slides along a corresponding guide wall 130 of the first and second first distal ramps 116, 126. Furthermore, during the expansion of spinal implant 100 each projection 257c of buttress block 257 may slide vertically up and down in a corresponding vertical guide wall 130a *(see* FIG. 17) of the top and bottom endplates 110, 120 as necessary. In this way, a distance between endplates 110, 120 at the proximal end 101 is increased and a distance between endplates 110, 120 at the distal end 102 is minutely decreased thereby adjusting an angle of inclination between top endplate 110 and bottom endplate 120. Those with skill in the art, will appreciate that in disclosed exemplary embodiments first set screw 252 is longer than second set screw 254 thereby providing more room for travel of the first trolley 256 such that the first trolley 256 may enable a greater distance of travel between endplates 110, 120 at the proximal end 101 than second trolley 258 enables at the distal end 102.

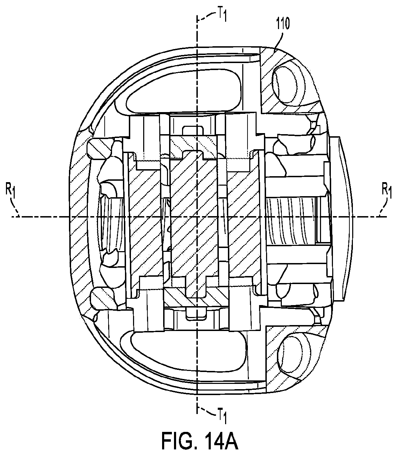

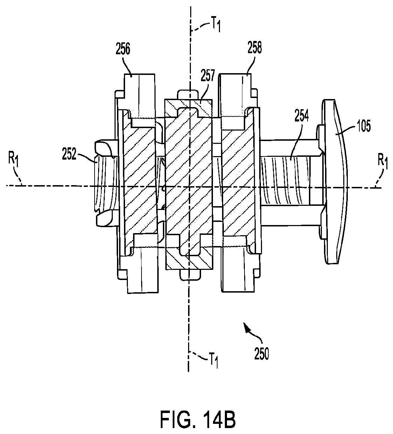

FIGS. 14A and 14B are cut-out views of a moving mechanism 250 in relation to a top endplate 110. As shown, moving mechanism 250 includes a rotation axis R₁ projecting in a longitudinal direction thereof and extending in a transverse direction of endplate 110 (from proximal side 101 to distal side 102). Rotation axis R₁ projects through the center of set screws 252, 254. Moving mechanism 250 includes a transverse axis T₁ intersecting a center of rotation axis and projecting perpendicular to rotation axis R₁ through buttress block 257.

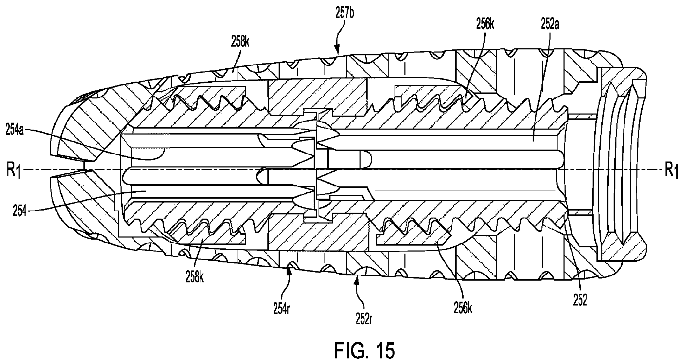

FIG. 15 illustrates a cross section of moving mechanism 250 taken along rotation axis R₁. As shown, first set screw 252 is operably coupled with first trolley 256 by a plurality of keyed projections 256k (thread pattern) that correspond to the pitch pattern of first set screw 252. Second set screw 254 is operably coupled with second trolley 258 by a plurality of keyed projections 258k (thread pattern). First set screw 252 includes a first internal circumferential surface 252a and second set screw 254 includes a second internal circumferential surface. The buttress block 257 includes an interior retention cavity 257b where a first retaining portion 252r of first set screw 252 and a second retaining portion 254r of second set screw 254 are retained. Interior retention cavity 257b may be an internal cavity spanning the inside circumference of buttress block 257 and configured to enable first set screw 252 and second set screw 254 to freely rotate along the rotation axis R₁ while preventing first set screw 252 and second set screw 254 from traveling in the longitudinal direction of moving mechanism 250.

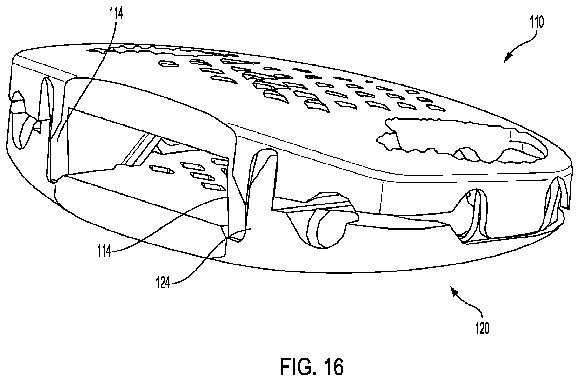

FIG. 16 is a perspective view of a top endplate 110 and bottom endplate 120 of spinal implant 100 and FIG. 17 is an exploded view of the top endplate 110 and bottom endplate 120 of FIG. 16. In the exemplary embodiment, when spinal implant 100 is in the closed position, inside surface 112 of top endplate 110 and inside surface 124 of bottom endplate 120 are nested or partially nested with respect to one another. For example, FIG. 16 shows first proximal ramps 114 of top endplate 110 inset from second proximal ramps 124 of bottom endplate 120. Additionally, top endplate 110 includes a first plurality of recesses 110n that allow corresponding components of bottom endplate 120a to nest inside of when spinal implant 100 is in the contracted position. For example, FIG. 16 shows second proximal ramps 124 nested inside of recess 110n. In some embodiments, recesses 110n may be referred to as nested recesses for convenience in explanation.

Top endplate 110 and/or bottom endplate 120 may optionally include at least one anchoring aperture 129. In the exemplary embodiment, top endplate 110 includes a pair of top anchoring apertures 129a, 129b, that pass through top endplate 110 at an inclined angle with respect to outside surface 111 of top endplate 110. Similarly, bottom endplate 120 includes a pair of bottom anchoring apertures 129c, 129d that pass through bottom endplate 120 at an inclined angle with respect to outside surface 121 of endplate 120. Each anchoring aperture 129 of the plurality of anchoring apertures 129a-129d is disposed adjacent an outside surface of a corresponding ramp 114, 116 however exemplary embodiments are not limited to the specific location shown in FIG. 17.

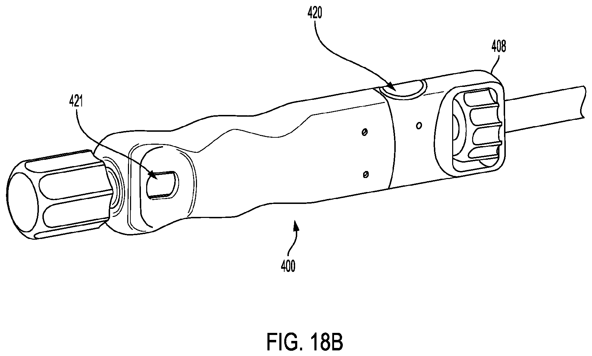

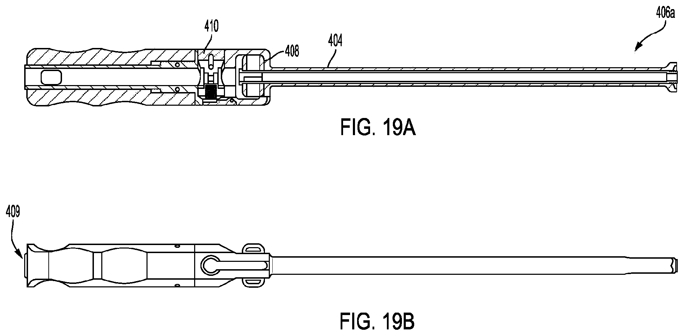

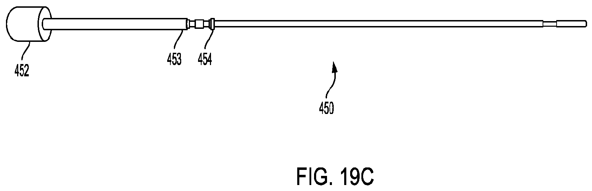

FIGS. 18A-18B are perspective views of a first surgical tool 400 of an adjustable spinal implant system in accordance with the principles of the present disclosure. FIGS. 19A-19B are side views of the first surgical tool 400 and a corresponding adjustment rod 450 configured for insertion inside of first surgical tool 400. Tip 406 is configured to connect to spinal implant 100 such that spinal implant 100 is securely attached to first surgical tool 400 by engaging locking mechanism 408. Similarly, tip 406 is configured to disconnect from spinal implant 100 such that spinal implant 100 is no longer securely attached to first surgical tool 400 by disengaging locking mechanism 408. For example, FIG. 19A shows tip 406 in a first locking position with tip grips 406a being expanded for gripping onto spinal implant 100 and FIG. 19B shows tip 406 in a second locking position with tip grips 406a being retracted. Locking mechanism 408 is configured to toggle between the first locking position and second locking position. In some embodiments, when locking mechanism 408 is engaged in the first locking position spinal implant 100 is fixedly coupled to first surgical tool 400 such that it will not rotate. This may be advantageous for initial positioning of spinal implant 100 between vertebral bodies during surgery. Additionally, first surgical tool 400 includes a positioning mechanism 410 configured to position adjustment rod 450 in a first position and a second position *(see* FIG. 19A). First surgical tool 400 may also include a push button 420 to toggle between positioning adjustment rod 450 in a first position to engage both first and second set screws 252, 254 and a second position to engage only the first set screw 252 *(see* FIG. 18B). Furthermore, in some embodiments first surgical tool 400 may include a window 421 to identify whether both first and second set screws 252 254 are engaged for parallel expansion/contraction of spinal implant 100 or whether only the first set screw 252 is engaged for adjusting an angle of inclination of spinal implant 100.

In the exemplary embodiment, first surgical tool 400 includes a central shaft aperture 409 extending through handle 402, shaft 404, and tip 406. Central shaft aperture 409 is configured to receive adjustment rod 450 therein such that adjustment knob 452 is rotatable therein and protrudes, at least partly, from both ends. Adjustment rod 450 includes an adjustment knob 452, first and second positioning surfaces 453, 454 and keyed circumferential surface 456. When adjustment rod 450 is positioned within central shaft aperture 409, adjustment knob 452 protrudes from one end and keyed circumferential surface 456 protrudes from the other end *(see* FIG. 14). With adjustment rod 450 inserted within central shaft aperture 409 positioning mechanism 410 can extend and retract adjustment rod 450 in the longitudinal direction of shaft 409. As explained above with respect to FIGS 13A and 13B, when first surgical tool 400 is in the first position, keyed circumferential surface 456 may rotate first and second set screws 252, 254 along the rotation axis and when first surgical tool 400 is in the second position, keyed circumferential surface 456 may rotate only the first set screw 252 along the rotation axis. In some embodiments, positioning mechanism 410 is configured to be toggled between a first position and a second position where it can act against positioning surfaces 453, 454 to extend and retract adjustment rod 450 in the longitudinal direction of shaft 409. For example, in the first position positioning mechanism 410 may extend adjustment rod 450 from tip 406 to an extended position where circumferential surface 456 may engage with internal circumferential surfaces of the first and second set screws 252, 254. In the second position, positioning mechanism 410 may retract adjustment rod 450 through tip 406 to a partially retracted position where circumferential surface 456 may only engage with internal circumferential surface of the first set screw 252. An internal gearing of positioning mechanism 410 may include internal locking pins and surfaces that act against positioning surfaces 453, 454 such that when an exposed turn dial knob of positioning mechanism 410 is turned to a particular position, the internal locking pins and surfaces act against the inclined and recessed surfaces of positioning surfaces 453, 454.

Additionally, in some embodiments, first surgical tool 400 may be configured to receive adjustment rods 450 of varying lengths having varying outside circumferential surfaces 456 and positioning surfaces 453, 454. For example, first surgical tool 400 may be configured to receive a first relatively shorter adjustment rod 450 optimized for use for a spinal implant 100 using corresponding relatively smaller endplates 110, 120 of FIGS. 6A -7C and a corresponding smaller moving mechanism 250 having a relatively shorter longitudinal axis optimized for such relatively shorter endplates 110x, 120x. For example still, first surgical tool 400 may be configured to receive a second relatively longer adjustment rod 450 optimized for use for a spinal implant 100 using corresponding relatively larger endplates 110z, 120z of FIGS. 6A -7C and a corresponding larger moving mechanism 250 having a relatively longer longitudinal axis optimized for such relatively longer endplates 110z, 120z.

Additionally, in some embodiments, first surgical tool 400 may be configured to receive multiple types of adjustment rods 450. In at least one embodiment, first surgical tool 400 may receive a first adjustment rod 450 with an outside circumferential surface 456 that is configured to engage (1) both the first and second set screws 252, 254 at the same time and (2) the first set screw 252. For example, the first adjustment rod 450 may be toggled between (1) a first position where outside circumferential surface 456 is fully extended and configured to engage both the first and second set screws 252, 254, and (2) a second position where outside circumferential surface 456 is partially extended (and/or partially retracted) to engage only the first set screw 252. In an alternate embodiment, first surgical tool 400 may receive a second adjustment rod 450 with an outside circumferential surface 456 that is configured to engage only one set screw 252, 254 at a time. For example, the outside circumferential surface 456 may have an engagement surface with a longitudinal length that corresponds to a single set screw 252, 254 such that it only engages with a single set screw 252, 254 at a time. For example, the second adjustment rod 450 may be toggled between (1) a first position where outside circumferential surface 456 is fully extended and configured to engage the second set screw 254 independently of the first set screw 252 and (2) a second position where outside circumferential surface 456 is partially extended (and/or partially retracted) to engage only the first set screw 252. At least one advantage of having first surgical tool 400 being configured to receive multiple types of adjustment rods 450 of varying lengths and having outside circumferential surfaces of different lengths is that a surgeon can quickly and easily select the appropriate adjustment rod 450. For example, a surgeon may select first adjustment rod 450 to expand/contract a spacing between endplates 110, 120 by the same or substantially the same amount while maintaining the angle of inclination between endplates 110, 120, i.e., by engaging both first and second set screws 252, 254. Additionally, a surgeon may select second adjustment rod 450 to selectively increase/decrease an angle of inclination between endplates of spinal implant 100 at the proximate side 101 and the distal side 102 independently, i.e., by only engaging one of first and second set screws 252, 254 at a time. For example still, the second adjustment rod 450 may be configured to adjust spinal implant 100 to enable anterior expansion separately from enabling posterior expansion which may enable spinal implant 100 to be placed in kyphosis as is consistent with above explained embodiments.

Furthermore, in some embodiments, first surgical tool 400 is configured to operate in three modes. In the first mode, tip grips 406a are securely connected to spinal implant 100. In the second mode, adjustment rod 450 may be positioned in a first position such that upon selective rotation of adjustment knob 452 a spacing between endplates 110, 120 selectively increase/decrease in minute increments. For example, by rotating each of first set screw 252 and second set screw 254. In the third mode, adjustment rod 450 may be positioned in a second position such that upon selective rotation of adjustment knob 452 an angle of inclination between endplates 110, 120 may selectively increase/decrease in minute increments. For example, by only rotating first set screw 252 an angle of inclination between endplates 110, 120 may increase/decrease by moving one side of the endplates 110,120 towards/away from each other and moving the opposite side of the endplates 110,120 in an opposite direction. In some embodiments, this may also happen by only rotating second set screw 254. For example, first surgical tool 400 may have a relatively short circumferential engagement surface 456 that will only engage a single one of the internal circumferential surfaces of first or second set 252, 254 at a time.

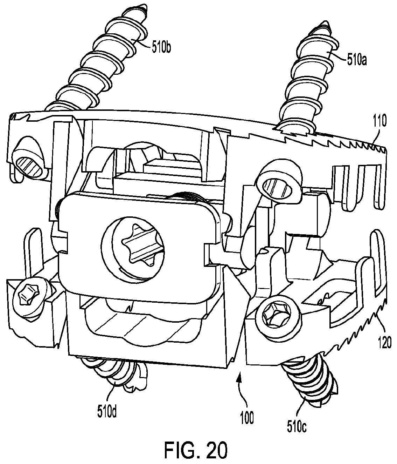

FIG. 20 illustrates a perspective view of one embodiment of an expandable spinal implant 100 including a plurality of anchoring screws 510. In some embodiments, anchoring screws 510 may be referred to as bone screws. In the exemplary spinal implant 100, top endplate 110 includes a first anchoring screw 510a, and a second anchoring screw 510b opposite the first anchoring screw 510a that each extend through a corresponding aperture. For example, first and second anchoring screws 510a, 510b pass through a corresponding aperture of top endplate 110 configured to orient them at an inclined angle with respect to outside surface 111 of top endplate 110. Similarly, bottom endplate 120 includes a third anchoring screw 510c, and a fourth anchoring screw 510d that each extend through a corresponding aperture. Anchoring screws 510c, 510d project from a proximal end 101 of spinal implant 100 at an inclined angle towards distal end 102. For example, third and fourth anchoring screws 510c, 510d pass through a corresponding aperture of bottom endplate 120 configured to orient them at an inclined angle with respect to outside surface 121 of bottom endplate 120. However, it shall be understood that in other embodiments at least one aperture may orient a corresponding anchoring screw 510a, 510b, 510c, 510d at any angle with respect to the corresponding endplate 110, 120 consistent with the disclosure herein. Anchoring screws 510a-510d are configured to anchor into corresponding adjacent vertebral bodies.

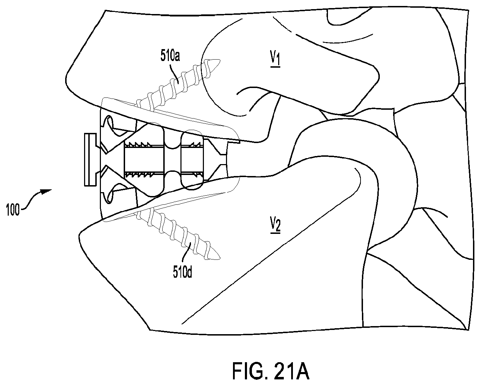

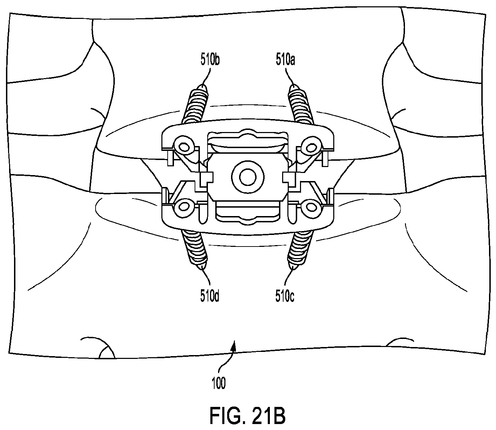

FIGS. 21A-21B illustrate a lateral side view and front side view, respectively, of one embodiment of an expandable spinal implant system in which anchoring screws 510a-510d are anchored into adjacent vertebral bodies. As illustrated, anchoring screws 510a, 510b project out from top endplate 110 of spinal implant 100 from a proximal end 101 at an inclined angle towards distal end 102 thereby anchoring into a top vertebral body V₁. Similarly, anchoring screws 510a, 510b project out from bottom endplate 120 of spinal implant 100 from a proximal end 101 at an inclined angle towards distal end 102 thereby anchoring into a bottom vertebral body V₂. As used herein, a pair of vertebral bodies, adjacent vertebral bodies, and/or first and second vertebral bodies may refer to, *e.g.,* top vertebral body V₁ and bottom vertebral body V₂.

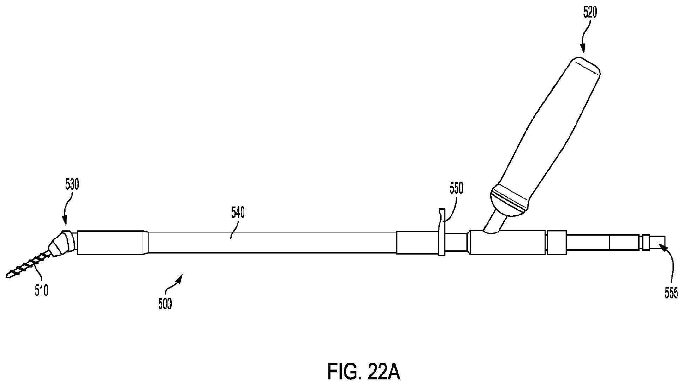

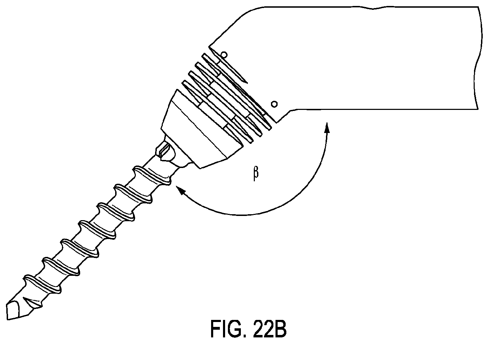

FIG. 22A is a side view of a second surgical tool 500 suitable for use with disclosed embodiments and systems herein, *e.g.,* to drive anchoring screws 510a-510d. FIG. 22B is a side view of an enlarged region of FIG. 22A. Exemplary, second surgical tool 500 includes a ratcheting drive shaft 555, a positioning handle 520, a tip portion 530, a drive shaft housing 540, and a trigger 550. Ratcheting drive shaft 555 may be configured to connect and disconnect with a ratcheting handle (not shown) and rotate within ratcheting drive shaft housing 540. For example, the drivable connection may comprise a variety of drive interfaces including but not limited to: multi-lobular drives; hexalobular drives; cross or Phillips head drives; straight or "flat head" drives; square or other polygonal drives; and/or combinations thereof. Positioning handle 520 may be configured to assist with maintaining and controlling the second surgical tool 500, *e.g.,* in view of torque transmitted through ratcheting drive shaft 555. Tip portion 530 is angled at a degree β with respect to a longitudinal direction of drive shaft housing 540. In some embodiments, tip portion 530 is angled such that the degree β corresponds to the inclination of anchoring screws 510a-510d and the inclination of anchoring aperture 129. For example, anchoring apertures 129 may be inclined about 30° - 50°, and more particularly about 40°, with respect to an outside surface 111, 121 of endplates 110, 120. This arrangement may be advantageous for driving anchoring screws 510a-510d while spinal implant 100 is positioned between adjacent vertebral bodies. Tip portion 530 may secure anchoring screw 510 in an internal cavity therein such that anchoring screw 510 may not disconnect during initial positioning of anchoring screw 510. For example, tip portion 530 may have a flexible elastic member configured to securely retain a head portion of anchoring screw 510. Tip portion 530 may, however, release anchoring screw 510 when anchoring screw is sufficiently anchored into an anatomical feature, such as a vertebrae for example. This feature may be particularly advantageous during surgery for maintaining the anchoring screw 510 in tip portion 530 such that anchoring screw 510 does not uncouple from tip portion 530 when initially positioning anchoring screw 510 in an anchoring aperture, for example anchoring aperture 129. Additionally, in some embodiments tip portion 530 is operably coupled with trigger 550 such that trigger 550 may disconnect anchoring screw 510 when anchoring screw 510 is installed. In some embodiments, trigger 550 may not be necessary because tip portion 530 may self-release anchoring screw 510 after installation.

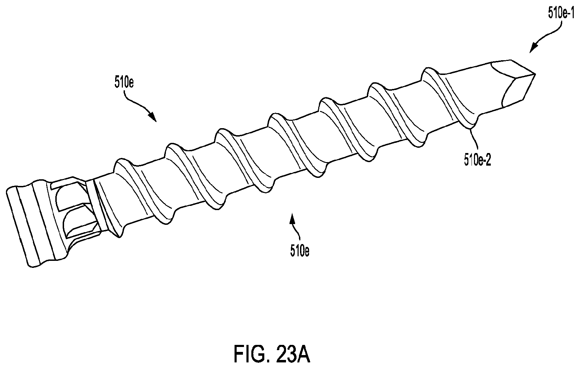

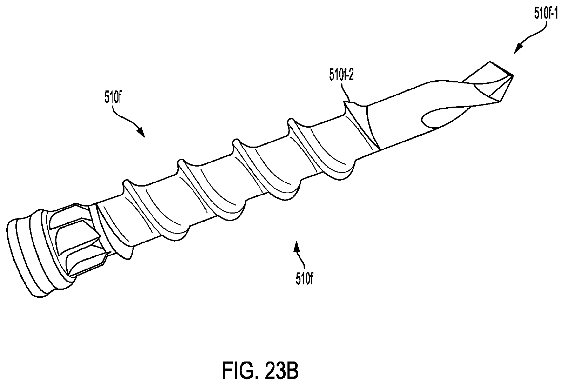

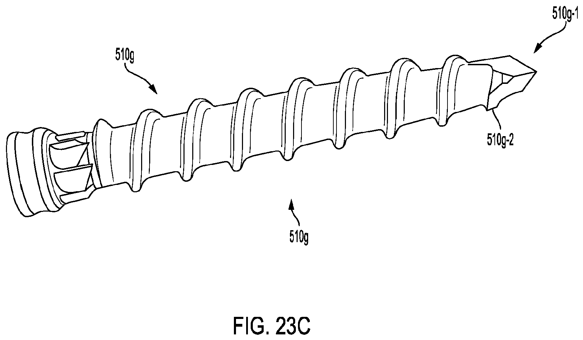

FIGS. 23A-23C are various perspective views of exemplary anchoring screws suitable for use with disclosed embodiments herein in conjunction with the second surgical tool 500. FIG. 23A shows a trocar tip anchoring screw 510e, FIG. 23B shows a flutes or fluted tip anchoring screw 510f, and FIG. 23C shows a speed anchoring screw 510g. Each anchoring screw 510e-510g may have a thread pitch and sizing that corresponds to a size of anchoring aperture 129. Trocar tip anchoring screw 510e includes an angled tip portion 510e-1 and a thread pattern including threads 510e-2. Threads 510e-2 may be spaced back from angled tip portion 510e-1 which may facilitate with aligning anchoring screw 510e with anchoring aperture 129. For example, in some embodiments, threads 510e-2 are spaced back about 3 mm from angled tip portion 510e-1. Fluted tip anchoring screw 510f includes a cutting tip 510f-1 and a thread pattern included threads 510f-2. Cutting tip 510f-1 may extend a relatively long distance from the beginning of threads 510f-2 such that the cutting tip 510f-1 may pre-drill into an adjacent vertebral body before the threads 510f-2 engage with anchoring aperture 129. For example, in some embodiments, threads 510f-2 are spaced back about 8 mm from cutting tip 510f-1. Speed anchoring screw 510g includes a conical tip 510g-1 and a thread pattern including threads 510g-2. Different from trocar tip anchoring screw 510e and fluted tip anchoring screw 510f, threads 510g-2 of speed anchoring screw 510g may begin immediately adjacent conical tip 510g-1.

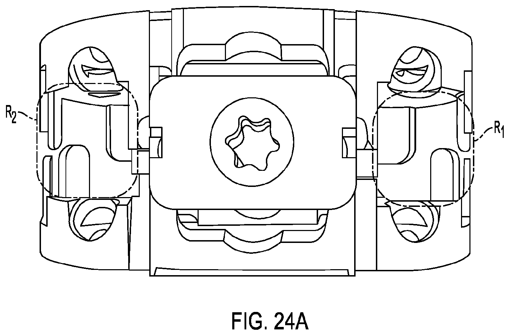

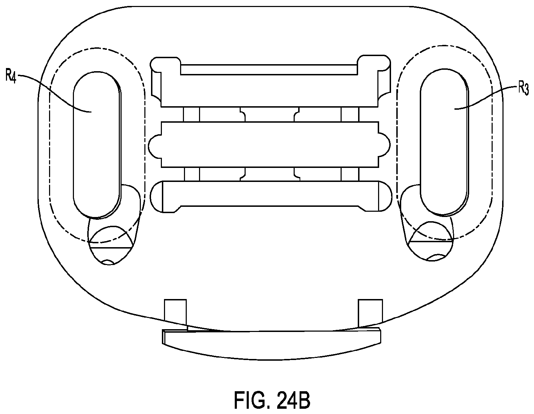

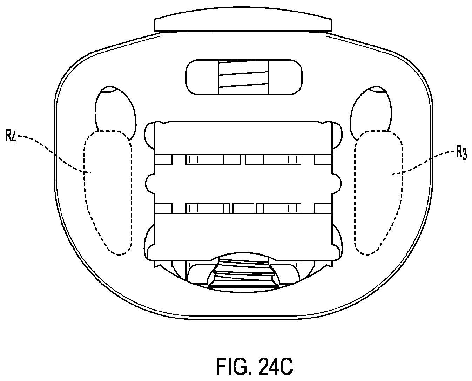

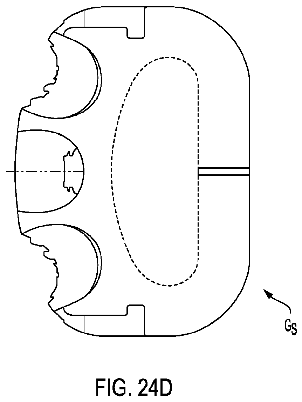

FIGS. 24A-24D are various side views and top down views of exemplary bone graft areas in accordance with the principles of the present disclosure. In the side view of FIG. 24A, first and second regions R₁, and R₂ are shown where bone growth material may be grafted and/or bone growth promoting materials may be used. In the top down view of FIG. 24B, third and fourth regions R₃, R₄ are shown where bone growth material may be grafted and/or bone growth promoting materials may be used. In some embodiments, third and fourth regions R₃, R₄ overlap vertically with first and second regions R₁, and R₂. In FIGS. 24C and 24D an exemplary grafting section GS is shown. Grafting section GS may be grafted to an endplate 110, 120. In some embodiments, grafting section GS may be filled with a bone growth material having a resultant surface area ranging from about 140 mm² to about 180 mm, and more particularly about 160 mm². For example, the bone growth material may extend through the grafting section GS three dimensionally and have a corresponding surface area ranging from about 140 mm² to about 180 mm², and more particularly about 160 mm². Consistent with disclosed embodiments herein, the open arrangement of spinal implant 100 and endplates 110, 120 in particular is advantageous for direct segmental fusion techniques. For example, the superior and inferior vertebral endplates allow the creation of a fusion bone bridge to solidify a segment. Additionally, the expandable and contractible nature of spinal implant 100 lends to bone packing techniques after positioning and adjusting spinal implant 100 between vertebral bodies. For example, after spinal implant 100 is positioned between adjacent vertebral bodies, spinal implant 100 may be packed with bone material in situ. In some embodiments, the endplate 110 may be considered a direct superior vertebral endplate and endplate 120 may be considered an inferior vertebral endplate where such endplates are configured to allow for a fusion bone bridge there through to solidify a segment.

In some embodiments, the spinal implant system includes an agent, including but not limited to the bone growth promoting materials described herein, which may be disposed, packed, coated or layered within, on or about the components and/or surfaces of the spinal implant system. In some embodiments the bone growth promoting material may be pre-packed in the interior of spinal implant 100, and/or may be packed during or after implantation of the implant via a tube, cannula, syringe or a combination of these or other access instruments. Additionally, bone growth promoting material may be further tamped into spinal implant 100 before, during or after implantation. In some embodiments, the bone growth promoting material and/or directly grafted material may enhance fixation of spinal implant 100 with adjacent bony structures. In some embodiments, the agent may include one or a plurality of therapeutic agents and/or pharmacological agents for release, including sustained release, to treat, for example, pain, inflammation and degeneration.

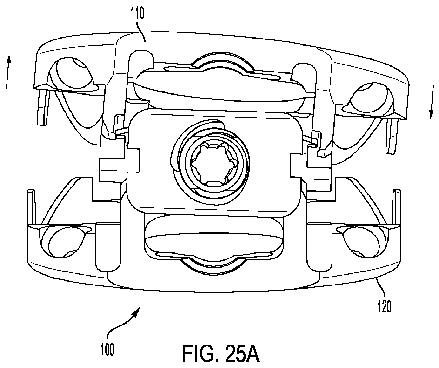

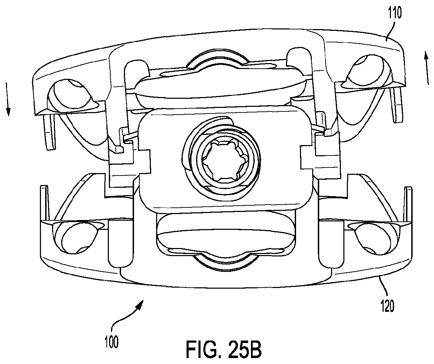

FIGS. 25A and 25B illustrate spin implant 100 in a first bent position and a second bent position, respectively. FIG. 25A shows spinal implant 100 where top endplate 110 is bent in a first lateral direction with respect to bottom endplate 120. FIG. 25B shows spinal implant 100 where top endplate 110 is bent in a second lateral direction, opposite the first lateral direction, with respect to bottom endplate 120. As explained in greater detail above, the various disclosed projections, guide walls, cavities, recesses, etc. are configured such that spinal implant 100 may allow for lateral bending to some predetermined degree. For example, projections 256c, 257c, 258c may pivot laterally in guide walls 130 to accommodate some degree of lateral bending. In this way, top endplate 110 and bottom endplate 120 may be configured to laterally bend with respect one another in a first direction and a second direction by a predetermined amount. However, in other embodiments it may be desirable for spinal implant 100 to be rigid in the lateral direction and for no lateral bending to be permissible.

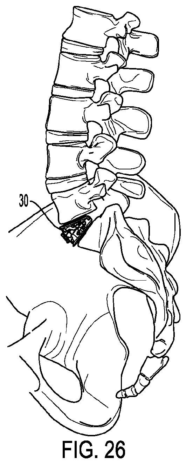

FIGS. 26-28 illustrate a left side view, right side view, and front side view, respectively, of an installed expandable spinal implant 100 positioned between adjacent vertebral bodies according to various surgical techniques, *e.g*., anterior techniques, oblique techniques, lateral techniques. For example, FIGS. 26-28 show spinal implant 100 after being installed according to an anterior lumbar interbody fusion (ALIF) technique.

Spinal implant systems of the present disclosure can be employed with a surgical arthrodesis procedure, such as, for example, an interbody fusion for treatment of an applicable condition or injury of an affected section of a spinal column and adjacent areas within a body, such as, for example, intervertebral disc space between adjacent vertebrae, and with additional surgical procedures and methods. In some embodiments, spinal implant systems can include an intervertebral implant that can be inserted between adjacent vertebral bodies to space apart articular joint surfaces, provide support for and maximize stabilization of vertebrae. In some embodiments, spinal implant systems may be employed with one or a plurality of vertebra.

Consistent with the disclosed embodiments herein, a medical practitioner may obtain access to a surgical site including vertebrae such as through incision and retraction of tissues. Spinal implant systems of the present disclosure can be used in any existing surgical method or technique including open surgery, mini-open surgery, minimally invasive surgery and percutaneous surgical implantation, whereby vertebrae are accessed through a mini-incision, retractor, tube or sleeve that provides a protected passageway to the area, including, for example, an expandable retractor wherein the sleeve is formed from multiple portions that may be moved apart or together and may be inserted with the portions closed or together and then expanded to allow for insertion of implants of larger size than the closed cross section of the unexpanded retractor portions. In one embodiment, the components of the spinal implant system are delivered through a surgical pathway to the surgical site along a surgical approach into intervertebral disc space between vertebrae. Various surgical approaches and pathways may be used.

As will be appreciated by one of skill in the art, a preparation instrument (not shown) may be employed to remove disc tissue, fluids, adjacent tissues and/or bone, and scrape and/or remove tissue from endplate surfaces of a first vertebra and/or endplate surface of a second vertebra in preparation for or as part of the procedures utilizing a system of the present disclosure. In some embodiments, the footprint of spinal implant 100 is selected after trialing using trialing instruments (not shown) that may approximate the size and configuration of spinal implant 100. In some embodiments, such trials may be fixed in size and/or be fitted with moving mechanisms 250 similar to embodiments described herein. In some embodiments, spinal implant 100 may be visualized by fluoroscopy and oriented before introduction into intervertebral disc space. Furthermore, first and second surgical tools 400, 500, and spinal implant 100 may be fitted with fiducial markers to enable image guided surgical navigation to be used prior to and/or during a procedure.

Components of a spinal implant systems of the present disclosure can be delivered or implanted as a pre-assembled device or can be assembled in situ. In one embodiment, spinal implant 100 is made of a single piece construction that may not be disassembled without destroying the device. In other embodiments, spinal implant 100 may comprise removable parts. Components of spinal implant system including implant 10, 20, 30 may be expanded, contracted, completely or partially revised, removed or replaced in situ. In some embodiments, spinal implant 100 can be delivered to the surgical site via mechanical manipulation and/or a free hand technique.

Additionally, components of spinal implant 100 can include radiolucent materials, *e.g*., polymers. Radiopaque markers may be included for identification under x-ray, fluoroscopy, CT or other imaging techniques. Furthermore, first and second surgical tools 400, 500 may be radiolucent and may optionally include markers added at a tip portion thereof to permit them to be seen on fluoroscopy/x-ray while advancing into the patient. At least one advantage to having spinal implant 100 is that a medical practitioner can verify the positioning of spinal implant 100 relative to adjacent vertebral bodies and make further adjustments to the spacing between endplates 110, 120, angle of inclination between endplates 110, 120, and the overall positioning of the device within a patient's body. In this way, spinal implant 100 may correct alignment of a patient's spine in a sagittal plane.

FIG. 29A is a perspective view of a second embodiment of an expandable spinal implant 200 in accordance with the principles of the present disclosure. Aspects of second spinal implant 100 may be the same as, substantially the same as, or similar to spinal implant 100. Additionally, second spinal implant 200 may be used in previously disclosed systems and methods. Accordingly, duplicative description thereof will be omitted.

FIG. 29B is an exploded view illustrating second spinal implant 200. Second spinal implant 200 a top endplate 110 (first endplate) and a bottom endplate 120 (second endplate) and a moving mechanism 2500, which will be described in greater detail below. The proximal end 101 includes a screw guide endplate 1050 disposed between endplates 110 and 120. In some embodiments, screw guide endplate 1050 may be pivotable left-right and up-down to accommodate insertion of first surgical tool 400 from an off angle position. For example, screw guide endplate 1050 may accommodate a surgical tool that is insert off angle (not axially aligned) in a range of about 1° to 20°, and more particularly about 1° to 15° in the horizontal and vertical directions. At least one advantage of this arrangement is that first surgical tool 400 may be inserted off angle with respect to guide aperture 107 of spinal implant 200.

In the exemplary embodiment, moving mechanism 2500 is operably coupled to top endplate 110 and bottom endplate 120 similarly as explained above. Moving mechanism 2500 differs from moving mechanism 250 in that moving mechanism 2500 may be miss aligned, for example by about 5°, 10°, 15°, or 20° when compared to moving mechanism 250 of the first embodiment. In at least one embodiment, moving mechanism 2500 is misaligned about 15° to facilitate insertion and posterior adjustment by reconnection posteriorly. In the exemplary embodiment, moving mechanism 2500 operates by the same principles as moving mechanism 250 although the interior contours of top endplate 110 and bottom endplate 120 are shifted to allow moving mechanism 2500 to be miss aligned.

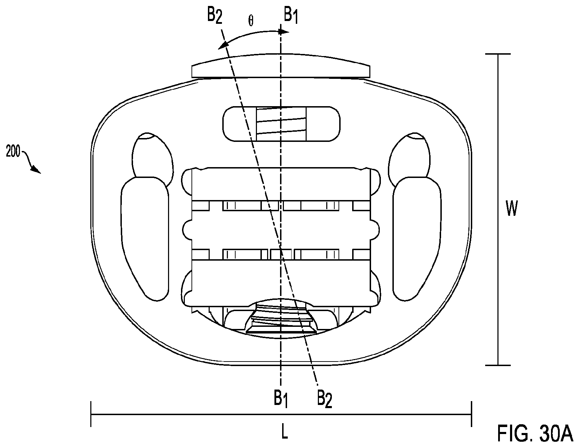

FIG. 30A is a top down view of spinal implant 200 contrasting an embodiment where moving mechanism 2500 is miss aligned. As illustrated, spinal implant 200 has a first reference axis B₁ and a second reference axis B₂. First reference axis B₁ may be understood as a projection where moving mechanism 2500 is not miss aligned and where moving mechanism 2500 is in a centered position. Second reference axis B₂ may be understood as a projection passing through a central portion of guide aperture 107 through moving mechanism 2500 when moving mechanism 2500 is miss aligned inside of endplates 110, 120 to an off-centered position.

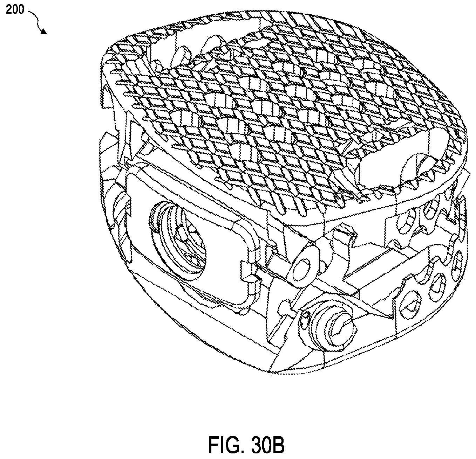

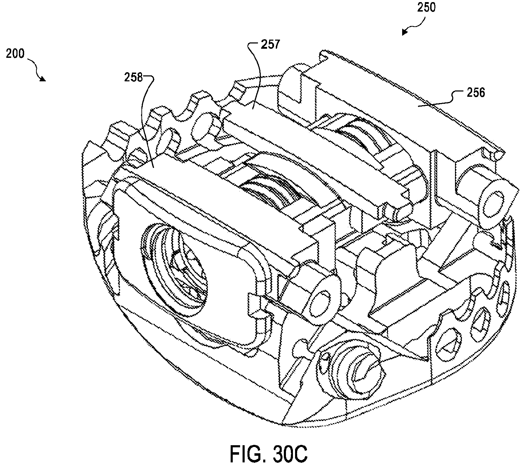

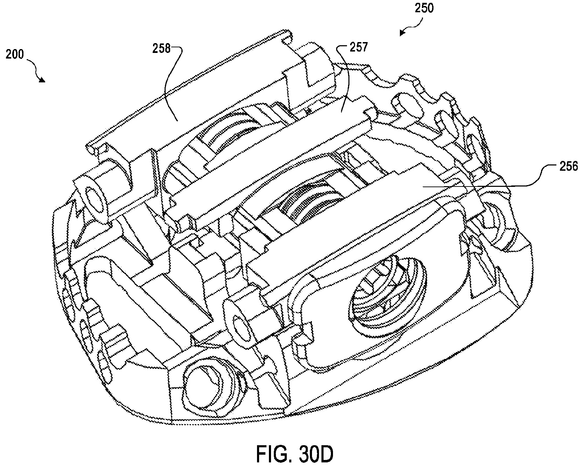

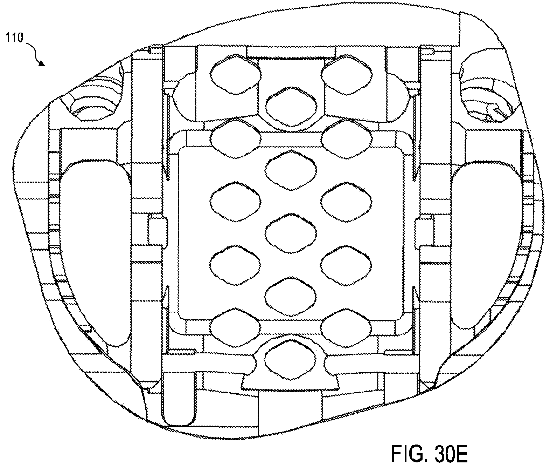

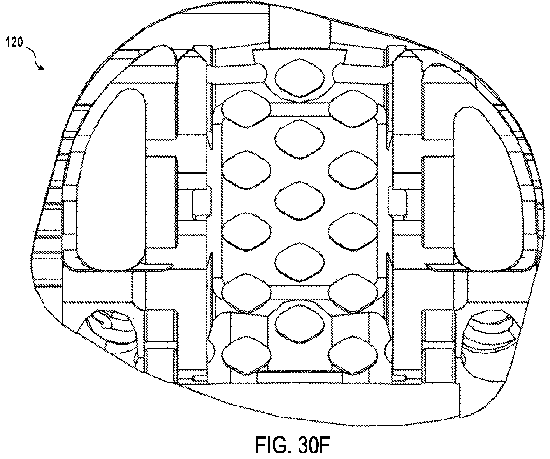

Referring generally to FIGS. 30B-30F, a modified embodiment of spinal implant 200 where moving mechanism 2500 is miss aligned is disclosed. In the disclosed embodiment, moving mechanism 2500 features the same parts as moving mechanism 250 and operates under the same principles as explained previously. In the disclosed embodiment, moving mechanism 2500 is miss aligned by about 15° when compared with moving mechanism 250 of spinal implant 100. In other embodiments, moving mechanism 2500 may be miss aligned within any suitable range, *e.g.,* from about 5° to 25°. FIG. 30C is a perspective view of the embodiment of FIG. 30B with a top endplate 110 removed for ease of understanding. As illustrated, moving mechanism 2500 is misaligned and the top and bottom endplates 110, 120 have a different geometry to accommodate the miss aligned moving mechanism 2500. Top and bottom endplates 110, 120 may feature the same or substantially the same characteristics as previously disclosed. FIG. 30D is an alternate perspective view of the embodiment of FIG. 30B with a top endplate 110 removed for ease of understanding. FIG. 30E is a top down view of an exemplary top endplate 110 for use with the embodiment of FIG. 30B and FIG. 30F is a top down view of an exemplary bottom endplate 120 for use with the embodiment of FIG. 30B.

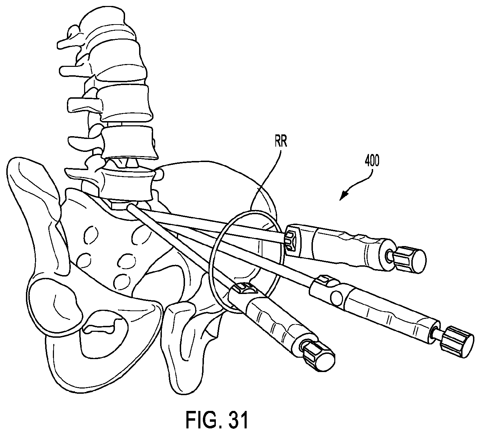

FIG. 31 is a perspective view of spinal implant 200 in an installed position between vertebral bodies and three alternate positions of first surgical tool 400. FIG. 31 shows how first surgical tool 400 may be inserted into guide aperture 107 off angle with respect to first reference axis B₁. Reference ring RR represents the extent of viable offset positions that first surgical tool 400 may be operably inserted in guide aperture 107. In some embodiments, first surgical tool 400 may be bent at a midsection area at 15° to enable a surgeon to adjust spinal implant 200 in such a way as to avoid anatomical features and organs, such as, for example the pelvic ring and iliac crest. Additionally, this advantage is further expanded upon when using a miss-aligned moving mechanism 2500 that is miss aligned by, for example, about 15°. Therefore, disclosed systems of spinal implant 200 are able to be manipulated by a surgeon via surgical tool 400 at the combined total angular extent the moving mechanism 2500 is offset and the angular extent the surgical tool is bent. In at least one embodiment, the total angular extent is about 30° on account of the moving mechanism 2500 being offset about 15° and the surgical tool 400 being bent about 15°.

FIG. 32A is a top down view of a third embodiment of an expandable spinal implant 300 in accordance with the principles of the present disclosure. FIG. 32B shows spinal implant 300 in a perspective view. Aspects of spinal implant 300 may be the same as, substantially the same as, or similar to spinal implant 100. Additionally, spinal implant 300 may be used in previously disclosed systems and methods. Accordingly, duplicative description thereof will be omitted.

In some embodiments, the sizing and orientation of top and bottom endplates 110, 120 and the sizing and orientation of moving mechanism 250d is particularly advantageous for lateral insertion techniques. Spinal implant 300 includes a first reference axis C₁ and a second reference axis C₂. Different than previous embodiments, first reference axis C₁ may span a longitudinal length of spinal implant 300 and pass directly through a rotation axis of moving mechanism 250d. Second reference axis C₂ may bisect spinal implant 300 transversely across the center thereof. Additionally, second reference axis C₂ may intersect first reference axis C₁ and project through a center of buttress block 257.

Spinal implant 300 may include a top endplate 110d and a bottom endplate 120d and a moving mechanism 250, which may be the same as or substantially the same as described above. Spinal implant 300 includes a proximal end 101 and a distal end 102 opposite the proximal end 101, and a first lateral end 103 and a second lateral end 104 opposite the first lateral end 103. The first and second lateral ends 103, 104 extend between the proximal end 101 and the distal end 102. The proximal end 101 includes an exposed screw guide endplate 105 defining a corresponding screw guide aperture 107, which are disposed between endplates 110d and 120d. The screw guide endplate 105 and guide aperture 107 may be the same as or substantially the same as described above.

Top endplate 110 may include a first outside surface 111d and a first inside surface 112d opposite the first outside surface 111d. Similarly, bottom endplate 120d may include a second outside surface 121d and a second inside surface 122d. The outside surfaces 111d, 121d may be configured to be positioned between and/or contact vertebral bodies in a patients spine and have various surface characteristics similar to those described above with reference to spinal implant 100. In some embodiments, outside surfaces 111d and 122d may have a substantially linear surface profile across faces of textured surfaces thereof. In other embodiments, outside surfaces 111d and 122d may have curved surface profiles across faces of textured surfaces thereof. Further details of endplates 110d, 120d will be described in greater detail below.

Inside surfaces 111d, 122d, may surround moving mechanism 250 and have various contours, guides, cavities, and other operable characteristics that facilitate movement and/or provide mechanical advantage to other operable and movable corresponding parts to facilitate contraction. angular adjustment, lateral bending, absorption of compression forces, shear forces, etc. as will be explained in greater detail below.

In the exemplary embodiment, top endplate 110d includes a pair of first proximal ramps 114d and a pair of first distal ramps 116d opposite the first proximal ramps 114d. Each ramp of the first proximal ramps 114d includes an inclined surface extending away from inside surface 112d and moving mechanism 250d. Similarly, each ramp of first distal ramps 116d includes an inclined surface extending away from inside surface 112d and moving mechanism 250d. Bottom endplate 120d includes a pair of second proximal ramps 124d and a pair of second distal ramps 126d opposite the second proximal ramps 124d. Each ramp of the second proximal ramps 124d includes an inclined surface extending away from inside surface 122d and moving mechanism 250d. Similarly, each ramp of second distal ramps 126d includes an inclined surface extending away from inside surface 11d1 and moving mechanism 250d.

Exemplary spinal implant 300 includes a moving mechanism 250d that is operably coupled to top endplate 110d and bottom endplate 120d, similarly as explained above with reference to spinal implant 100. Accordingly, duplicative description will not be repeated. A first functional feature of moving mechanism 250d is that it is further configured to increase and decrease a spacing between the top and bottom endplates 110d, 120d upon simultaneous rotation of first and second set screws 252, 254 in a clockwise and counterclockwise direction, respectively. A second functional feature of moving mechanism 250d is that it is further configured to increase and decrease an angle of inclination between top and bottom endplates 110d, 120d upon rotation of the first set screw 252 in a clockwise and counterclockwise direction, respectively.

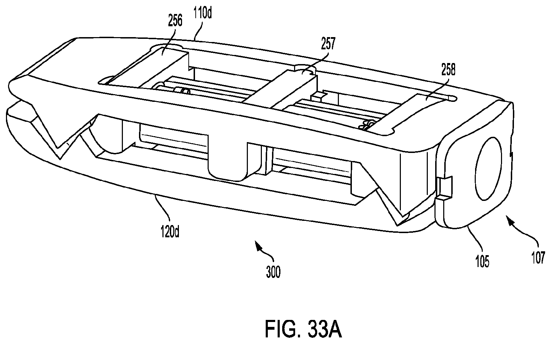

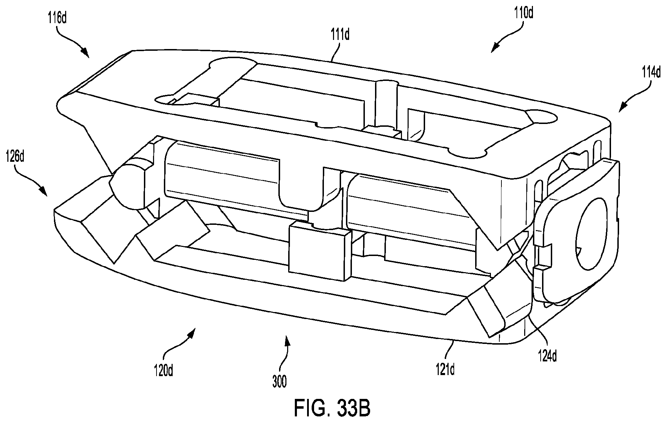

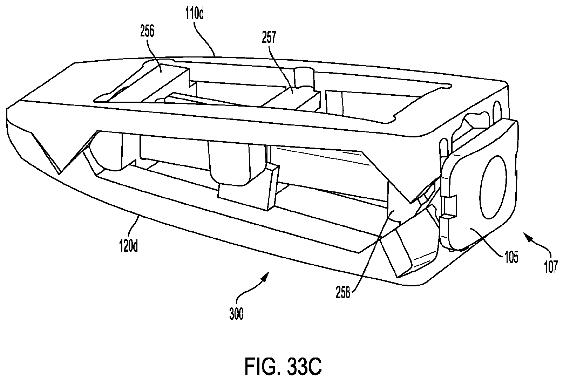

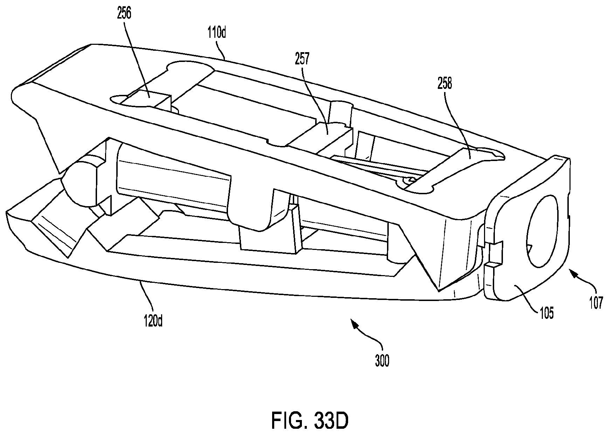

FIG. 33A is a perspective view of spinal implant 300 in a contracted position and FIG. 33B is a perspective view of spinal implant 300 in an expanded position. In the contracted position of FIG. 33A, top endplate 110d and bottom endplate 120d are contracted to a fully closed position. In the expanded position of FIG. 33B, top endplate 110d and bottom endplate 120d are expanded an equal amount. Similarly as explained above with reference to spinal implant 100 and FIGS. 9A-9B when first surgical tool 400 is inserted in guide aperture 107 in a first position and rotated in a first direction (clockwise direction) the first and second trolleys 256, 258 move away from one another an equal amount in opposite directions. In turn, the first and second trolleys 256, 258 cause the top and bottom endplates 110d, 120d to move apart from one another an equal amount. Likewise, when first surgical tool 400 is rotated in a second direction (counter-clockwise direction) first and second trolleys 256, 258 cause the top and bottom endplates 110d, 120d to move towards one another an equal amount in a contraction direction (not illustrated). In summary, when positioning the first surgical tool 400 in the first position and rotating the first surgical tool 400 in either the first or second direction the moving mechanism 250d operably adjusts a spacing between the top and bottom endplates 110d, 120d. FIG. 33C is a perspective view of spinal implant 300 in a first angled position and FIG. 33D is a perspective view of spinal implant 300 in a second angled position. Spinal implant 300 may have the same or similar features as explained above with respect to spinal implants 100, 200. Spinal implant 300 may be capable of (1) expanding/contracting the proximal end while the distal end remains stationary, (2) expanding/contracting the distal end while the proximal end remains stationary, and (3) expanding/contracting both the proximal end and distal end simultaneously. Similarly as explained above with reference to spinal implant 100 and FIGS. 10A-10B when first surgical tool 400 is inserted in guide aperture 107 in a second position, and rotated in a first direction (clockwise direction) the first trolley 256 moves away from the proximal end 101 of spinal implant 100 and the second trolley 258 remains stationary in place. In effect, the top and bottom endplates 110d, 120d move towards one another at the distal end 102 (not shown) and move away from one another at the proximal end 101 thereby decreasing an angle of inclination between the top and bottom endplates 110, 120.

Likewise, when first surgical tool 400 is in the second position and is rotated in the second direction (counter-clockwise direction) the first trolley 256 moves towards the stationary second trolley 258. In effect, the top and bottom endplates 110d, 120d move towards one another at the proximal end 101 (not shown) thereby decreasing an angle of inclination between the top and bottom endplates 110d, 120d. In summary, when positioning the first surgical tool 400 in the second position and rotating the first surgical tool 400 in either the first or second direction the moving mechanism 250 operably adjusts an angle of inclination between the top and bottom endplates 110, 120 upon rotating the first set screw along the rotation axis.

In the contracted position of FIG. 33A, a first height between top endplate 110d and bottom endplate 120d on the proximal side 101 and distal side 102 is about 9mm. In the first expanded position of FIG. 33B, a second height of spinal implant 300 between top endplate 110d and bottom endplate 120d on the proximal side 101 and distal side 102 is about 9mm. Additionally, in the first expanded position of FIG. 33B, top endplate 110d is parallel with respect to bottom endplate 110d. In the first angled position of FIG. 33C, the top and bottom endplates 110d, 120d are contacting each other at the distal side 102 and are spaced apart from one another at the proximal side 101. For example, at the distal side 102, the height between top endplate 110d and bottom endplate 120d is about 9mm. For example still, at the proximate side 101, the height between top endplate 110d and bottom endplate 120d is about 16mm. Accordingly. an angle of inclination between top endplate 110d and bottom endplate 120d at the distal side 101 is about 11°. In the second angled position of FIG. 33D, the top and bottom endplates 110d, 120d are contacting each other at the proximal side 102 and are spaced apart from one another at the distal side 101. For example, at the proximal side 102, the height between top endplate 110d and bottom endplate 120d is about 9mm. For example still, at the distal side 101, the height between top endplate 110d and bottom endplate 120d is about 16mm. Accordingly, an angle of inclination between top endplate 110d and bottom endplate 120d at the proximal side 101 is about 11°.

In some embodiments, spinal implant 300 may comprise a three position inner drive shaft (not illustrated) complimentary to or in place of components of moving mechanism 250. The three position inner drive shaft may enable the first and second set screws 252, 254 to be adjusted independently from one another as well as enabling the first and second set screws 252, 254 to be adjusted concurrently or simultaneously. For example, first surgical tool 400 may have a relatively short circumferential surface 456 that will only engage one of the internal circumferential surfaces of first or second set screws 252, 254 at a time. For example still, another first surgical tool 400 having a relatively longer circumferential surface 456 may engage both of the internal circumferential surfaces of the first and second set screws 252, 254 at the same time. Consistent with disclosed embodiments, a surgeon can use a first surgical tool 400 having a relatively shorter circumferential surface 456 to perform angular adjustments of spinal implant 300 and then use a first surgical tool 400 having a relatively longer circumferential surface 456 to perform height adjustments of spinal implant 300. In other embodiments, spinal implant 300 may include a screw guide aperture 107 on both sides of the spinal implant 300 thereby providing access to the first set screw 252 independently from second set screw 254.

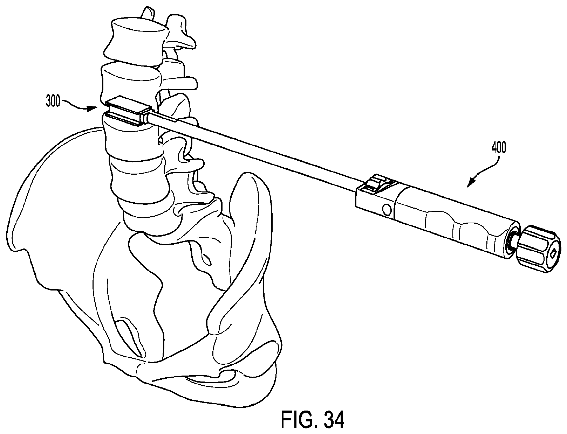

FIG. 34 is a perspective view of a spinal implant system utilizing spinal implant 300 and first surgical tool 400. In the exemplary system, spinal implant 300 is positioned in an installed position between vertebral bodies by first surgical tool 400 according to lateral insertion techniques as explained in greater detail above. First surgical tool 400 may operably adjust spinal implant 300 in situ between vertebral bodies as explained in greater above. For example, first surgical tool 400 may operably expand spinal implant 300 at a proximal side 101 and/or a distal side 102 thereof. In this way, spinal implant 300 may correct alignment of a patient's spine in a coronal plane.

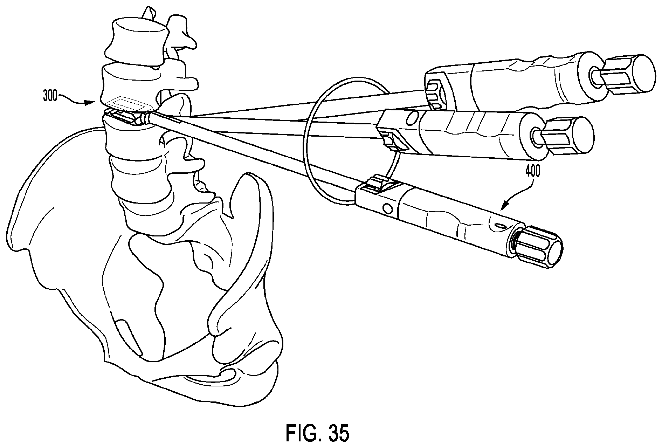

FIG. 35 is a perspective view of a spinal implant system utilizing spinal implant 300 highlighting how first surgical tool 400 may manipulate spinal implant 300 from various angles. For example, spinal implant 300 may include the same, substantially the same, or similar components to moving mechanism 2500 as explained above. In the exemplary embodiment, first surgical tool 400 may be inserted into guide aperture 107 off angle with respect to first reference axis B1. Reference ring RR represents the extent of viable offset positions that first surgical tool 400 may be operably inserted in guide aperture 107. In some embodiments, first surgical tool 400 may be bent at a midsection area at 15° (not illustrated) to enable a surgeon to adjust spinal implant 300 in such a way as to avoid anatomical features and organs, such as, for example the pelvic ring and iliac crest.

Referring generally to FIGS. 36-39B an additional expandable spinal implant 600 is disclosed. Expandable spinal implant 600 may have the same, substantially the same, and/or similar components and attributes as spinal implants 100, 200, and 300 including general applicability with other relevant systems and surgical tools disclosed hereinabove. Spinal implant 600 may include a screw guide endplate 6150 having at least one aperture 610 configured to receive an anchoring screw 510 therein. Screw guide endplate 6150 may be relatively longer in length than screw guide endplate 150 discussed above and screw guide endplate 6150 may be operably coupled with moving mechanism 250 similarly as explained above with respect to spinal implants 100, 200, and 300.

In the illustrated embodiment, top endplate 110 and bottom endplate 120 may each have an accommodating portion 630 having a corresponding size and geometry to the end portions of screw guide endplate 6150 such that when spinal implant 600 is in the fully collapsed position the end portions of screw guide endplate 6150 will not increase a relative height of implant 600 in a fully collapsed position. For example, endplates 110, 120 may fully close without being impacted by screw guide endplate 6150 and therefore maintain a relatively compact size.

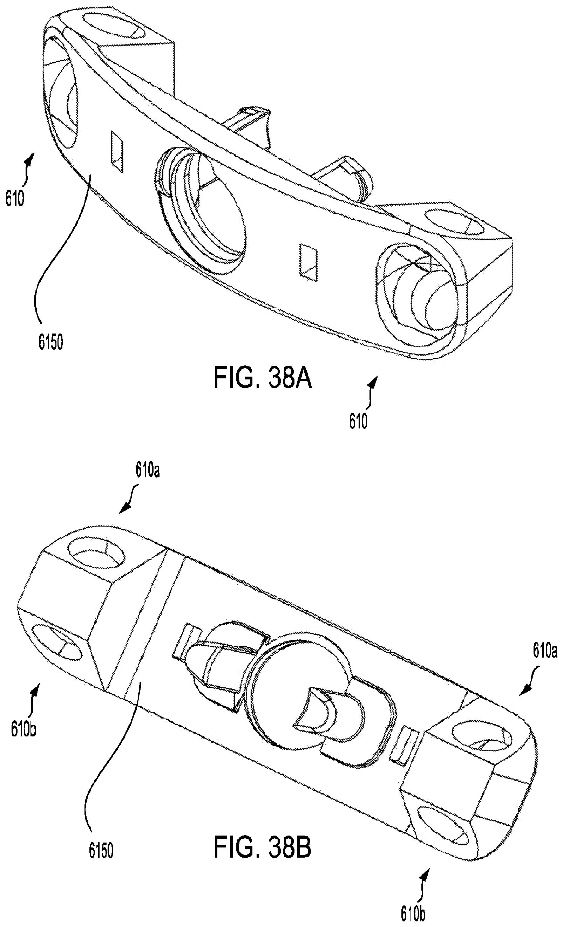

FIGS. 38A and 38B illustrate a front perspective view and a rear perspective view of an exemplary screw guide endplate 6150 having at least one aperture 610 configured to receive an anchoring screw 510 therein. In the illustrated embodiment, two apertures 610 are shown although embodiments in accordance with the principles of this disclosure may have any number of apertures 610. As illustrated, each aperture 610 may be configured to selectively receive a corresponding anchoring screw therein. The outside entrance to each aperture 610 may define two alternate guided paths. For example, a first guided path may be defined by the entrance to aperture 610 and a first exit aperture 610a and a second guided path may be defined by the entrance to aperture 610 and a second exit aperture 610b. In this way aperture 610 may be configured to orient one corresponding anchoring screw 510 at a time in either of a first orientation or a second orientation.

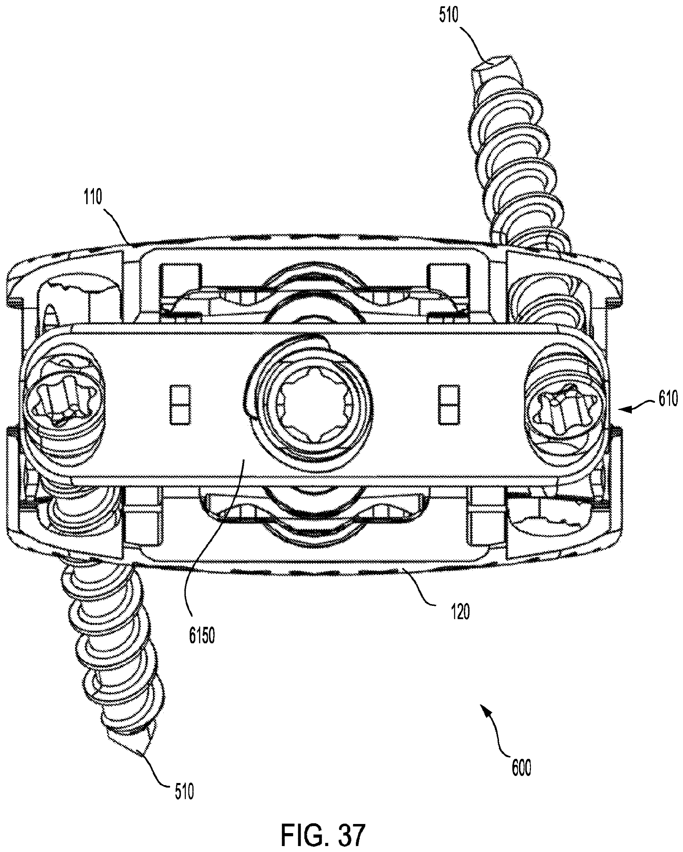

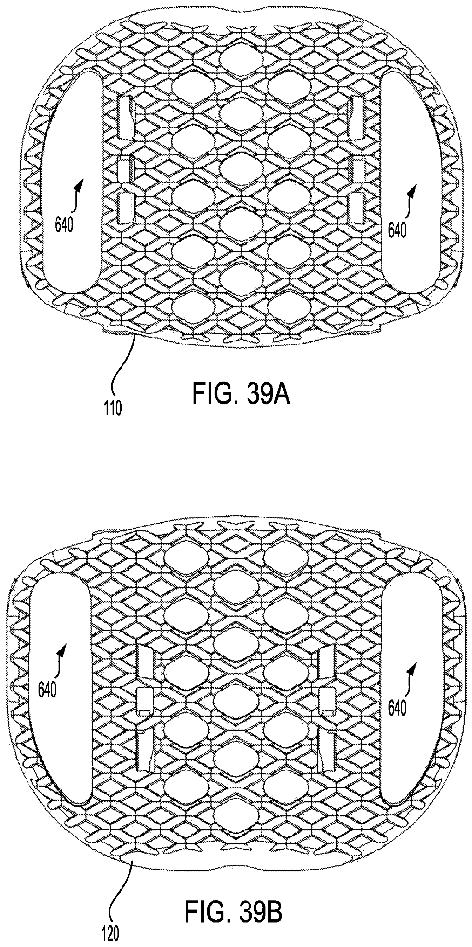

Corresponding exemplary first and second orientations are illustrated in FIG. 37 which shows a first anchoring screw 510 (right anchoring screw) oriented upward at an inclined angle with respect to top endplate 110 and a second anchoring screw 510 (left anchoring screw) oriented downward at an inclined angle with respect to bottom endplate 120. Additionally, the first orientation may align a corresponding anchoring screw 510 such that it projects through a corresponding slotted aperture 640 of the first endplate 110 *(see* FIGS. 36 and 39A). Similarly, the second orientation may align a corresponding anchoring screw 510 such that it projects through a corresponding slotted aperture 640 of the second endplate 120 *(see* FIGS. 36 and 39B).

At least one advantage of the disclosed spinal implant 600 is that screw guide endplate 6150 and moving mechanism 250 may be configured such that the moving mechanism 250 can selectively adjust a spacing between the first and second endplates 110, 120 and adjust an angle of inclination between the first and second endplates while the at least one corresponding anchoring screw 510 is anchored within a corresponding vertebrae. For example, a surgeon may initially position spinal implant 600 between adjacent vertebrae of a patient and install a corresponding first anchoring screw 510 in a first orientation projecting through slotted aperture 640 of first endplate 110 and a corresponding second anchoring screw 510 in a second orientation projecting through slotted aperture 640 of second endplate 120. Next, the surgeon may continue to adjust the spacing and/or angle of inclination between endplates 110, 120 until the endplates 110, 120 are in the desired position. This is possible, at least partly, because the relative location of the screw guide endplate 6150 remains fixed due to the anchored anchoring screws 510 and the first and second endplates can freely expand/contract and/or incline/decline via moving mechanism 250 while anchoring screws 510 extend through slotted aperture 640 (which has a geometry such that the anchored anchoring screws 510 do not interfere with the movement of endplates 110, 120). For example, the endplates 110, 120 may freely move while anchoring screws 510 remain anchored in place in the corresponding vertebrae while also changing a relative positioning with respect to the slotted aperture 640 due to movement of endplates 110, 120.

Referring generally to FIGS. 40-44B an additional expandable spinal implant 700 is disclosed. Expandable spinal implant 700 may have the same, substantially the same, and/or similar components and attributes as spinal implants 100, 200, 300, and 600 including general applicability with other relevant systems and surgical tools disclosed hereinabove. Spinal implant 700 may include a screw guide endplate 7150 having at least one aperture 710 configured to receive an anchoring screw 510 therein. Screw guide endplate 7150 may be relatively longer in length than screw guide endplate 150 discussed above and screw guide endplate 7150 may be operably coupled with moving mechanism 250 similarly as explained above with respect to spinal implants 100, 200, and 300.

In the illustrated embodiment, top endplate 110 and bottom endplate 120 may each have an accommodating portion 730 having a corresponding size and geometry to the end portions of screw guide endplate 7150 such that when spinal implant 700 is in the fully collapsed position the end portions of screw guide endplate 7150 will not increase a relative height of implant 700 in a fully collapsed position. For example, endplates 110, 120 may fully close without being impacted by screw guide endplate 7150 and therefore maintain a relatively compact size.

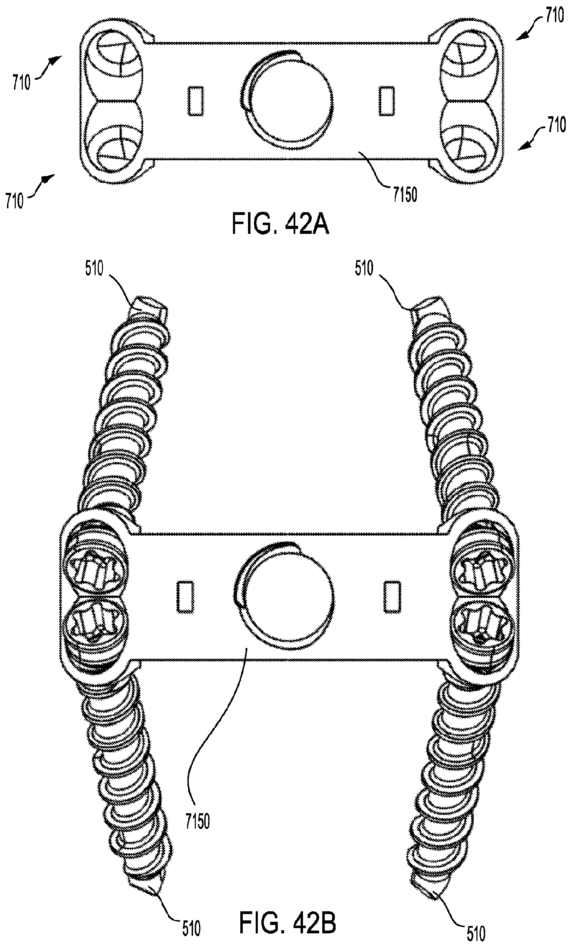

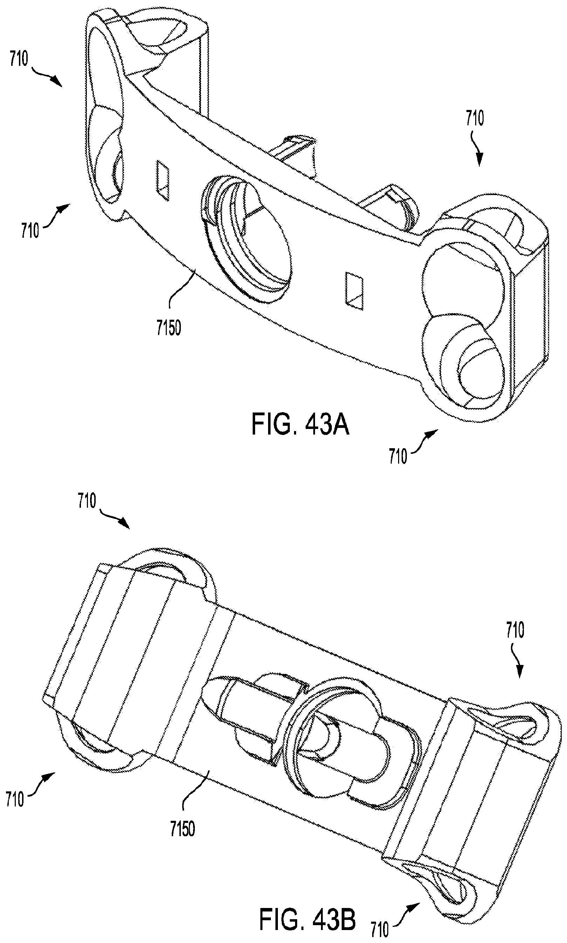

FIGS. 42A and 42B illustrate an exemplary screw guide endplate 7150 with and without corresponding anchoring screws 510, respectively. FIGS. 43A and 43B illustrate a front perspective view and a rear perspective view of an exemplary screw guide endplate 7150 having at least one aperture 710 configured to receive an anchoring screw 510 therein. In the illustrated embodiment, four apertures 710 are shown, although embodiments in accordance with the principles of this disclosure may have any number of apertures 710.

As illustrated, each aperture 710 may be configured to selectively receive a corresponding anchoring screw 510 therein. The outside entrance to each aperture 710 may define a guided path configured to orient a corresponding anchoring screw 510 in an inclined position extending away from a proximal side of a corresponding endplate 110 or 120. For example, screw guide endplate 7150 may include a total of four apertures 710, and the four apertures 710 may include two top most apertures 710 and two bottom most apertures 710. In the disclosed embodiment. the two top most apertures 710 may be configured to incline a corresponding anchoring screw 510 with respect to top endplate 110 that extends away from a proximal side of implant 700 towards a distal side of implant 700. Similarly, the two bottom most apertures 710 may be configured to incline a corresponding anchoring screw 510 with respect to bottom endplate 120 that extends from a proximal side of implant 700 towards a distal side of implant 700. Corresponding orientations are illustrated in FIGS. 40, 41, and 42B which show two top anchoring screws 510 oriented upward at an inclined angle with respect to top endplate 110 and two bottom anchoring screws 510 oriented downward at an inclined angle with respect to bottom endplate 120. Alternatively, the screw holes in the plate may be arranged and numbered in various alternative designs including, instead of two holes on top and bottom, presenting a single hole in the center or on one side or the other on top and bottom, or two holes on one of the top or bottom and one hole on the opposite side, top or bottom. These screw holes may further include protrusions, threads or other features to control, guide, and/or retain the screws in place or include features such as retaining clips, springs, or covers to retain the screws in place once inserted. The screw holes may be of various shapes including cylindrical, conical, or designed to receive a bulbous or spherical screw head.

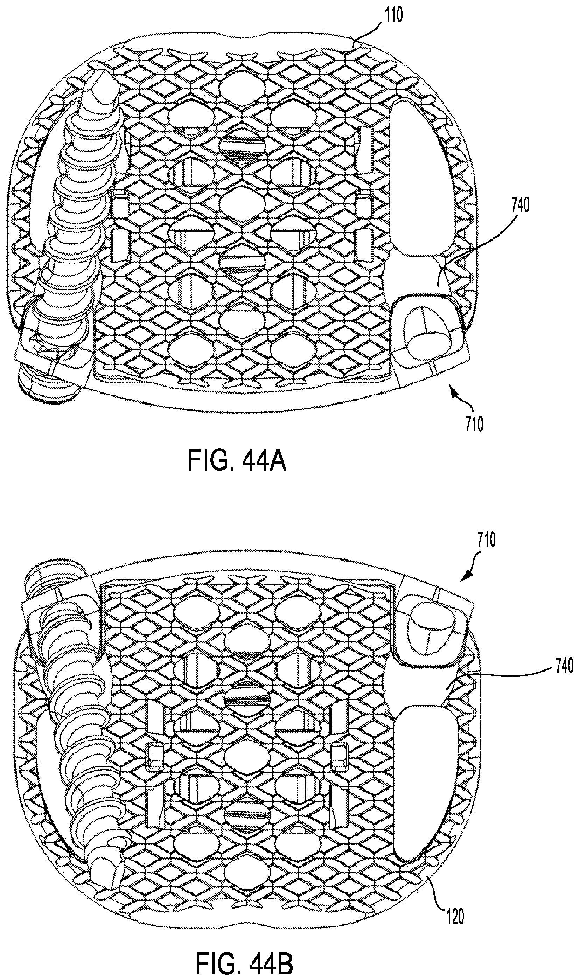

FIGS. 44A and 44B may illustrate a top endplate 110 and a bottom endplate 120, respectively, with an anchoring screw 510 in one corresponding aperture 710 and without an anchoring screw 510 in the other corresponding aperture 710 for ease of explanation. As illustrated, the top endplate 110 may include at least one anchoring screw 510 such that it projects through or across a corresponding recess 740 of the first endplate 110. Similarly, the bottom endplate 120 may include at least one anchoring screw 510 such that it projects through or across a corresponding recess 740 of the first endplate 110.

At least one advantage of the disclosed spinal implant 700 is that screw guide endplate 7150 and moving mechanism 250 may be configured such that the moving mechanism 250 can selectively adjust a spacing between the first and second endplates 110, 120 and adjust an angle of inclination between the first and second endplates while the at least one corresponding anchoring screw 510 is anchored within a corresponding vertebrae. For example, a surgeon may initially position spinal implant 700 between adjacent vertebrae of a patient and install at least one corresponding anchoring screw 510 in a first orientation projecting through or across a corresponding recess 740 of first endplate 110 and at least one corresponding anchoring screw 510 in a second orientation projecting through or across recess 740 of second endplate 120. Next, the surgeon may continue to adjust the spacing and/or angle of inclination between endplates 110, 120 until the endplates 110, 120 are in the desired position. This is possible, at least partly, because the relative location of the screw guide endplate 7150 remains fixed due to the anchored anchoring screws 510 and the first and second endplates can freely expand/contract and/or incline/decline via moving mechanism 250 while anchoring screws 510 extend through or across recess 740 (which has a geometry such that anchored anchoring screws 510 do not interfere with the movement of endplates 110, 120). For example, the endplates 110, 120 may freely move while anchoring screws 510 remain anchored in place in the corresponding vertebrae.

FIG. 45 is a reference diagram illustrating various cardinal directions and planes with respect to a patient that the exemplary embodiments of FIGS. 1-44B may operate, adjust, and/or move along in accordance with the principles of the present disclosure.

## Claims

1. An expandable spinal implant (100, 200, 300, 600, 700) deployable between a contracted position and an expanded position, comprising:
a first endplate (110), the first endplate (110) including:
a first outside surface (111) and a first inside surface (112) opposite the first outside surface (111), the first inside surface (112) including a first plurality of guide walls (130,) a first proximal end (101) and a first distal end (102) opposite the first proximal end (101), first proximal ramps (114) and first distal ramps (116) disposed opposite the first proximal ramps (114), and a first lateral surface and a second lateral surface opposite the first lateral surface, the first and second lateral surfaces extending between the first proximal end and the first distal end;
a second endplate (120), the second endplate (120) including:
a second outside surface (121) and a second inside surface (122) opposite the second outside surface (121), the second inside surface (122) including a second plurality of guide walls, a second proximal end and a second distal end opposite the second proximal end, second proximal ramps (124) and second distal ramps (126) disposed opposite the second proximal ramps (124), and a third lateral surface and a fourth lateral surface opposite the third lateral surface, the third and fourth lateral surfaces extending between the second proximal end and the second distal end;
a moving mechanism (250) operably coupled to the first endplate (110) and the second endplate (120) and positioned therebetween, the moving mechanism (250) including:
a buttress block (257) and a first trolley (256) and a second trolley (258) disposed on opposite sides of the buttress block (257), a screw guide wall housing a rotatable first set screw (252) and a rotatable second set screw (254) opposite the first set screw (252), the first set screw (252) being operably coupled to the first trolley (256) and the second set screw (254) being operably coupled to the second trolley (258), the first set screw (252) and second set screw (254) being configured to rotate in a first rotation direction and a second rotation direction about a rotation axis, the rotation axis projecting in a longitudinal direction of the moving mechanism (250),
wherein the first trolley (256) is operably coupled to the first set screw (252) and movable toward and away the buttress block (257) in the longitudinal direction of the moving mechanism (250) by rotation of the first set screw (252) along the rotation axis, the second trolley (258) is operably coupled to the second set screw (254) and movable toward and away the buttress block (257) in the longitudinal direction of the moving mechanism (250) by rotation of the second set screw (254) along the rotation axis,
wherein the first trolley (256) includes a first side surface and a second side surface opposite the first side surface and has a first plurality of projections (256c) projecting from the first and second side surfaces, the second trolley (258) includes a third side surface and a fourth side surface opposite the third side surface and has a second plurality of projections (258c) projecting from the third and fourth side surfaces,
wherein the first and second plurality of projections (256c, 258c) correspond to a cross sectional shape of the first and second plurality of guide walls (130) and are operably coupled thereto, respectively, such that the first and second plurality of projections (256c, 258c) move along the first and second plurality of guide walls, respectively,
wherein the moving mechanism (250) is configured to operably adjust a spacing between the first and second endplates (110, 120) upon simultaneous rotation of the first and second set screws (252, 254) along the rotation axis, and
wherein the moving mechanism (250) is configured to operably adjust an angle of inclination between the first and second endplates (110, 120) upon rotating the first set screw or second set screw (252, 254) along the rotation axis.

2. The spinal implant (100, 200, 300, 600, 700) of claim 1, wherein the moving mechanism (250) is further configured to:
increase a first distance between the first endplate (110) and the moving mechanism (250) and increase a second distance between the second endplate (120) and the moving mechanism (250) an equal amount upon simultaneous rotation of the first and second set screws in the first rotation direction;
decrease the first distance between the first endplate (110) and the moving mechanism (250) and decrease the second distance between the second endplate (110) and the moving mechanism (250) an equal amount upon simultaneous rotation of the first and second set screws in the second rotation direction;
increase the angle of inclination between the first and second endplates (110, 120) upon rotating at least one of the first set screw or second set screw along the rotation axis in the first direction; and
decrease the angle of inclination of the first and second endplates (110, 120) upon rotating at least one of the first set screw or second set screw along the rotation axis in the first direction.

3. The spinal implant (100, 200, 300, 600, 700) of claim 1 or 2,
wherein the first proximal ramps (114) include a first and second ramp disposed adjacent the first proximal end that project away from the first inside surface,
wherein the first distal ramps (116) include a third and fourth ramp disposed adjacent the first distal end that project away from the first inside surface,
wherein the second proximal ramps (124) include a fifth and sixth ramp disposed adjacent the second proximal end that project away from the second inside surface, and
wherein the second distal ramps (126) include a seventh and eighth ramp disposed adjacent the second distal end that project away from the second inside surface,

4. The spinal implant (100, 200, 300, 600, 700) of claim 3,
wherein the first trolley (256) further comprises a first wedge (256d) projecting from the first side surface in a transverse direction of the moving mechanism (250) and a second wedge (256d) projecting from the second side surface in the transverse direction of the moving mechanism (250), and
wherein the second trolley (258) further comprises a third wedge (258d) projecting from the third side surface in the transverse direction of the moving mechanism (250) and a fourth wedge (258d) projecting from the fourth side surface in the transverse direction of the moving mechanism (250).

5. The spinal implant (100, 200, 300, 600, 700) of claim 4, wherein the first wedge (256d) includes a first upper contact surface (256e) and a first lower contact surface (256f), the second wedge (256d) includes a second upper contact surface (256e) and a second lower contact surface (256f), the third wedge (258d) includes a third upper contact surface (258e) and a third lower contact surface (258f), the fourth wedge (258d) includes a fourth upper contact surface (258e) and a fourth lower contact surface (258f), and
wherein the first and second upper contact surfaces (256e) contact the first proximal ramps (114) and the first and second lower contact surfaces (256f) contact the second proximal ramps (124), and
wherein the third and fourth upper contact surfaces (258e) contact the first distal ramps (116) and the third and fourth lower contact surfaces (258f) contact the second distal ramps (126).

6. The spinal implant (100, 200, 300, 600, 700) of claim 4, wherein the first wedge (256d) includes a first curved upper contact surface (256e) and a first curved lower contact surface (256f), the second wedge (256d) includes a second curved upper contact surface (256e) and a second curved lower contact surface (256f), the third wedge (258d) includes a third curved upper contact surface (258e) and a third curved lower contact surface (258f), the fourth wedge (258d) includes a fourth curved upper contact surface (258e) and a fourth curved lower contact surface (258f),
wherein the first and second curved upper contact surfaces (256e) contact the first proximal ramps (114) and the first and second curved lower contact surfaces (256f) contact the second proximal ramps (224),
wherein the third and fourth curved upper contact surfaces (258e) contact the first distal ramps (116) and the third and fourth curved lower contact surfaces (258f) contact the second distal ramps (226), and
wherein the first through fourth curved upper surfaces (256e, 258e) and the first through fourth curved lower surfaces (256f, 258f) are configured to facilitate adjustment of the angle of inclination between the first and second endplates (110, 120) upon rotating the first set screw (252) along the rotation axis by enabling the respective curved surfaces to pivot on a corresponding ramp of the first and second proximal ramps (114, 224) and first and second distal ramps (116, 216).

7. The spinal implant (100, 200, 300, 600, 700) one of claims 4-6,
wherein the first and second wedges (256d) are configured to move along first and second inclined contact surfaces of the first and second proximal ramps (114, 224), respectively, and
wherein the third and fourth wedges (258d) are configured to move along third and fourth inclined contact surfaces of the first and second distal ramps (116, 226), respectively, optionally,
wherein the first inclined contact surfaces of the first proximal ramps (114) are inclined with respect to the first inside surface of the first endplate (110) and extend away from the first inside surface by a first inclined distance and the second inclined contact surfaces of the second proximal ramps (224) are inclined with respect to the second inside surface of the second endplate (120) and extend away from the second inside surface by a second inclined distance,
wherein the third inclined contact surfaces of the first distal ramps (116) are inclined with respect to the first inside surface of the first endplate (110) and extend away from the first inside surface by a third inclined distance and the fourth inclined contact surfaces of the second distal ramps (226) are inclined with respect to the second inside surface of the second endplate (120) and extend away from the second inside surface by a second inclined distance, and
wherein the first inclined distance is greater than the third inclined distance and the second inclined distance is greater than the fourth inclined distance thereby facilitating adjustment of the angle of inclination between the first and second endplates (110, 120) upon rotating the first set screw along the rotation axis.

8. The spinal implant (100, 200, 300, 600, 700) of one of claims 1-7, wherein each ramp of the first and second proximal ramps (114, 224) and first and second distal ramps (116, 226) includes a corresponding contact surface, and
wherein each guide wall of the first plurality of guide walls and each guide wall of the second plurality of guide walls extends in a parallel direction with at least one ramp of the first and second proximal ramps (114, 224) and first and second distal ramps (116, 226).

9. The spinal implant (100, 200, 300, 600, 700) of one of claims 1-8, wherein the first and second endplates (110, 120) are pivotable in a lateral direction thereof with respect to the moving mechanism (250).

10. The spinal implant (100, 200, 300, 600, 700) of one of claims 1-9, wherein the first and second endplates (110, 120) are configured to promote bone growth therebetween.

11. The spinal implant (100, 200, 300, 600, 700) of one of claims 1-10, wherein the first and second endplates (110, 120) include a plurality of inclined apertures and a corresponding plurality of anchoring screws, each anchoring screw passing through a corresponding inclined aperture and being configured to anchor into a vertebral body.

12. The spinal implant (100, 200, 300, 600, 700) of one of claims 1-11, wherein the screw guide wall housing includes an aperture passing through the first proximal end of the first endplate (110) and the second proximal end of the second endplate (120), the aperture being configured to receive a corresponding surgical tool having circumferential edges extending in a longitudinal direction thereof,
wherein the circumferential edges are configured to selectively engage with the first set screw and second set screw in a first insertion position, and engage with only the second set screw in a second insertion position,
wherein the corresponding surgical tool is configured to selectively rotate the first set screw and/or the second set screw along the rotation axis, optionally, wherein the screw guide wall housing is further configured to receive the corresponding surgical tool at an offset angle with respect to the rotation axis of the moving mechanism (250).

13. The spinal implant (100, 200, 300, 600, 700) of one of claims 1-12, wherein the first and second endplates (110, 120) each have a footprint configured for at least one surgical technique chosen from: anterior surgical insertion and adjustment techniques, oblique surgical insertion and adjustment techniques, and lateral surgical insertion and adjustment techniques,
optionally, wherein the moving mechanism (250) is configured to:
adjust the spacing between the first and second endplates (110, 120) at the proximal end from about 10 mm to about 22 mm and adjust the spacing between the first and second endplates (110, 120) at the distal end from about 7 mm to about 12 mm; and
adjust the angle of inclination between the first and second endplates (110, 120) within an angular range from about 7° to about 25°.

14. The spinal implant (100, 200, 300, 600, 700) of one of claims 1-13,
wherein the moving mechanism (250) is configured to:
adjust the spacing between the first and second endplates (110, 120) at the proximal end from about 9 mm to about 16 mm and adjust the spacing between the first and second endplates (110, 120) at the distal end from about 9 mm to about 16 mm; and
adjust the angle of inclination between the first and second endplates (110, 120) within an angular range from about 6° to about 11°.

15. The spinal implant (100, 200, 300, 600, 700) of one of claims 1-14,
wherein the first endplate (110) has a concave surface profile with respect to the moving mechanism (250) and the second endplate (110) has a convex surface profile with respect to the moving mechanism (250).

## Patentansprüche

1. Expandierbares Wirbelsäulenimplantat (100, 200, 300, 600, 700), das zwischen einer kontrahierten Position und einer expandierten Position entfaltbar ist, umfassend:
eine erste Endplatte (110), wobei die erste Endplatte (110) einschließt:
eine erste Außenfläche (111) und eine erste Innenfläche (112) gegenüber der ersten Außenfläche (111), wobei die erste Innenfläche (112) eine erste Vielzahl von Führungswänden (130) einschließt, ein erstes proximales Ende (101) und ein erstes distales Ende (102) gegenüber dem ersten proximalen Ende (101), erste proximale Rampen (114) und erste distale Rampen (116), die gegenüber den ersten proximalen Rampen (114) angeordnet sind, und eine erste Seitenfläche und eine zweite Seitenfläche gegenüber der ersten Seitenfläche, wobei sich die erste und die zweite Seitenfläche zwischen dem ersten proximalen Ende und dem ersten distalen Ende erstrecken;
eine zweite Endplatte (120), wobei die zweite Endplatte (120) einschließt:
eine zweite Außenoberfläche (121) und eine zweite Innenoberfläche (122) gegenüber der zweiten Außenoberfläche (121), wobei die zweite Innenoberfläche (122) eine zweite Vielzahl von Führungswänden, ein zweites proximales Ende und ein zweites distales Ende gegenüber dem zweiten proximalen Ende, zweite proximale Rampen (124) und zweite distale Rampen (126), die gegenüber den zweiten proximalen Rampen (124) angeordnet sind, und eine dritte Seitenoberfläche und eine vierte Seitenoberfläche gegenüber der dritten Seitenoberfläche einschließt, wobei sich die dritte und die vierte Seitenoberfläche zwischen dem zweiten proximalen Ende und dem zweiten distalen Ende erstrecken;
einen Bewegungsmechanismus (250), der mit der ersten Endplatte (110) und der zweiten Endplatte (120) wirkgekoppelt und dazwischen positioniert ist, wobei der Bewegungsmechanismus (250) einschließt:
einen Stützblock (257) und einen ersten Schlitten (256) und einen zweiten Schlitten (258), die auf gegenüberliegenden Seiten des Stützblocks (257) angeordnet sind, eine Schraubführungswand, die eine drehbare erste Stellschraube (252) und eine drehbare zweite Stellschraube (254) gegenüber der ersten Stellschraube (252) aufnimmt, wobei die erste Stellschraube (252) mit dem ersten Schlitten (256) wirkgekoppelt ist und die zweite Stellschraube (254) mit dem zweiten Schlitten (258) wirkgekoppelt ist, wobei die erste Stellschraube (252) und die zweite Stellschraube (254) konfiguriert sind, um sich in einer ersten Drehrichtung und einer zweiten Drehrichtung um eine Drehachse zu drehen, wobei die Drehachse in Längsrichtung des Bewegungsmechanismus (250) verläuft,
wobei der erste Schlitten (256) mit der ersten Stellschraube (252) wirkgekoppelt ist und durch Drehung der ersten Stellschraube (252) entlang der Drehachse in Längsrichtung des Bewegungsmechanismus (250) auf den Stützblock (257) zu und von diesem weg bewegbar ist, der zweite Schlitten (258) mit der zweiten Stellschraube (254) wirkgekoppelt ist und durch Drehung der zweiten Stellschraube (254) entlang der Drehachse in Längsrichtung des Bewegungsmechanismus (250) auf den Stützblock (257) zu und von diesem weg bewegbar ist,
wobei der erste Schlitten (256) eine erste Seitenoberfläche und eine zweite Seitenoberfläche gegenüber der ersten Seitenoberfläche einschließt und eine erste Vielzahl von Vorsprüngen (256c) aufweist, die von der ersten und der zweiten Seitenoberfläche vorstehen, der zweite Schlitten (258) eine dritte Seitenoberfläche und eine vierte Seitenoberfläche gegenüber der dritten Seitenoberfläche einschließt und eine zweite Vielzahl von Vorsprüngen (258c) aufweist, die von der dritten und der vierten Seitenoberfläche vorstehen,
wobei die erste und die zweite Vielzahl von Vorsprüngen (256c, 258c) einer Querschnittsform der ersten und der zweiten Vielzahl von Führungswänden (130) entsprechen und jeweils mit diesen wirkgekoppelt sind, sodass sich die erste und die zweite Vielzahl von Vorsprüngen (256c, 258c) jeweils entlang der ersten und der zweiten Vielzahl von Führungswänden bewegen,
wobei der Bewegungsmechanismus (250) konfiguriert ist, um einen Abstand zwischen der ersten und der zweiten Endplatte (110, 120) bei gleichzeitiger Drehung der ersten und der zweiten Stellschraube (252, 254) entlang der Drehachse wirkeinzustellen, und
wobei der Bewegungsmechanismus (250) konfiguriert ist, um einen Neigungswinkel zwischen der ersten und der zweiten Endplatte (110, 120) beim Drehen der ersten Stellschraube oder der zweiten Stellschraube (252, 254) entlang der Drehachse wirkeinzustellen.

2. Wirbelsäulenimplantat (100, 200, 300, 600, 700) nach Anspruch 1, wobei der Bewegungsmechanismus (250) ferner konfiguriert ist zum:
Vergrößern eines ersten Abstands zwischen der ersten Endplatte (110) und dem Bewegungsmechanismus (250) und Vergrößern eines zweiten Abstands zwischen der zweiten Endplatte (120) und dem Bewegungsmechanismus (250) um den gleichen Betrag bei gleichzeitiger Drehung der ersten und der zweiten Stellschraube in der ersten Drehrichtung;
Verringern des ersten Abstands zwischen der ersten Endplatte (110) und dem Bewegungsmechanismus (250) und Verringern des zweiten Abstands zwischen der zweiten Endplatte (110) und dem Bewegungsmechanismus (250) um den gleichen Betrag bei gleichzeitiger Drehung der ersten und der zweiten Stellschraube in der zweiten Drehrichtung;
Vergrößern des Neigungswinkels zwischen der ersten und der zweiten Endplatte (110, 120) beim Drehen mindestens einer von der ersten Stellschraube oder der zweiten Stellschraube entlang der Drehachse in der ersten Richtung; und
Verringern des Neigungswinkels der ersten und der zweiten Endplatte (110, 120) beim Drehen mindestens einer von der ersten Stellschraube oder der zweiten Stellschraube entlang der Drehachse in der ersten Richtung.

3. Wirbelsäulenimplantat (100, 200, 300, 600, 700) nach Anspruch 1 oder 2,
wobei die ersten proximalen Rampen (114) eine erste und eine zweite Rampe einschließen, die neben dem ersten proximalen Ende angeordnet sind und von der ersten Innenoberfläche weg vorstehen,
wobei die ersten distalen Rampen (116) eine dritte und eine vierte Rampe einschließen, die neben dem ersten distalen Ende angeordnet sind und von der ersten Innenoberfläche weg vorstehen,
wobei die zweiten proximalen Rampen (124) eine fünfte und eine sechste Rampe einschließen, die neben dem zweiten proximalen Ende angeordnet sind und von der zweiten Innenoberfläche weg vorstehen, und
wobei die zweiten distalen Rampen (126) eine siebte und eine achte Rampe einschließen, die neben dem zweiten distalen Ende angeordnet sind und von der zweiten Innenoberfläche weg vorstehen,

4. Wirbelsäulenimplantat (100, 200, 300, 600, 700) nach Anspruch 3,
wobei der erste Schlitten (256) ferner einen ersten Keil (256d) umfasst, der von der ersten Seitenoberfläche in einer Querrichtung des Bewegungsmechanismus (250) vorsteht, und einen zweiten Keil (256d), der von der zweiten Seitenoberfläche in Querrichtung des Bewegungsmechanismus (250) vorsteht, und
wobei der zweite Schlitten (258) ferner einen dritten Keil (258d) umfasst, der von der dritten Seitenoberfläche in der Querrichtung des Bewegungsmechanismus (250) vorsteht, und einen vierten Keil (258d), der von der vierten Seitenoberfläche in Querrichtung des Bewegungsmechanismus (250) vorsteht.

5. Wirbelsäulenimplantat (100, 200, 300, 600, 700) nach Anspruch 4, wobei der erste Keil (256d) eine erste obere Kontaktoberfläche (256e) und eine erste untere Kontaktoberfläche (256f) einschließt, der zweite Keil (256d) eine zweite obere Kontaktoberfläche (256e) und eine zweite untere Kontaktoberfläche (256f) einschließt, der dritte Keil (258d) eine dritte obere Kontaktoberfläche (258e) und eine dritte untere Kontaktoberfläche (258f) einschließt, der vierte Keil (258d) eine vierte obere Kontaktoberfläche (258e) und eine vierte untere Kontaktoberfläche (258f) einschließt, und
wobei die erste und die zweite obere Kontaktoberfläche (256e) die ersten proximalen Rampen (114) berühren und die erste und die zweite untere Kontaktoberfläche (256f) die zweiten proximalen Rampen (124) berühren, und
wobei die dritte und die vierte obere Kontaktoberfläche (258e) die ersten distalen Rampen (116) berühren und die dritte und die vierte untere Kontaktoberfläche (258f) die zweiten distalen Rampen (126) berühren.

6. Wirbelsäulenimplantat (100, 200, 300, 600, 700) nach Anspruch 4, wobei der erste Keil (256d) eine erste gekrümmte obere Kontaktoberfläche (256e) und eine erste gekrümmte untere Kontaktoberfläche (256f) einschließt, der zweite Keil (256d) eine zweite gekrümmte obere Kontaktoberfläche (256e) und eine zweite gekrümmte untere Kontaktoberfläche (256f) einschließt, der dritte Keil (258d) eine dritte gekrümmte obere Kontaktoberfläche (258e) und eine dritte gekrümmte untere Kontaktoberfläche (258f) einschließt, der vierte Keil (258d) eine vierte gekrümmte obere Kontaktoberfläche (258e) und eine vierte gekrümmte untere Kontaktoberfläche (258f) einschließt,
wobei die erste und die zweite gekrümmte obere Kontaktoberfläche (256e) die ersten proximalen Rampen (114) berühren und die erste und die zweite gekrümmte untere Kontaktoberfläche (256f) die zweiten proximalen Rampen (224) berühren,
wobei die dritte und die vierte gekrümmte obere Kontaktoberfläche (258e) die ersten distalen Rampen (116) berühren und die dritte und vierte gekrümmte untere Kontaktoberfläche (258f) die zweiten distalen Rampen (226) berühren, und
wobei die erste bis vierte gekrümmte obere Oberfläche (256e, 258e) und die erste bis vierte gekrümmte untere Oberfläche (256f, 258f) konfiguriert sind, um die Einstellung des Neigungswinkels zwischen der ersten und der zweiten Endplatte (110, 120) beim Drehen der ersten Stellschraube (252) entlang der Drehachse zu erleichtern, indem sie es den jeweiligen gekrümmten Oberflächen ermöglichen, auf einer entsprechenden Rampe der ersten und der zweiten proximalen Rampe (114, 224) und der ersten und der zweiten distalen Rampe (116, 216) zu schwenken.

7. Wirbelsäulenimplantat (100, 200, 300, 600, 700) nach einem der Ansprüche 4 bis 6,
wobei der erste und der zweite Keil (256d) konfiguriert sind, um sich entlang der ersten und der zweiten geneigten Kontaktoberfläche jeweils der ersten und der zweiten proximalen Rampe (114, 224) zu bewegen, und
wobei der dritte und der vierte Keil (258d) konfiguriert sind, um sich optional entlang dritter und vierter geneigter Kontaktoberflächen jeweils der ersten und der zweiten distalen Rampe (116, 226) zu bewegen,
wobei die ersten geneigten Kontaktoberflächen der ersten proximalen Rampen (114) in Bezug auf die erste Innenoberfläche der ersten Endplatte (110) geneigt sind und sich von der ersten Innenoberfläche um eine erste geneigte Distanz weg erstrecken und die zweiten geneigten Kontaktoberflächen der zweiten proximalen Rampen (224) in Bezug auf die zweite Innenoberfläche der zweiten Endplatte (120) geneigt sind, und sich von der zweiten Innenoberfläche um eine zweite geneigte Distanz weg erstrecken,
wobei die dritten geneigten Kontaktoberflächen der ersten distalen Rampen (116) in Bezug auf die erste Innenoberfläche der ersten Endplatte (110) geneigt sind und sich von der ersten Innenoberfläche um eine dritte geneigte Distanz weg erstrecken und die vierten geneigten Kontaktoberflächen der zweiten distalen Rampen (226) in Bezug auf die zweite Innenoberfläche der zweiten Endplatte (120) geneigt sind, und sich von der zweiten Innenoberfläche um eine zweite geneigte Distanz weg erstrecken, und
wobei der erste geneigte Abstand größer ist als der dritte geneigte Abstand und der zweite geneigte Abstand größer ist als der vierte geneigte Abstand, wodurch das Einstellen des Neigungswinkels zwischen der ersten und der zweiten Endplatte (110, 120) beim Drehen der ersten Stellschraube entlang der Drehachse erleichtert wird.

8. Wirbelsäulenimplantat (100, 200, 300, 600, 700) nach einem der Ansprüche 1 bis 7, wobei jede Rampe der ersten und der zweiten proximalen Rampe (114, 224) und der ersten und der zweiten distalen Rampe (116, 226) eine entsprechende Kontaktoberfläche einschließt, und
wobei sich jede Führungswand der ersten Vielzahl von Führungswänden und jede Führungswand der zweiten Vielzahl von Führungswänden in einer parallelen Richtung mit mindestens einer Rampe der ersten und der zweiten proximalen Rampen (114, 224) und der ersten und der weiten distalen Rampen (116, 226) erstreckt.

9. Wirbelsäulenimplantat (100, 200, 300, 600, 700) nach einem der Ansprüche 1 bis 8, wobei die erste und die zweite Endplatte (110, 120) in einer seitlichen Richtung davon in Bezug auf den Bewegungsmechanismus (250) schwenkbar sind.

10. Wirbelsäulenimplantat (100, 200, 300, 600, 700) nach einem der Ansprüche 1 bis 9, wobei die erste und die zweite Endplatte (110, 120) konfiguriert sind, um das Knochenwachstum dazwischen zu fördern.

11. Wirbelsäulenimplantat (100, 200, 300, 600, 700) nach einem der Ansprüche 1 bis 10, wobei die erste und die zweite Endplatte (110, 120) eine Vielzahl von geneigten Öffnungen und eine entsprechende Vielzahl von Verankerungsschrauben einschließen, wobei jede Verankerungsschraube durch eine entsprechende geneigte Öffnung verläuft und konfiguriert ist, um sich in einem Wirbelkörper zu verankern.

12. Wirbelsäulenimplantat (100, 200, 300, 600, 700) nach einem der Ansprüche 1 bis 11, wobei das Schraubenführungswandgehäuse eine Öffnung einschließt, die durch das erste proximale Ende der ersten Endplatte (110) und das zweite proximale Ende der zweiten Endplatte (120) verläuft, wobei die Öffnung konfiguriert ist, um ein entsprechendes chirurgisches Werkzeug aufzunehmen, das Umfangskanten aufweist, die sich in einer Längsrichtung davon erstrecken,
wobei die Umfangskanten konfiguriert sind, um in einer ersten Einführposition selektiv mit der ersten Stellschraube und der zweiten Stellschraube in Eingriff zu gehen und in einer zweiten Einführposition nur mit der zweiten Stellschraube in Eingriff zu gehen,
wobei das entsprechende chirurgische Werkzeug konfiguriert ist, um selektiv die erste Stellschraube und/oder die zweite Stellschraube entlang der Drehachse zu drehen, wobei das Schraubenführungswandgehäuse optional ferner konfiguriert ist, um das entsprechende chirurgische Werkzeug in einem versetzten Winkel in Bezug auf die Drehachse des Bewegungsmechanismus (250) aufzunehmen.

13. Wirbelsäulenimplantat (100, 200, 300, 600, 700) nach einem der Ansprüche 1 bis 12, wobei die erste und die zweite Endplatte (110, 120) jeweils eine Grundfläche aufweisen, die für mindestens eine chirurgische Technik konfiguriert ist, die ausgewählt wird aus: vorderen chirurgischen Einführ- und Einstellungstechniken, schrägen chirurgischen Einführ- und Einstellungstechniken und seitlichen chirurgischen Einführ- und Einstellungstechniken,
optional wobei der Bewegungsmechanismus (250) konfiguriert ist zum:
Einstellen des Abstands zwischen der ersten und der zweiten Endplatte (110, 120) am proximalen Ende von etwa 10 mm auf etwa 22 mm und Einstellen des Abstands zwischen der ersten und der zweiten Endplatte (110, 120) am distalen Ende von etwa 7 mm auf etwa 12 mm; und
Einstellen des Neigungswinkels zwischen der ersten und der zweiten Endplatte (110, 120) innerhalb eines Winkelbereichs von etwa 7° bis etwa 25°.

14. Wirbelsäulenimplantat (100, 200, 300, 600, 700) nach einem der Ansprüche 1 bis 13, wobei der Bewegungsmechanismus (250) ferner konfiguriert ist zum:
Einstellen des Abstands zwischen der ersten und der zweiten Endplatte (110, 120) am proximalen Ende von etwa 9 mm auf etwa 16 mm und Einstellen des Abstands zwischen der ersten und der zweiten Endplatte (110, 120) am distalen Ende von etwa 9 mm auf etwa 16 mm; und
Einstellen des Neigungswinkels zwischen der ersten und der zweiten Endplatte (110, 120) innerhalb eines Winkelbereichs von etwa 6° bis etwa 11°.

15. Wirbelsäulenimplantat (100, 200, 300, 600, 700) nach einem der Ansprüche 1 bis 14, wobei die erste Endplatte (110) ein konkaves Oberflächenprofil in Bezug auf den Bewegungsmechanismus (250) aufweist und die zweite Endplatte (110) ein konvexes Oberflächenprofil in Bezug auf den Bewegungsmechanismus (250) aufweist.

## Revendications

1. Implant rachidien expansible (100, 200, 300, 600, 700) pouvant être déployé entre une position contractée et une position expansée, comprenant :
une première plaque d'extrémité (110), la première plaque d'extrémité (110) comportant :
une première surface extérieure (111) et une première surface intérieure (112) à l'opposé de la première surface extérieure (111), la première surface intérieure (112) comportant une première pluralité de parois de guidage (130), une première extrémité proximale (101) et une première extrémité distale (102) opposée à la première extrémité proximale (101), des premières rampes proximales (114) et des premières rampes distales (116) disposées à l'opposé des premières rampes proximales (114), et une première surface latérale et une deuxième surface latérale opposée à la première surface latérale, les première et deuxième surfaces latérales s'étendant entre la première extrémité proximale et la première extrémité distale ;
une seconde plaque d'extrémité (120), la seconde plaque d'extrémité (120) comportant :
une seconde surface extérieure (121) et une seconde surface intérieure (122) à l'opposé de la seconde surface extérieure (121), la seconde surface intérieure (122) comportant une seconde pluralité de parois de guidage, une seconde extrémité proximale et une seconde extrémité distale à l'opposé de la seconde extrémité proximale, des secondes rampes proximales (124) et des secondes rampes distales (126) disposées à l'opposé des secondes rampes proximales (124), et une troisième surface latérale et une quatrième surface latérale à l'opposé de la troisième surface latérale, les troisième et quatrième surfaces latérales s'étendant entre la seconde extrémité proximale et la seconde extrémité distale ;
un mécanisme de déplacement (250) accouplé de manière opérationnelle à la première plaque d'extrémité (110) et à la seconde plaque d'extrémité (120) et positionné entre celles-ci, le mécanisme de déplacement (250) comportant :
un bloc de contrefort (257) et un premier chariot (256) et un second chariot (258) disposés sur les côtés opposés du bloc de contrefort (257), un logement de la paroi de guidage de vis, une première vis de pression rotative (252) et une seconde vis de pression rotative (254) à l'opposé de la première vis de pression (252), la première vis de pression (252) étant accouplée de manière opérationnelle au premier chariot (256) et la seconde vis de pression (254) étant accouplée de manière opérationnelle au second chariot (258), la première vis de pression (252) et la seconde vis de pression (254) étant conçues pour tourner dans un premier sens de rotation et un second sens de rotation autour d'un axe de rotation, l'axe de rotation faisant saillie dans une direction longitudinale du mécanisme de déplacement (250),
dans lequel le premier chariot (256) est accouplé de manière opérationnelle à la première vis de pression (252) et peut se rapprocher et s'éloigner du bloc de contrefort (257) dans le sens longitudinal du mécanisme de déplacement (250) par rotation de la première vis de pression (252) le long de l'axe de rotation, le second chariot (258) est accouplé de manière opérationnelle à la seconde vis de pression (254) et peut se rapprocher et s'éloigner du bloc de contrefort (257) dans le sens longitudinal du mécanisme de déplacement (250) par rotation de la seconde vis de pression (254) le long de l'axe de rotation,
dans lequel le premier chariot (256) comporte une première surface latérale et une deuxième surface latérale à l'opposé de la première surface latérale et a une première pluralité de saillies (256c) faisant saillie à partir des première et deuxième surfaces latérales, le second chariot (258) comporte une troisième surface latérale et une quatrième surface latérale à l'opposé de la troisième surface latérale et a une seconde pluralité de saillies (258c) faisant saillie à partir des troisième et quatrième surfaces latérales,
dans lequel la première et la seconde pluralité de saillies (256c, 258c) correspondent à une forme de section transversale de la première et de la seconde pluralité de parois de guidage (130) et sont accouplées de manière opérationnelle à celles-ci, respectivement, de telle sorte que la première et la seconde pluralité de saillies (256c, 258c) se déplacent le long de la première et de la seconde pluralité de parois de guidage, respectivement,
dans lequel le mécanisme de déplacement (250) est conçu pour ajuster de manière opérationnelle un espacement entre la première et la seconde plaque d'extrémité (110, 120) lors de la rotation simultanée de la première et de la seconde vis de pression (252, 254) le long de l'axe de rotation, et
dans lequel le mécanisme de déplacement (250) est conçu pour ajuster de manière opérationnelle un angle d'inclinaison entre la première et la seconde plaque d'extrémité (110, 120) lors de la rotation de la première vis de pression ou de la seconde vis de pression (252, 254) le long de l'axe de rotation.

2. Implant rachidien (100, 200, 300, 600, 700,) selon la revendication 1, dans lequel le mécanisme de déplacement (250) est en outre conçu pour :
augmenter une première distance entre la première plaque d'extrémité (110) et le mécanisme de déplacement (250) et augmenter une seconde distance entre la seconde plaque d'extrémité (120) et le mécanisme de déplacement (250) d'une valeur égale lors de la rotation simultanée de la première et de la seconde vis de pression dans le premier sens de rotation ;
diminuer la première distance entre la première plaque d'extrémité (110) et le mécanisme de déplacement (250) et diminuer la seconde distance entre la seconde plaque d'extrémité (110) et le mécanisme de déplacement (250) d'une valeur égale lors de la rotation simultanée de la première et de la seconde vis de pression dans le second sens de rotation ;
augmenter l'angle d'inclinaison entre les première et seconde plaques d'extrémité (110, 120) lors de la rotation d'au moins l'une parmi la première vis de pression ou la seconde vis de pression le long de l'axe de rotation dans le premier sens ; et
diminuer l'angle d'inclinaison entre des première et seconde plaques d'extrémité (110, 120) lors de la rotation d'au moins l'une parmi la première vis de pression ou la seconde vis de pression le long de l'axe de rotation dans le premier sens .

3. Implant rachidien (100, 200, 300, 600, 700) selon la revendication 1 ou 2,
dans lequel les premières rampes proximales (114) comportent une première et une deuxième rampe disposées de manière adjacente à la première extrémité proximale qui font saillie au-delà de la première surface intérieure,
dans lequel les premières rampes distales (116) comportent une troisième et une quatrième rampe disposées de manière adjacente à la première extrémité distale qui font saillie au-delà de la première surface intérieure,
dans lequel les secondes rampes proximales (124) comportent une cinquième et une sixième rampe disposées de manière adjacente à la seconde extrémité proximale qui font saillie au-delà de la seconde surface intérieure, et
dans lequel les secondes rampes distales (126) comportent une septième et une huitième rampe disposées de manière adjacente à la seconde extrémité distale qui font saillie au-delà de la seconde surface intérieure,

4. Implant rachidien (100, 200, 300, 600, 700) selon la revendication 3,
dans lequel le premier chariot (256) comprend en outre une première cale (256d) faisant saillie à partir de la première surface latérale dans un sens transversal du mécanisme de déplacement (250) et une deuxième cale (256d) faisant saillie à partir de la deuxième surface latérale dans le sens transversal du mécanisme de déplacement (250), et
dans lequel le second chariot (258) comprend en outre une troisième cale (258d) faisant saillie à partir de la troisième surface latérale dans le sens transversal du mécanisme de déplacement (250) et une quatrième cale (258d) faisant saillie à partir de la quatrième surface latérale dans le sens transversal du mécanisme de déplacement (250).

5. Implant rachidien (100, 200, 300, 600, 700) selon la revendication 4, dans lequel la première cale (256d) comporte une première surface de contact supérieure (256e) et une première surface de contact inférieure (256f), la deuxième cale (256d) comporte une deuxième surface de contact supérieure (256e) et une deuxième surface de contact inférieure (256f), la troisième cale (258d) comporte une troisième surface de contact supérieure (258e) et une troisième surface de contact inférieure (258f), la quatrième cale (258d) comporte une quatrième surface de contact supérieure (258e) et une quatrième surface de contact inférieure (258f), et
dans lequel les première et deuxième surfaces de contact supérieures (256e) sont en contact avec les premières rampes proximales (114) et les première et deuxième surfaces de contact inférieures (256f) sont en contact avec les secondes rampes proximales (124), et
dans lequel les troisième et quatrième surfaces de contact supérieures (258e) sont en contact avec les premières rampes distales (116) et les troisième et quatrième surfaces de contact inférieures (258f) sont en contact avec les secondes rampes distales (126).

6. Implant rachidien (100, 200, 300, 600, 700) selon la revendication 4, dans lequel la première cale (256d) comporte une première surface de contact supérieure incurvée (256e) et une première surface de contact inférieure incurvée (256f), la deuxième cale (256d) comporte une deuxième surface de contact supérieure incurvée (256e) et une deuxième surface de contact inférieure incurvée (256f), la troisième cale (258d) comporte une troisième surface de contact supérieure incurvée (258e) et une troisième surface de contact inférieure incurvée (258f), la quatrième cale (258d) comporte une quatrième surface de contact supérieure incurvée (258e) et une quatrième surface de contact inférieure incurvée (258f),
dans lequel les première et deuxième surfaces de contact supérieures incurvées (256e) sont en contact avec les premières rampes proximales (114) et les première et deuxième surfaces de contact inférieures incurvées (256f) sont en contact avec les secondes rampes proximales (224),
dans lequel les troisième et quatrième surfaces de contact supérieures incurvées (258e) sont en contact avec les premières rampes distales (116) et les troisième et quatrième surfaces de contact inférieures incurvées (258f) sont en contact avec les secondes rampes distales (226), et
dans lequel les première à quatrième surfaces supérieures incurvées (256e, 258e) et les première à quatrième surfaces inférieures incurvées (256f, 258f) sont conçues pour faciliter le réglage de l'angle d'inclinaison entre les première et deuxième plaques d'extrémité (110, 120) lors de la rotation de la première vis de pression (252) le long de l'axe de rotation en permettant aux surfaces incurvées respectives de pivoter sur une rampe correspondante des premières et secondes rampes proximales (114, 224) et des premières et secondes rampes distales (116, 216).

7. Implant rachidien (100, 200, 300, 600, 700) selon l'une des revendications 4-6,
dans lequel les première et deuxième cales (256d) sont conçues pour se déplacer le long des premières et deuxièmes surfaces de contact inclinées des premières et secondes rampes proximales (114, 224), respectivement, et
dans lequel les troisième et quatrième cales (258d) sont conçues pour se déplacer le long des troisièmes et quatrièmes surfaces de contact inclinées des premières et secondes rampes distales (116, 226), respectivement, éventuellement,
dans lequel les premières surfaces de contact inclinées des premières rampes proximales (114) sont inclinées par rapport à la première surface intérieure de la première plaque d'extrémité (110) et s'étendent à partir de la première surface intérieure sur une première distance inclinée, et les deuxièmes surfaces de contact inclinées des secondes rampes proximales (224) sont inclinées par rapport à la seconde surface intérieure de la seconde plaque d'extrémité (120) et s'étendent à partir de la seconde surface intérieure sur une deuxième distance inclinée,
dans lequel les troisièmes surfaces de contact inclinées des premières rampes distales (116) sont inclinées par rapport à la première surface intérieure de la première plaque d'extrémité (110) et s'étendent à partir de la première surface intérieure sur une troisième distance inclinée, et les quatrièmes surfaces de contact inclinées des secondes rampes distales (226) sont inclinées par rapport à la seconde surface intérieure de la seconde plaque d'extrémité (120) et s'étendent à partir de la seconde surface intérieure sur une deuxième distance inclinée, et
dans lequel la première distance inclinée est supérieure à la troisième distance inclinée et la deuxième distance inclinée est supérieure à la quatrième distance inclinée, facilitant ainsi le réglage de l'angle d'inclinaison entre les première et seconde plaques d'extrémité (110, 120) lors de la rotation de la première vis de pression le long de l'axe de rotation.

8. Implant rachidien (100, 200, 300, 600, 700) selon l'une des revendications 1-7, dans lequel chaque rampe des premières et secondes rampes proximales (114, 224) et des premières et secondes rampes distales (116, 226) comporte une surface de contact correspondante, et
dans lequel chaque paroi de guidage de la première pluralité de parois de guidage et chaque paroi de guidage de la seconde pluralité de parois de guidage s'étend dans un sens parallèle par rapport à au moins une rampe parmi les premières et secondes rampes proximales (114, 224) et les premières et secondes rampes distales (116, 226).

9. Implant rachidien (100, 200, 300, 600, 700) selon l'une des revendications 1-8, dans lequel les première et seconde plaques d'extrémité (110, 120) peuvent pivoter dans un sens latéral de celui-ci par rapport au mécanisme de déplacement (250).

10. Implant rachidien (100, 200, 300, 600, 700) selon l'une des revendications 1-9, dans lequel les première et seconde plaques d'extrémité (110, 120) sont conçues pour favoriser la croissance osseuse entre elles.

11. Implant rachidien (100, 200, 300, 600, 700) selon l'une des revendications 1-10, dans lequel les première et seconde plaques d'extrémité (110, 120) comportent une pluralité d'ouvertures inclinées et une pluralité correspondante de vis d'ancrage, chaque vis d'ancrage traversant une ouverture inclinée correspondante et étant conçue pour s'ancrer dans un corps vertébral.

12. Implant rachidien (100, 200, 300, 600, 700) selon l'une des revendications 1-11, dans lequel le logement de la paroi de guidage de vis comporte une ouverture traversant la première extrémité proximale de la première plaque d'extrémité (110) et la seconde extrémité proximale de la seconde plaque d'extrémité (120), l'ouverture étant conçue pour recevoir un outil chirurgical correspondant ayant des bords circonférentiels s'étendant dans un sens longitudinal de celui-ci,
dans lequel les bords circonférentiels sont conçus pour venir en prise de manière sélective avec la première et la seconde vis de pression dans une première position d'insertion, et pour venir en prise uniquement avec la seconde vis de pression dans une seconde position d'insertion,
dans lequel l'outil chirurgical correspondant est conçu pour faire tourner sélectivement la première vis de pression et/ou la seconde vis de pression le long de l'axe de rotation, éventuellement, dans lequel le logement de la paroi de guidage de vis est en outre conçu pour recevoir l'outil chirurgical correspondant à un angle décalé par rapport à l'axe de rotation du mécanisme de déplacement (250).

13. Implant rachidien (100, 200, 300, 600, 700) selon l'une des revendications 1-12, dans lequel les première et seconde plaques d'extrémité (110, 120) ont chacune une empreinte conçue pour au moins une technique chirurgicale choisie parmi : les techniques d'insertion et d'ajustement chirurgicaux antérieurs, les techniques d'insertion et d'ajustement chirurgicaux obliques, et les techniques d'insertion et d'ajustement chirurgicaux latéraux,
éventuellement, dans lequel le mécanisme de déplacement (250) est conçu pour :
ajuster l'espacement entre la première et la seconde plaque d'extrémité (110, 120) au niveau de l'extrémité proximale d'environ 10 mm à environ 22 mm et ajuster l'espacement entre la première et la seconde plaque d'extrémité (110, 120) au niveau de l'extrémité distale d'environ 7 mm à environ 12 mm ; et
ajuster l'angle d'inclinaison entre la première et la seconde plaque d'extrémité (110, 120) dans une plage angulaire d'environ 7° à environ 25°.

14. Implant rachidien (100, 200, 300, 600, 700) selon l'une des revendications 1-13, dans lequel le mécanisme de déplacement (250) est conçu pour :
ajuster l'espacement entre la première et la seconde plaque d'extrémité (110, 120) au niveau de l'extrémité proximale d'environ 9 mm à environ 16 mm et ajuster l'espacement entre la première et la seconde plaque d'extrémité (110, 120) au niveau l'extrémité distale d'environ 9 mm à environ 16 mm ; et
ajuster l'angle d'inclinaison entre la première et la seconde plaque d'extrémité (110, 120) dans une plage angulaire d'environ 6° à environ 11°.

15. Implant rachidien (100, 200, 300, 600, 700) selon l'une des revendications 1-14, dans lequel la première plaque d'extrémité (110) a un profil de surface concave par rapport au mécanisme de déplacement (250) et la seconde plaque d'extrémité (110) a un profil de surface convexe par rapport au mécanisme de déplacement (250).
